# EUROPEAN PATENT SPECIFICATION

(11) **EP 1 265 927 B1**
(45) Date of publication and mention of the grant of the patent: **29.04.2009**
(21) Application number: 01923873.2
(22) Date of filing: 09.03.2001
(51) Int. Cl.: C07K 14/72

(54) **CRYSTAL**
KRISTALL
CRISTAL

(30) Priority: 09.03.2000 GB 0005689
(43) Date of publication of application: 18.12.2002
(73) Proprietor: Bayer Schering Pharma Aktiengesellschaft, 13353 Berlin (DE)
(72) Inventor: DONNER, Peter, 12169 Berlin (DE); EGNER, Ursula, 10777 Berlin (DE); CARRONDO, Maria, Armenia, P-2780 469 Paco de Arcos (PT); MATIAS, Pedro, M., P-2780 Porto Salvo (PT)
(74) Representative: Harding, Charles Thomas
(86) International application number: PCT/IB2001/000475
(87) International publication number: WO 2001/066599

(56) References cited:
- WO-A-01/27622
- WO-A-97/21993
- WILLIAMS SHAWN P ET AL: "Atomic structure of progesterone complexed with its receptor." NATURE (LONDON), vol. 393, no. 6683, 28 May 1998 (1998-05-28), pages 392-396, XP002173773 ISSN: 0028-0836 cited in the application
- YONG E L ET AL: "Partial androgen insensitivity and correlations with the predicted three dimensional structure of the androgen receptor ligand-binding domain." MOLECULAR AND CELLULAR ENDOCRINOLOGY, vol. 13, no. 1, 13 February 1998 (1998-02-13), pages 41-50, XP001013088 ISSN: 0303-7207 cited in the application
- GOTTLIEB BRUCE ET AL: "Update of the androgen receptor gene mutations database." HUMAN MUTATION, vol. 14, no. 2, 1999, pages 103-114, XP002173777 ISSN: 1059-7794
- DATABASE BIOSIS [Online] BIOSCIENCES INFORMATION SERVICE, PHILADELPHIA, PA, US; 1979 ZAVA D T ET AL: "ANDROGEN RECEPTOR ASSAY WITH TRITIATED METHYL TRIENOLONE R-1881 IN THE PRESENCE OF PROGESTERONE RECEPTORS" Database accession no. PREV197968033984 XP002173787 & ENDOCRINOLOGY, vol. 104, no. 4, 1979, pages 1007-1012, EN ISSN: 0013-7227
- MATIAS PEDRO M ET AL: "Structural evidence for ligand specificity in the binding domain of the human androgen receptor: Implications for pathogenic gene mutations." JOURNAL OF BIOLOGICAL CHEMISTRY, vol. 275, no. 34, 25 August 2000 (2000-08-25), pages 26164-26171, XP002173776 ISSN: 0021-9258
- KLEBE GERHARD: "Recent developments in structure-based drug design." JOURNAL OF MOLECULAR MEDICINE (BERLIN), vol. 78, no. 5, 2000, pages 269-281, XP002173778 ISSN: 0946-2716

## Description

### FIELD OF THE INVENTION

The present invention relates to a crystal structure.

In particular, the present invention relates to a crystal structure for a ligand binding domain (LBD).

In particular, the present invention relates to a crystal structure for a ligand binding domain (LBD) optionally having a ligand which is associated therewith.

In particular, the present invention relates to a crystal structure for a LBD of a receptor.

More in particular, the present invention relates to a crystal structure for a LBD of an androgen receptor (AR-LBD) and also to a crystal structure for an AR-LBD-ligand complex.

The structure may be used to determine androgen receptor homologues and information about secondary and tertiary structures of polypeptides which are as yet structurally uncharacterised. The structure may also be used to identify ligands which are capable of binding to the androgen receptor. Such ligands may be capable of acting as modulators of androgen receptor activity.

The crystal structure of AR-LBD enables a model to be produced for androgen receptor activity. Thus, the present invention provides a model which can be used to understand the structural implications of the binding mechanism.

### BACKGROUND TO THE INVENTION

The androgen receptor (AR) is a member of the superfamily of nuclear receptors which includes, amongst others, the steroid receptors as well as the vitamin D, thyroid, retinoic acid receptors and the so-called orphan receptors. In addition, the AR is a member of a group of four closely related steroid receptors including the progesterone receptors (PR), the mineralocorticoid receptor and the glucocorticoid receptor all of which recognise the same hormone response element. In general, steroid receptors are comprised of five to six domains which act as ligand-activated transcription factors that control the expression of specific genes. The ligand binding region is located in the C terminal domain and is called the ligand binding domain (LBD). Binding of a ligand (such as a steroid hormone) to the LBD induces changes in receptor conformation that control transcriptional activation and repression and also regulate homo- or heterodimerisation. In the absence of ligand, these receptors repress basal gene expression, probably through the expression of co-repressor proteins.

The androgen hormones and their receptors play an important role in male physiology and pathology. The androgen receptor binds the male sex steroids, dihydrotestosterone (DHT) and testosterone [Teutsch, 1994], and regulates genes for male differentiation and development. Consequently, constitutional mutations in the androgen receptor gene may lead to several disease states. Some examples of these diesease states include prostate cancer (PC) and the androgen insensitivity syndrome (AIS) which are capable of impairing androgen-dependent male sexual differentiation to various degrees. In addition, complete androgen insensitivity syndrome (CAIS) leads to an unequivocally external female phenotype. In contrast, partial or incomplete androgen insensitivity syndrome (PAIS) comprises a wide spectrum of clinical phenotypes while mild androgen insensitivity syndrome (MAIS), is connected to forms of undervirilisation [Bellis, 1992 ]. About 50% of the mutated residues reported in the human androgen receptor ligand binding domain (hAR LBD) to date are found to be involved in prostate cancer (PC) and in AIS [Gottlieb, 1998]. These mutations have been well documented in the Androgen Receptor Gene Mutations Database of the Lady Davis Institute for Medical Research [Gottlieb, 1998].

To date, there are a total of 20 known amino acid residues in the AR LBD which are involved in ligand interaction. Of these 20 amino acid residues, to date, mutations have been reported in 14 of the 20 amino acid residues. These mutations are largely in the ligand binding pocket (LBP) which is part of the AR-LBD. By way of example, the three mutations in the LBP of the hAR, which have been described for CAIS, these being N705S [Bellis, 1992; Pinsky, 1992], L707R [Lumbroso, 1996] and M749V [Bellis, 1992; Jakubicza, 1992]} are recognised as substitutions that considerably change the size and charge properties of the respective amino acid side chains. However, while it is known that these amino acid substitutions result in a considerably change in size of the respective amino acid side chains, it is not known how this change in size alters the AR-LBD such that the local structure and interactions with the ligand are disturbed. Moreover, because both the structural implications and the effects of these known mutation have not been determined, no ligand binding data are available for many of the published mutations in the AR-LBD.

In order to develop an understanding of the structural implications of mutations resulting in amino acid substitutions in the AR-LBD, attempts have been made by workers to determine the primary, secondary and tertiary structures of the AR-LBD. In this regard, the LBDs of the different nuclear receptor families have been analysed and shown to share a similar fold in spite of their low (about 20%) sequence homology. In this respect, the receptor fold has been shown to comprise about 12 helices and several small β-sheet arranged in a so-called "α-helical sandwich". Up until now, this kind of fold has only been observed for the LBDs of nuclear receptors. However, it has also been shown that, depending on the nature of the bound ligand, which may be an agonist or an antagonist, the carboxyterminal helix H12 may be found in either one of two orientations. In the agonist-bound conformation, helix H12 serves as a 'lid' to close the ligand-binding pocket (LBP), which contains the LBD, whereas in the antagonist-bound conformation, helix H12 is positioned in a different orientation thus opening the entrance to the LBP.

Despite the availability of information regarding the role of the helix H12 region in ligand binding, there is very little experimental information available about the structure or the role of the other helical regions (such as helices H1 to H11) with respect to ligand binding. By way of example, there has been a suggestion that helices H4 and H5 may be regions involved in ligand binding. However, no experimental information is available with respect to these helices in the AR-LBD. In addition, while it is thought that while about 50% of the mutated residues reported in the hAR LBD are found to be involved in prostate cancer (PC) and in AIS [Gottlieb, 1998], it is not known experimentally whether the mutations are predominantly found in the interior of the receptor protein or at the surface of the receptor protein.

Structurally, it is known that the nuclear receptors, such as the androgen receptor, can be organised into functional modules comprising an N-terminal transcriptional activation domain, a central DNA binding domain (DBD) and a C-terminal ligand binding domain (LBD). During the past few years, X-ray structures have been published for two of the domains, the DNA-binding domain as well as for a number of ligand-binding domains (LBD) including LBD-ligand complexes of receptors such as the estrogen receptor α and β, the progesterone receptor (PR), the vitamin D receptor, the retinoic acid receptors (X: RXR, acid: RAR), the thyroid hormone receptor and the peroxisome proliferator-activated receptors [Moras, 1998; Brzozowski, 1997; Tannenbaum, 1998; Shiau, 1998; Bourguet, 1995; Renaud, 1995; Wagner, 1995; Ribeiro, 1998; Williams, 1998; Nolte, 1998; Uppenberg, 1998 ; Klaholz, 1998; Rochel, 1999].

To date, no X-ray structures have been published for the AR-LBD either alone or in combination with a ligand. Although a model structure of the AR-LBD has been developed by Yong et al (1998), this model is based on the crystal structure of the RARα LBD [Bourguet, 1995] and not on either the AR-LBD or a more closely related receptor such as a PR-LBD. In addition, no experimentally determined three-dimensional (3D) structure is available for a complete androgen receptor either alone or in combination with a ligand. Furthermore, although the crystal structure of the progesterone receptor (PR) LBD in complex with progesterone was published in 1998 by Williams and Sigler, no comparative experimental analyses have been carried out between closely related steroid receptors such as an androgen receptor-LBD and progesterone receptor, either alone or complexed with ligands in order to identify ligand specificities and/or ligand specific residues.

### SUMMARY OF THE INVENTION

In a broad aspect the present invention relates to cystal structures of receptor ligand binding domains including the uses thereof.

### SUMMARY ASPECTS

According a first aspect of the invention there is provided a crystal structure comprising an AR-LBD.

In a preferred embodiment the crystal structure is a crystal structure for an AR-LBD.

The structure of a crystal AR-LBD has been solved and is set forth in Table 4.

In a second aspect the present invention provides a crystal structure comprising an AR-LBD-ligand complex.

In a third aspect the present invention provides a crystal structure comprising an AR-LBP.

According to a fourth aspect of the invention, there is provided a model of at least part of an AR-LBD made using or comprising or depicting a crystal structure according to any one of the first, second and third aspects of the invention. The crystal structure of the first, second and third aspect of the invention and the model of the fourth aspect of the invention may be provided in the form of a computer readable medium.

The crystals and models of earlier aspects of the invention may provide information about the atomic contacts involved in the interaction between the receptor and a known ligand, which can be used to screen for unknown ligands.

According to a fifth aspect of the invention, there is provided a method of screening for a ligand capable of binding an androgen receptor binding domain, comprising the use of a crystal structure according to any one of the first, second or third aspects of the invention or a model according to the fourth aspect of the invention. For example, the method may comprise the step of contacting the AR-LBD with a test compound, and determining if said test compound binds to said ligand binding domain. The method may be an *in vitro* method and/or an *in silico* method and/or an *in vivo* method.

In a sixth aspect, the present invention provides a ligand identified by a screening method of the fifth aspect of the invention. Preferably the ligand is capable of modulating the activity of an AR-LBD. As mentioned above, ligands which are capable of modulating the activity of AR-LBDs have considerable therapeutic and prophylactic potential.

In a seventh aspect, the present invention provides the use of a ligand according to the sixth aspect of the invention, in the manufacture of a medicament to treat and/or prevent a disease in a mammalian patient. There is also provided a pharmaceutical composition comprising such a ligand and a method of treating and/or preventing a disease comprising administering the step of administering such a ligand according or pharmaceutical composition to a mammalian patient.

The crystal structures and models described above also provide information about the secondary and tertiary structure of AR-LBDs. This can be used to gleen structural information about other, previously uncharacterised polypeptides. Thus, according to an eighth aspect of the invention there is provided a method of determining the secondary and/or tertiary structures of polypeptides with unknown (or only partially known) structure comprising the step of using such a crystal or model. The polypeptide under investigation is preferably structurally or functionally related to the androgen receptor ligand binding domain. For example, the polypeptide may show a degree of homology over some or all parts of the primary amino acid sequence. Alternatively, the polypeptide may perform an analogous function or be suspected to show a similar binding mechanism to the AR-LBD.

The present invention demonstrates that the hAR-LBD crystal structure can be used to analyse and explain the structural implications of 14 known mutations in the LBP of the hAR LBD which are associated with either prostate cancer (PC), the partial androgen receptor insensitivity syndrome (PAIS), mild androgen receptor insensitivity syndrome (MAIS) or complete androgen receptor insensitivity syndrome (CAIS).

The present invention also demonstrates that a crystal structure of an AR-LBD may be used to identify ligands (such as agonists/antagonists) with binding specificity for the AR LBD. In this way, compounds may be selected, improved or modified to improve this ligand binding interaction.

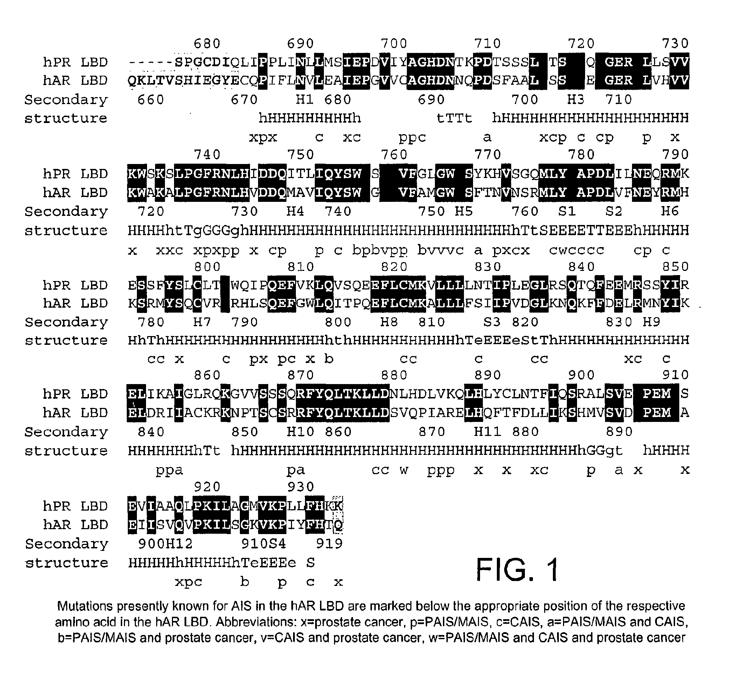

The present invention also provides the crystal structure of the human hAR LBD in complex with the ligand metribolone (R1881) and the crystal structure of the human hPR LBD in complex with the ligand metribolone (R1881). The provision, for the first time, of these two experimentally determined three dimensional (3D) crystal stuctures has facilitated a comparison to be drawn between the crystal structure of both receptors in complex with the same ligand. Up until now, it has been known from studies on model receptros that the AR-LBD and the PR-LBD have a number of similarities in that:
(i) they belong to the same steroid receptor subfamily;
(ii) they share about 54% LBD sequence identity (Figure 1); and
(iii) there are a number of different ligands with similar binding affinities for both receptors [Teutsch, 1994].

The present invention highlights an additional similarity between the hAR-LBD and hPR-LBD ligand complexes in that the three-dimensional structures of the hAR LBD as well as the hPR LBD demonstrate the typical nuclear receptor fold.

The present invention also demonstrates some hitherto unknown, but important, differences between the two receptors. These include:
(i) the identification of a two amino acid residue change in the ligand binding pocket (LBP) of the AR-LBD which is the most likely site for the specific binding of the R1881 ligand to the hAR-LBD. The AR-LBD amino acid residues are Leu 880 and Thr 877. The corresponding PR-LBD amino acid residues are Thr 894 and Cys 891. In addition, there are three other amino acid changes which maybe involved in binding of ligands other than R1881. The AR amino acid residues are Gln 783, Met 749 and Phe 876. The PR amino acid residues are Leu 797, Leu 763 and Tyr 890.
(ii) the demonstration that the hPR LBD - R1881 complex crystallises as a dimer in the asymmetric unit whereas the hAR LBD-R1881 complex crystallises as a monomeric unit.
(iii) the demonstration that the two independent molecules in the crystal structure of hPR LBD - R1881 exhibit different modes of ligand binding. One orientation of R1881 in one monomer resembles that of R1881 in the hAR LBD complex, while in the second monomer, R1881 is orientated similar to progesterone in the hPR LBD - progesterone complex.

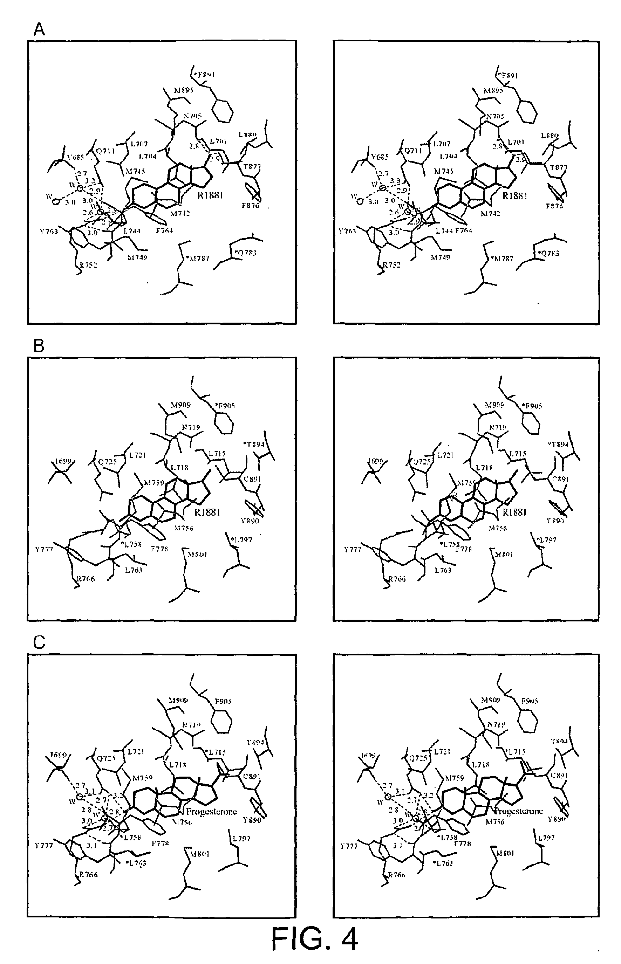

The present invention demonstrates the surprising and unexpected findings that:
(i) the helix H6 in the AR-LBD is an α-helix. In striking contrast, no α-helix was found either in the model hAR-LBD in this area or in the hPR-LBD-progesterone complex (Molecule A) (see Figure 4) whereas in the hPR-LBD-progesterone complex (Molecule B), an α-helix is observed.
(ii) helices H4 and H5 and helices H10 and H11 are preferably contiguous helices. That is, these helices H4 and H5 and H10 and H11 are connected to each other to form 2 continuous helices rather than 4 separate helices. Accordingly, the α-helical sandwich structure for the AR-LBD comprises preferably 9 α-helical regions instead preferably 11 α-helices. This observation was not seen in the liganded PR-LBD (Williams, 1998) which comprises 10 α-helices and where only helices H10 and H11 are contiguous sequences.
(iii) in the hAR-LBD-R1881 complex, the helix H12 is split into two shorter helical segments with 9 and 5 amino acid residues respectively. This observation was not seen in the hPR LBD-R1881 complex structure although a bending of helix H12 was also seen. As it is known that helix H12 may influence the binding of antagonists and agonists, this finding may have important implications for ligand binding.
(iv) the demonstration that the two independent molecules in the crystal structure of hPR LBD - R1881 exhibit different modes of ligand binding. One orientation of R1881 in one monomer resembles that of R1881 in the hAR LBD complex, in the second monomer R1881 is orientated similar to progesterone in the hPR LBD - progesterone complex.

The present invention is advantageous as the determination of the 3D structure of the AR-LBD allows the AR-LBD to be mapped.

The use of the crystals stucture in conjunction with this map enables a better understanding of ligand specificities for the AR-LBD.

In particular, the crystal structure of the present invention now makes it possible to see:
(i) not only how a ligand binds to the AR-LBD but also
(ii) the structural reasons why a ligand binds to an AR-LBD.

Using the crystal structure, these effects can not only be understood but can also be predicted. This improved understanding of the AR-LBD facilitates the identification and modification of ligands which are capable of specifically and/or preferentially interacting with the AR-LBD.

The present invention is also advantageous as it facilitates:
(i) the identification and characterization of the key residues within the AR-LBD and a comparison with those associated with the PR-LBD. In this regard, the present invention demonstrates an important new finding in relation to the PR-LBD-progesterone complex. In this respect, Asn 705 in the AR-LBD and Asn 719 in the PR-LBD have been shown to be capable of acting as hydrogen bond partners for ligands, which have, for example, a hydroxyl group attached to position 17 or to a substituent attached to position 17 on a steroidal ligand.
(ii) the identification and characterization of the interaction of ligands with the AR-LBD sites.
(iii) the identification of ligands with enhanced properties capable of interacting with one or more residues of the LBD. These enhanced properties include but not limited to: (a) higher affinity, (b) improved selectivity for the AR, and/or (c) a designated degree of efficacy (agonism vs. partial agonism vs. antagonism vs partial antagonism).
(iv) the design of one or more ligands which may specifically bind to an AR-LBD but not to a PR-LBD (ie a selective ligand).
(v) the determination of the structural effects associated with a mutation. (In this respect, although, many of the phenotypic traits associated with the characterised mutations in the androgen receptor gene are known, the structural implications of such mutations have not been determined).
(vi) the identification of ligands capable of overcoming the mutation/structural disturbance in the AR-LBD and/or LBP comprising the AR-LBD.
(vii) the determination of ligand binding data (affinity constants etc) which have not been available for many of the published mutant receptors.
(viii) the implementation of an iterative drug design and/or for "reverse-engineering" or " *de novo* design" of compounds and/or "structure-based drug design".
(ix) a detailed understanding of the structure of the LBDs receptors, such as the AR and PR which enables *in vitro* ligand binding data to be explained and understood.
(x) a reduction in the length of time required to discover compounds that target the AR-LBD.

Other aspects of the present invention are presented in the accompanying claims and in the following description and drawings. These aspects are presented under separate section headings. However, it is to be understood that the teachings under each section are not necessarily limited to that particular section heading.

### DETAILED ASPECTS OF THE INVENTION

Unless otherwise indicated, all terms used herein have the same meaning as they would to one skilled in the art of the present invention. Practitioners are particularly directed to Current Protocols in Molecular Biology (Ansubel) for definition and terms of the art.

According to one aspect of the present invention, there is provided a crystal structure comprising an androgen receptor ligand binding domain (AR-LBD).

Preferably the AR-LBD is a human AR-LBD.

In a preferred aspect of the present invention, there is provided a crystal structure comprising a ligand binding domain (LBD) wherein the LBD is arranged in an α-helical sandwich comprising preferably the α-helices H1, H3, H4, H5, H6, H7, H8, H9, H10, H1 and H12; preferably two 3₁₀ helices; and preferably four short β strands (S1, S2, S3 and S4) associated in two anti-parallel β-sheets; wherein the helices H4, H5, H10 and H11 are preferably contiguous helices; and wherein either helix H6 is preferably an α-helix and/or helix H12 comprises preferably two helical segments of preferably 9 amino acid residues and preferably 5 amino acid residues.

### CRYSTAL

As used herein, the term "crystal" means a structure (such as a three dimensional (3D) solid aggregate) in which the plane faces intersect at definite angles and in which there is a regular structure (such as internal structure) of the constituent chemical species. Thus, the term "crystal" can include any one of: a solid physical crystal form such as an experimentally prepared crystal, a 3D model based on the crystal structure, a representation thereof such as a schematic representation thereof or a diagrammatic representation thereof, a data set thereof for a computer.

### CRYSTAL PREPARATION

The crystals of the present invention may be prepared by expressing a nucleotide sequence encoding the AR-LBD and PR-LBD by use of a suitable host cell and then crystallising the purified receptor protein.

The invention also features a method for creating crystalline AR-LBD structures described herein. The method may utilize a polypeptide comprising an AR-LBD described herein to form a crystal. A polypeptide used in the method may be chemically synthesized in whole or in part using techniques that are well-known in the art. Alternatively, methods are well known to the skilled artisan to construct expression vectors containing the native or mutated AR-LBD coding sequence and appropriate transcriptional/translational control signals. These methods include *in vitro* recombinant DNA techniques, synthetic techniques, and *in vivo* recombination/genetic recombination. See for example the techniques described in Sambrook et al. (Molecular Cloning: A Laboratory Manual, 2nd Edition, Cold Spring Harbor Laboratory press (1989)), and other laboratory textbooks. (See also Sarker et al, Glycoconjugate J. 7:380, 1990; Sarker et al, Proc. Natl. Acad, Sci. USA 88:234-238, 1991, Sarker et al, Glycoconjugate J. 11: 204-209, 1994; Hull et al, Biochem Biophys Res Commun 176:608, 1991 and Pownall et al, Genomics 12:699-704, 1992).

Crystals are grown from an aqueous solution containing the purified AR-LBD polypeptide by a variety of conventional processes. These processes include batch, liquid, bridge, dialysis, vapor diffusion, and hanging drop methods. (See for example, McPherson, 1982 John Wiley, New York; McPherson, 1990, Eur. J. Biochem. 189: 1-23; Webber. 1991, Adv. Protein Chem. 41:1-36). Generally, the native crystals of the invention are grown by adding precipitants to the concentrated solution of the AR-LBD polypeptide. The precipitants are added at a concentration just below that necessary to precipitate the protein. Water is removed by controlled evaporation to produce precipitating conditions, which are maintained until crystal growth ceases.

Derivative crystals of the invention can be obtained by soaking native crystals in a solution containing salts of heavy metal atoms. A complex of the invention can be obtained by soaking a native crystal in a solution containing a compound that binds the AR-LBD, or they can be obtained by co-crystallizing the AR-LBD polypeptide in the presence of one or more compounds that bind to the AR-LBD.

Once the crystal is grown it can be placed in a glass capillary tube and mounted onto a holding device connected to an X-ray generator and an X-ray detection device. Collection of X-ray diffraction patterns are well documented by those skilled in the art (See for example, Ducruix and Geige, 1992, IRL Press, Oxford, England). A beam of X-rays enter the crystal and diffract from the crystal. An X-ray detection device can be utilized to record the diffraction patterns emanating from the crystal. Suitable devices include the Marr 345 imaging plate detector system with an RU200 rotating anode generator.

Methods for obtaining the three dimensional structure of the crystalline form of a molecule or complex are described herein and known to those skilled in the art (see Ducruix and Geige). Generally, the x-ray crystal structure is given by the diffraction patterns. Each diffraction pattern reflection is characterized as a vector and the data collected at this stage determines the amplitude of each vector. The phases of the vectors may be determined by the isomorphous replacement method where heavy atoms soaked into the crystal are used as reference points in the X-ray analysis (see for example, Otwinowski, 1991, Daresbury, United Kingdom, 80-86). The phases of the vectors may also be determined by molecular replacement (see for example, Naraza, 1994, Proteins 11:281-296). The amplitudes and phases of vectors from the crystalline form of an AR-LBD determined in accordance with these methods can be used to analyze other crystalline AR-LBDs.

The unit cell dimensions and symmetry, and vector amplitude and phase information can be used in a Fourier transform function to calculate the electron density in the unit cell i.e. to generate an experimental electron density map. This may be accomplished using the PHASES package (Furey, 1990). Amino acid sequence structures are fit to the experimental electron density map (i.e. model building) using computer programs (e.g. Jones, TA. et al, Acta Crystallogr A47, 100-119, 1991). This structure can also be used to calculate a theoretical electron density map. The theoretical and experimental electron density maps can be compared and the agreement between the maps can be described by a parameter referred to as R-factor. A high degree of overlap in the maps is represented by a low value R-factor. The R-factor can be minimized by using computer programs that refine the structure to achieve agreement between the theoretical and observed electron density map. For example, the XPLOR program, developed by Brunger (1992, Nature 355:472-475) can be used for model refinement.

A three dimensional structure of the molecule or complex may be described by atoms that fit the theoretical electron density characterized by a minimum R value. Files can be created for the structure that defines each atom by coordinates in three dimensions.

### AR AND PR CONSTRUCTS

The proteins comprising the AR-LBD and PR-LBD may be produced by a host recombinant cell may be secreted or may be contained intracellularly depending on the nucleotide sequence and/or the vector used. As will be understood by those of skill in the art, expression vectors containing the AR and PR encoding nucleotide sequences can be designed with signal sequences which direct secretion of the AR and PR coding sequences through a particular prokaryotic or eukaryotic cell membrane. Other recombinant constructions may join the AR or PR encoding sequence to nucleotide sequence encoding a polypeptide domain which will facilitate purification of soluble proteins (Kroll DJ et al (1993) DNA Cell Biol 12:441-53). Such purification facilitating domains include, but are not limited to, metal chelating peptides such as histidine-tryptophan modules that allow purification on immobilized metals (Porath J (1992) Protein Expr Purif 3 - .26328 1), protein A domains that allow purification on immobilized immunoglobulin, and the domain utilized in the FLAGS extension/affinity purification system (Immunex Corp, Seattle, WA). The inclusion of a cleavable linker sequence such as Factor XA or enterokinase (Invitrogen, San Diego, CA) between the purification domain and the AR and PR is useful to facilitate purification.

### HOST CELLS

A wide variety of host cells can be employed for expression of the nucleotide sequences encoding the AR and PR proteins of the present invention. These cells may be both prokaryotic and eukaryotic host cells. Suitable host cells include bacteria such as *E. coli,* yeast, filamentous fungi, insect cells, mammalian cells, typically immortalized, e.g., mouse, CHO, human and monkey cell lines and derivatives thereof. Preferred host cells are able to process the expression products to produce an appropriate mature polypeptide. Processing includes but is not limited to glycosylation, ubiquitination, disulfide bond formation and general post-translational modification.

### NUCLEOTIDE SEQUENCES

As used herein, the term "nucleotide sequence" refers to nucleotide sequences, oligonucleotide sequences, polynucleotide sequences and variants, homologues, fragments and derivatives thereof (such as portions thereof) which comprise the nucleotide sequences encoding the AR-LBD and PR-LBD. The nucleotide sequence may be DNA or RNA of genomic or synthetic or recombinant origin which may be double-stranded or single-stranded whether representing the sense or antisense strand or combinations thereof. Preferably, the term nucleotide sequence is prepared by use of recombinant DNA techniques (e.g. recombinant DNA). The nucleotide sequence may include within them synthetic or modified nucleotides. A number of different types of modification to oligonucleotides are known in the art. These include methylphosphonate and phosphorothioate backbones, addition of acridine or polylysine chains at the 3' and/or 5' ends of the molecule. For the purposes of the present invention, it is to be understood that the nucleotide sequences described herein may be modified by any method available in the art. Such modifications may be carried out in order to enhance the *in vitro* activity or life span of nucleotide sequences of the invention.

Preferably, the term "nucleotide sequence" means cDNA.

### FUSION PROTEINS

The AR and PR proteins comprising the AR-LBD and PR-LBD of the present invention may also be produced as fusion proteins, for example to aid in extraction and purification. Examples of fusion protein partners include glutathione-S-transferase (GST), 6xHis, GAL4 (DNA binding and/or transcriptional activation domains) and β-galactosidase. It may also be convenient to include a proteolytic cleavage site between the fusion protein partner and the protein sequence of interest to allow removal of fusion protein sequences.

### AMINO ACID SEQUENCES

Preferably the fusion protein will not hinder the ligand binding activity of the AR-LBD and PR-LBD comprising the amino acid sequences (SEQ ID No 1 and SEQ ID No 3 respectively) of the present invention.

Preferably AR-LBD comprises at least SEQ ID No 1, or a homologue or mutant thereof.

Preferably the PR-LBD comprises at least SEQ ID No 3, or a homologue or mutant thereof.

### CRYSTALLISATION

After cleavage of the fusion protein, the AR-LBD and PR-LBD may be separated from the cleavage products by chromatographic methods. Concentration may be performed with the aid of a filtration system and the protein concentrate may be immediatedly used for crystallisation purposes. The protein concentrate may be crystallised using, for example, the vapour diffusion method at a temperature of from about 1°C to about 30°C, preferably from about 4°C to about 20°C. The crystallisation temperature is dependent on the additives present in the protein solution.

Typically, the crystals comprising the AR-LBD are purified to homogeneity for crystallisation. Purity of the AR-LBDs may be measured by typical techniques such as with SDS-PAGE, mass spectrometry and hydrophobic HPLC.

Preferably crystal comprises the AR-LBD or a homologue or mutant thereof.

Preferably the crystal comprises the PR-LBD or a homologue or mutant thereof.

Preferably the crystal is usable in X-ray crystallography techniques. Preferably the crystals used can withstand exposure to X-ray beams used to produce a diffraction pattern data necessary to solve the X-ray crystallographic structure.

Preferably the crystal has a resolution determined by X-ray crystallography of from about 1.5Å to about 3.5Å, preferably about 1.5Å.

Preferably the crystal has a resolution determined by X-ray crystallography of from about 1.5Å to about 3.0 Å.

Preferably the crystal comprising the AR-LBD has the secondary structure presented as SEQ ID No 2, or a homologue or mutant thereof.

The crystal may be formed from an aqueous solution comprising a purified polypeptide comprising an AR-LBD.

The term "purified" in reference to a polypeptide, does not require absolute purity such as a homogenous preparation rather it represents an indication that the polypeptide is relatively purer than in the natural environment. Generally, a purified polypeptide is substantially free of other proteins, lipids, carbohydrates, or other materials with which it is naturally associated, preferably at a functionally significant level for example at least 97.5% pure, more preferably at least 99% pure, most preferably at least 99.5% pure. A skilled artisan can purify a polypeptide comprising an AR-LBD using standard techniques for protein purification. A substantially pure polypeptide comprising an AR-LBD will yield a single major band on a non-reducing polyacrylamide gel. The purity of the AR-LBD can also be determined by amino-terminal amino acid sequence analysis.

The term "associate", "association" or "associating" refers to a condition of proximity between a moiety (i.e. chemical entity or compound or portions or fragments thereof), and an AR-LBD, or parts or fragments thereof (e.g. binding sites or domains). The association may be non-covalent i.e. where the juxtaposition is energetically favored by for example, hydrogen-bonding, van der Waals, or electrostatic or hydrophobic interactions, or it may be covalent.

### ANDROGEN

As used herein, the term "androgen refers to any substance, natural or synthetic, that is able to stimulate the development of male sexual characteristics. Naturally occuring androgens are represented by the C₁₉-steroid hormones. They are produced especially by the testis (such as testosterone) and also by the adrenal cortex, ovary and the placenta. As used herein, the term "androgen" relates to the male sex steroids, dihydrotestosterone (DHT) and testosterone [Teutsch, 1994] which bind to the AR-LBD and which regulate the genes for male differentiation and development.

### ANDROGEN RECEPTOR

As used herein, the term "androgen receptor (AR)" means any of the androgen-binding nuclear proteins that mediate the effects of androgens by regulating gene expression. The androgen receptor proteins are discrete zinc-finger proteins which bind discrete DNA sequences, located upstream of transcriptional start sites, when an AR-ligand complex is formed. The androgen receptor (AR) binding domain, also known as the androgen receptor ligand binding domain (AR-LBD), or the hormone binding domain (HBD), is in the C-terminal region. In humans, a number of variants are known that are associated with abnormalities, including prostate cancer (PC), testicular feminisation syndrome, complete androgen insensitivity syndrome (CAIS) and/or partial androgen insensitivity syndrome (PAIS) and/or mild androgen insensitivity syndrome (MAIS) which may lead to external genitalia varying between female and nearly normal male.

As used herein, the term "androgen receptor" means the wild type androgen receptor or a mutant androgen receptor.

### WILD TYPE

The term "wild type" refers to the phenotype that is characteristic of most of the members of a species occuring naturally and which contrasts with the phenotype of a mutant species. As used herein, the term "wild type androgen receptor" refers to the an androgen receptor comprising the amino acid sequence presented as SEQ ID No 1. In particular, the term "wild type androgen receptor" refers to the androgen receptor comprising a ligand binding pocket (LBP) wherein the LBP is defined by the structural co-ordinates of the AR-LBD amino acid residues L701; L704; N705; L707; Q711; M742; L744; M745; M749; R752; F764; Q783; M787; F876; T877; L880; F891; M895 or a homologue thereof.

### MUTANT

As used herein, the term "mutant" refers to any organism that has undergone mutation or that carries a mutant gene that is expressed in the phenotype of that organism. A mutation may arise due to a substitution of one nucleotide for another or from a deletion of a nucleotide or an insertion of a nucleotide relative to a referenced wild type sequence. These single nucleotide variations are sometimes referred to as single nucleotide polymorphisms (SNPs). Some SNPs may occur in protein-coding sequences, in which case, one of the polymorphic forms may give rise to the expression of a defective or other variant protein and, potentially, a genetic disease. Other SNPs may occur in noncoding regions. Some of these polymorphisms may also result in defective protein expression (e.g., as a result of defective splicing). Other SNPs may have no phenotypic effects.

As used herein, the term "mutant" refers to an androgen receptor comprising any one or more changes in the sequence (and/or the structural co-ordinates) and of the amino acid residues in the AR-LBD which interact with bound ligand wherein the amino acid changes in the AR-LBD may be selected from any one or more of the group of LBD amino acid residues substitutions consisting of L701H; M749I; T877A; T877S; L880Q; F891L;N705S; L707R; M749V; G708A; G708V; M742V; M742I; M745T; V746M; R752Q; F764S; M787V. In this regard, the sequence and amino acid residues (such as L701H) are described using the one letter format for the amino acid residue (such as L), followed by the amino acid designations nunber which refers to the amino acid residue in the wild type sequence directly above the last digit, followed by the mutant amino acid residue (here a substituted amino acid residue) which is also described using the one letter format for the amino acid residue (in this case H).

For some embodiments the androgen receptor may comprise two or more mutated amino acid residues. An example of such an embodiment is L701H and T877A.

The term "mutant" is not limited to the above mutations which are reflected in amino acid substitutions of the key amino acid residues in the AR-LBD but may also include and is not limited to other deletions or insertions of nucleotides in the wild type sequence which may result in changes in the amino acid residues in the deduced amino acid sequence of the AR-LBD. The term "mutant" also includes uncharacterised mutants.

Preferably the mutated androgen receptor comprises one or more of the characterised mutations in the LBP of the AR-LBD as set out in Table 3.

Preferably the mutated amino acid residue(s) is/are located in helices H4 and H5 of the AR-LBD.

Preferably the mutated amino acid residue(s) is/are evenly distributed between buried, medium and fully accessible amino acid residues within the ligand binding pocket (LBP) comprising the AR-LBD.

Preferably the mutated amino acid residue(s) is/are distributed as set out in Figure 1.

### STRUCTURAL CO-ORDINATES

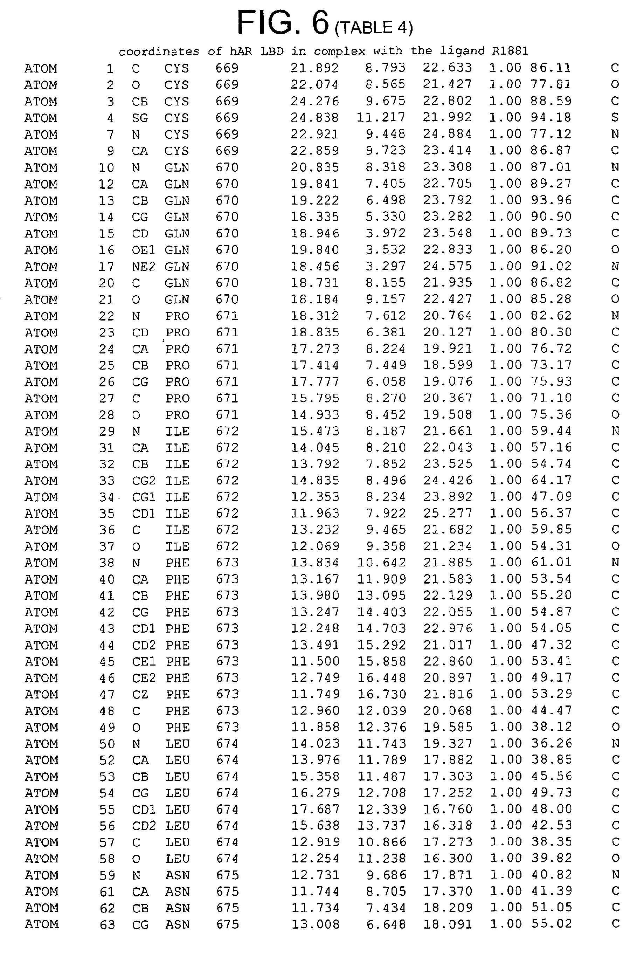

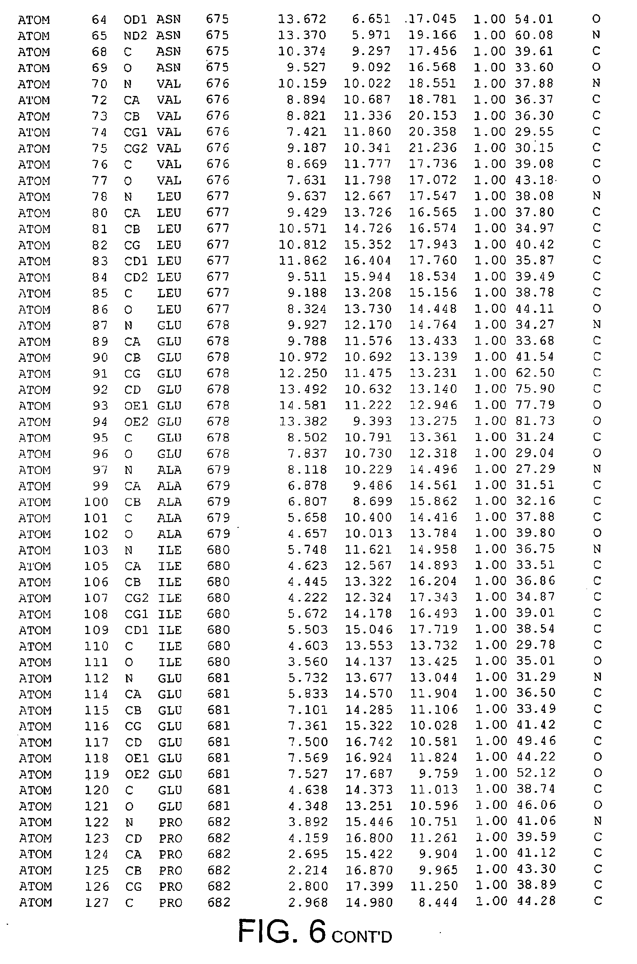

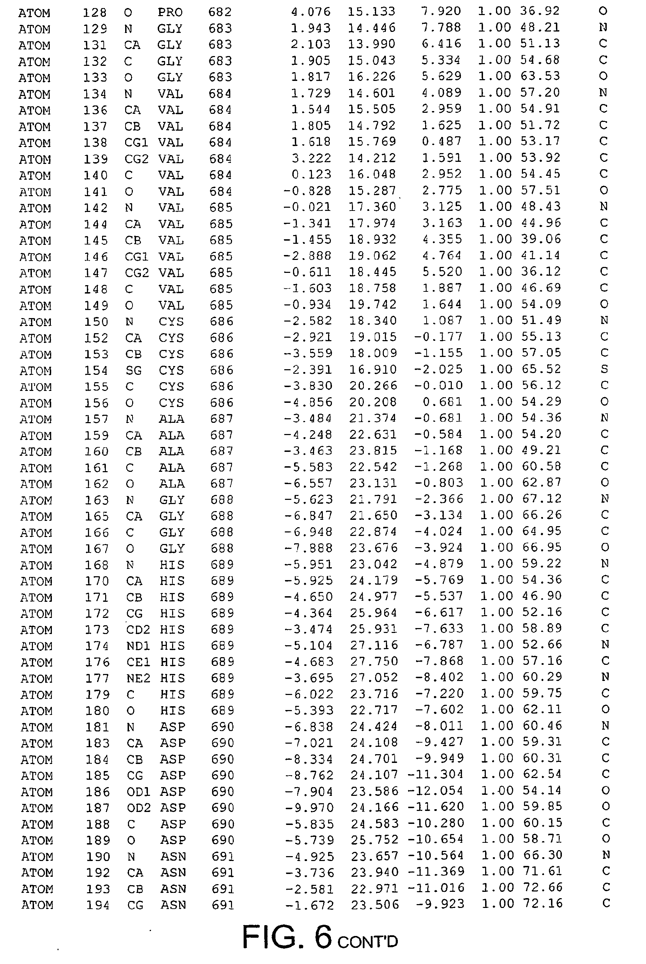

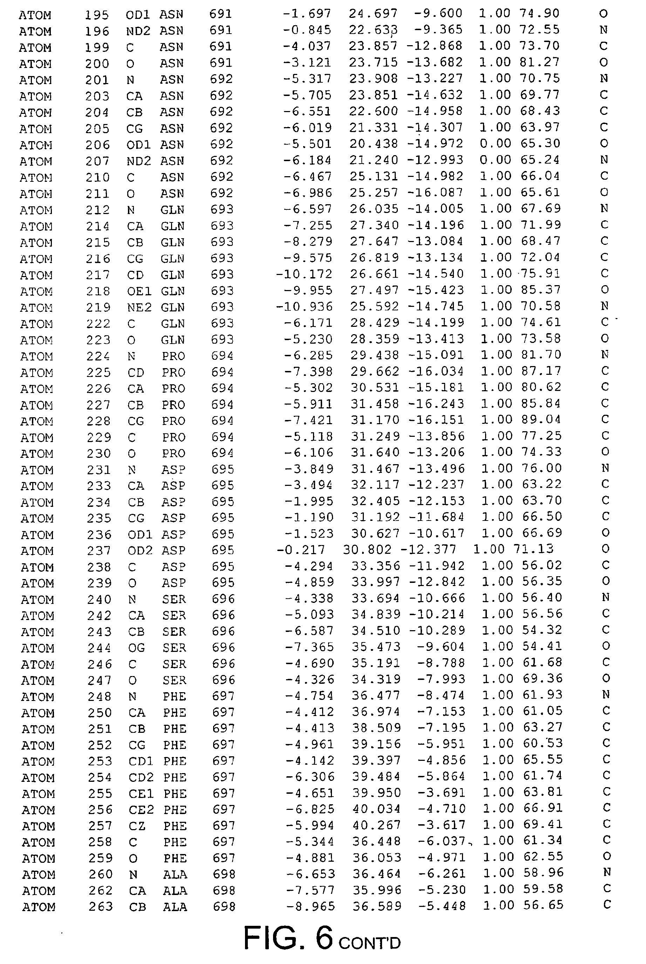

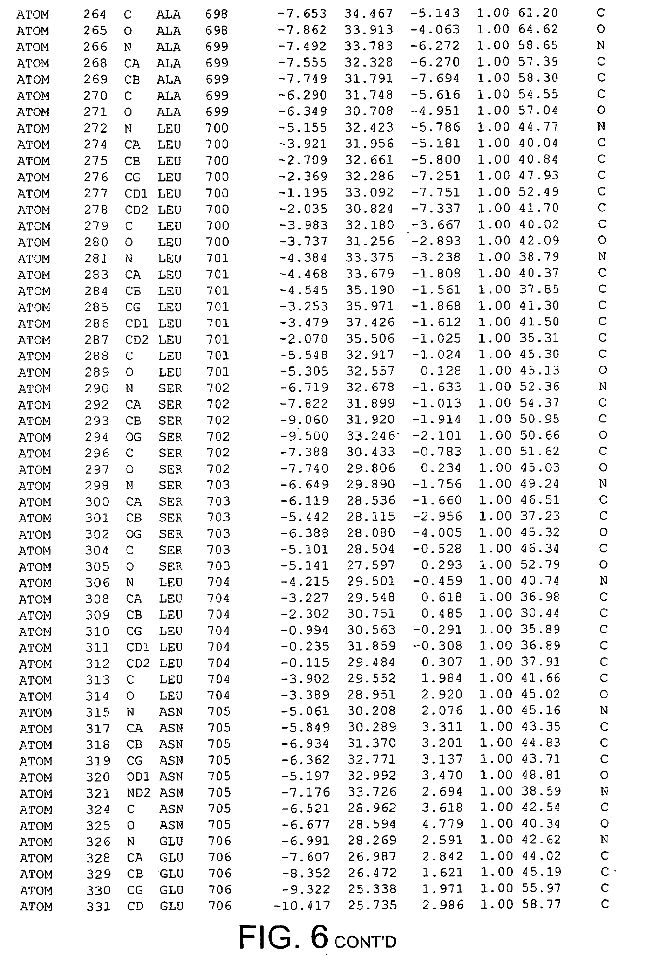

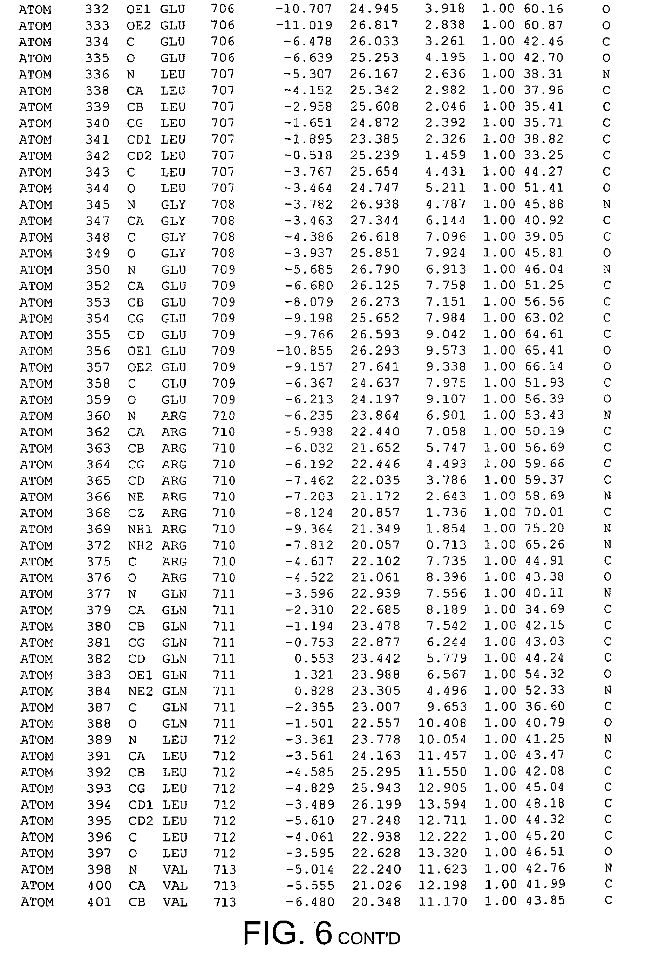

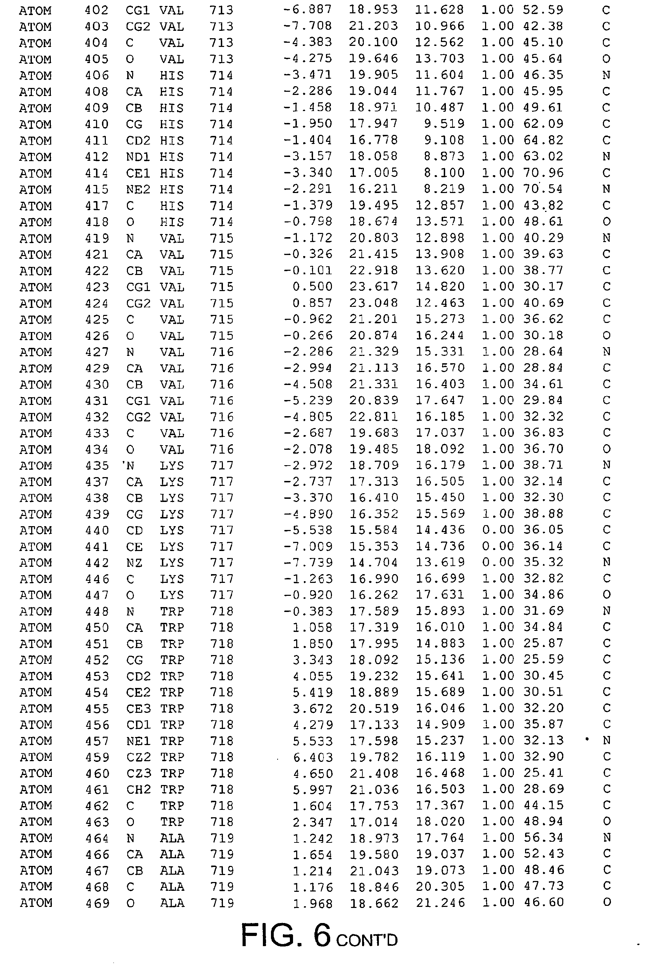

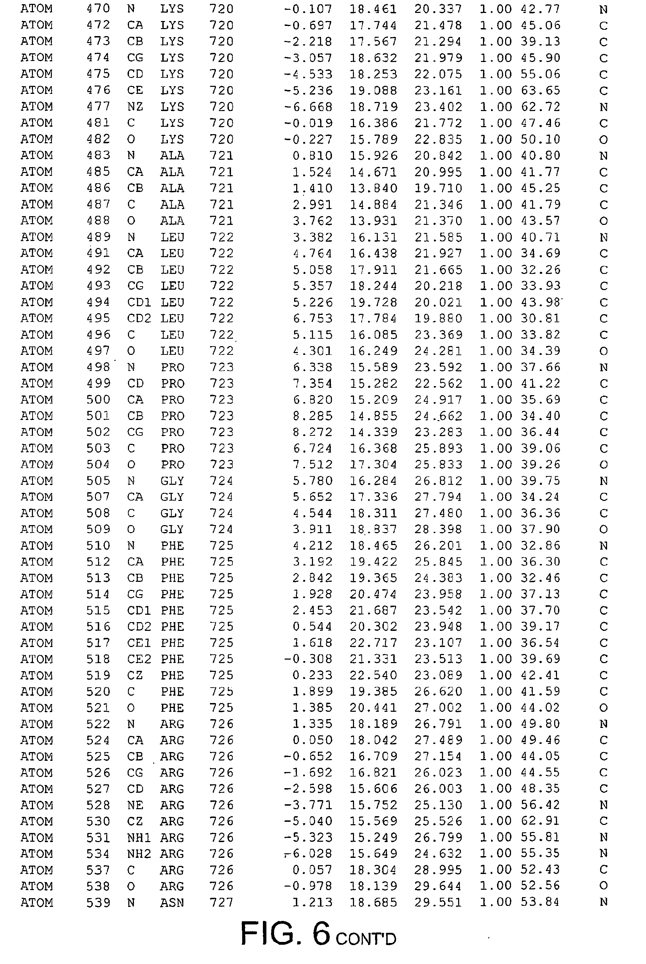

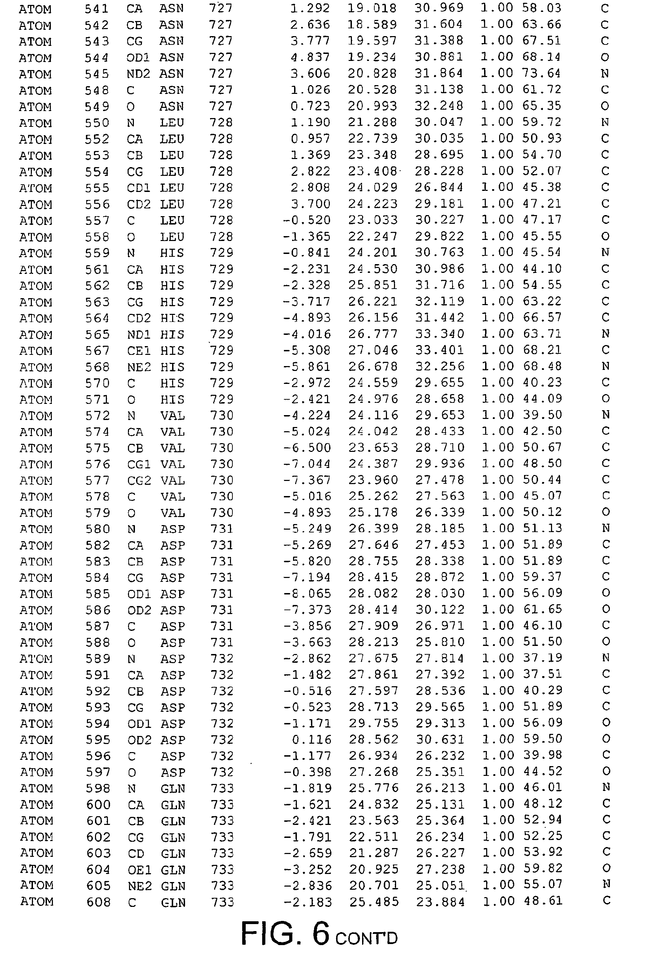

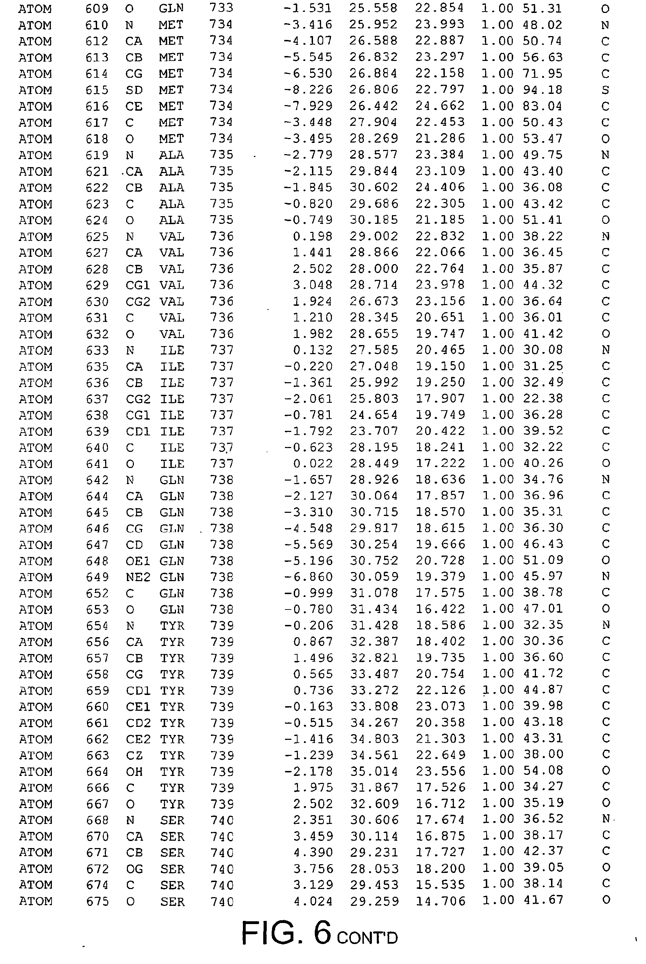

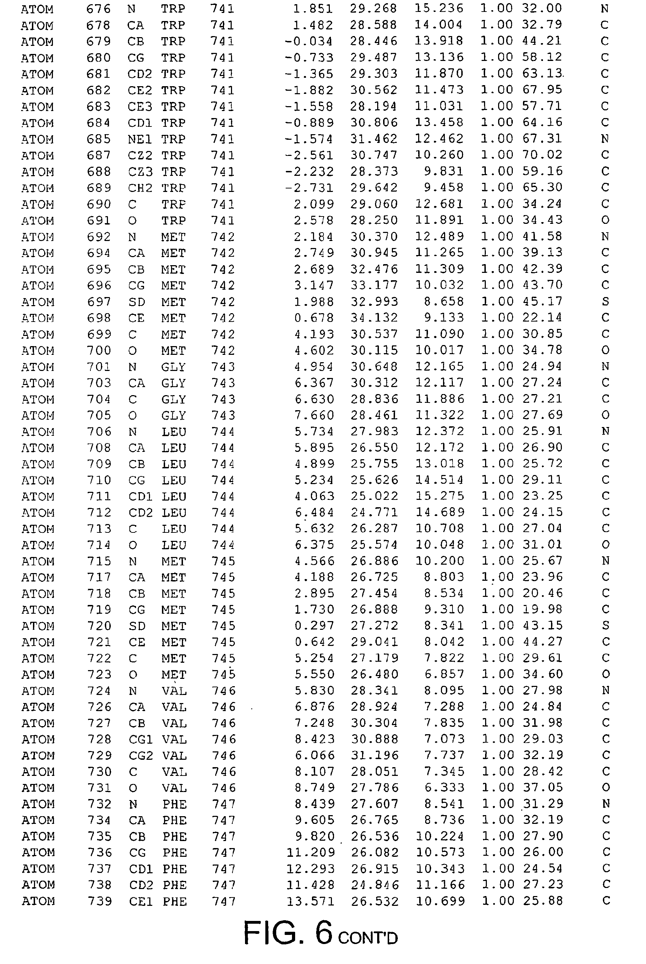

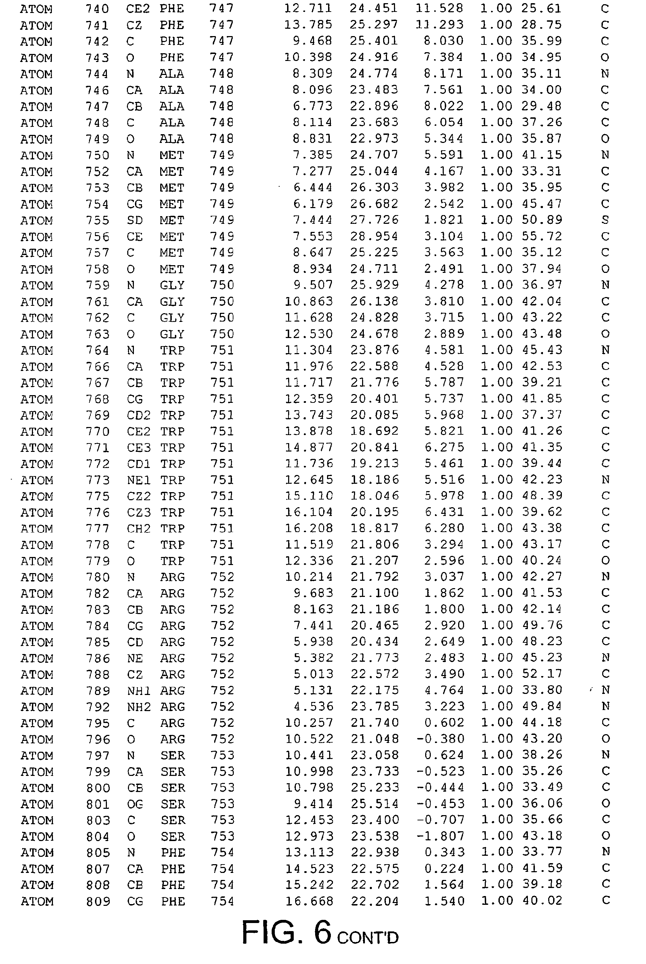

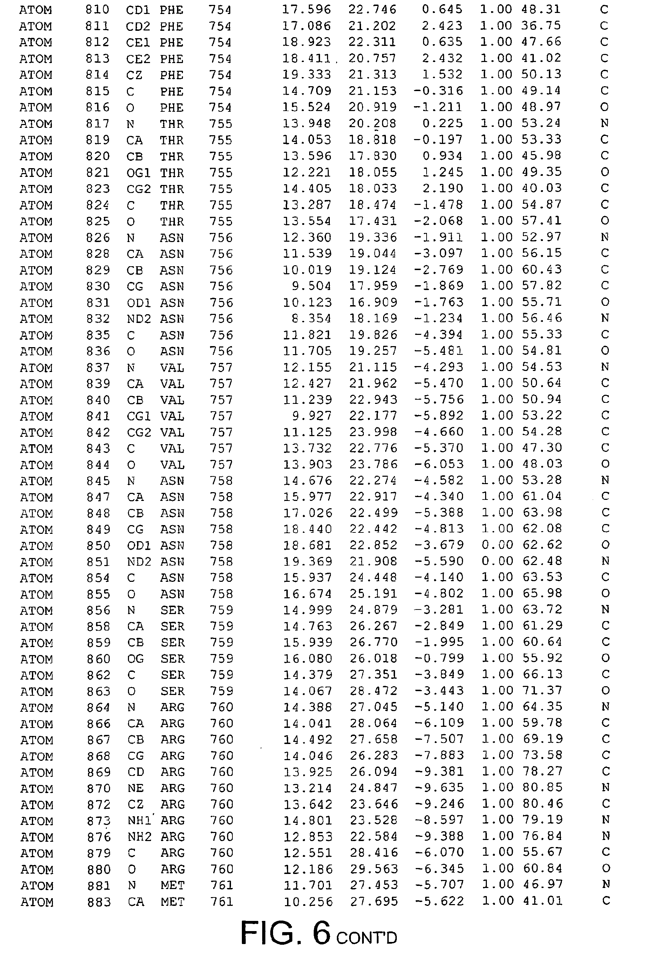

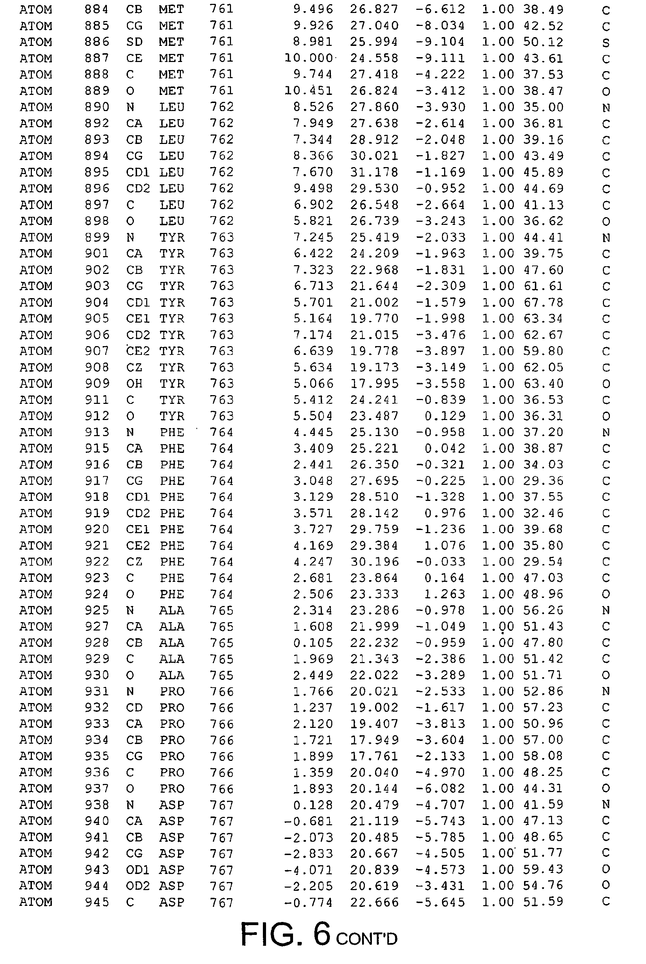

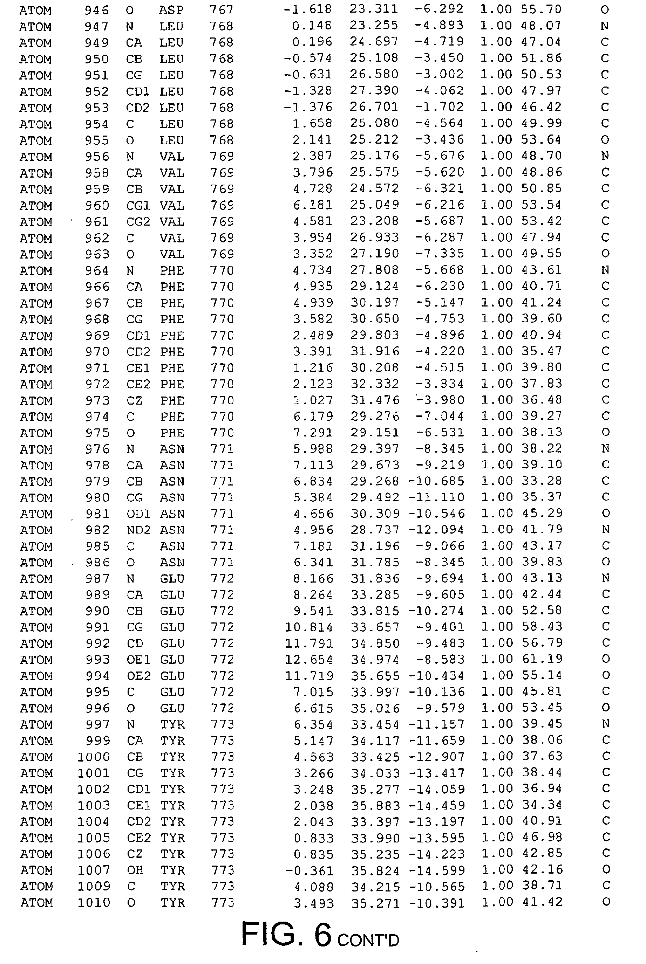

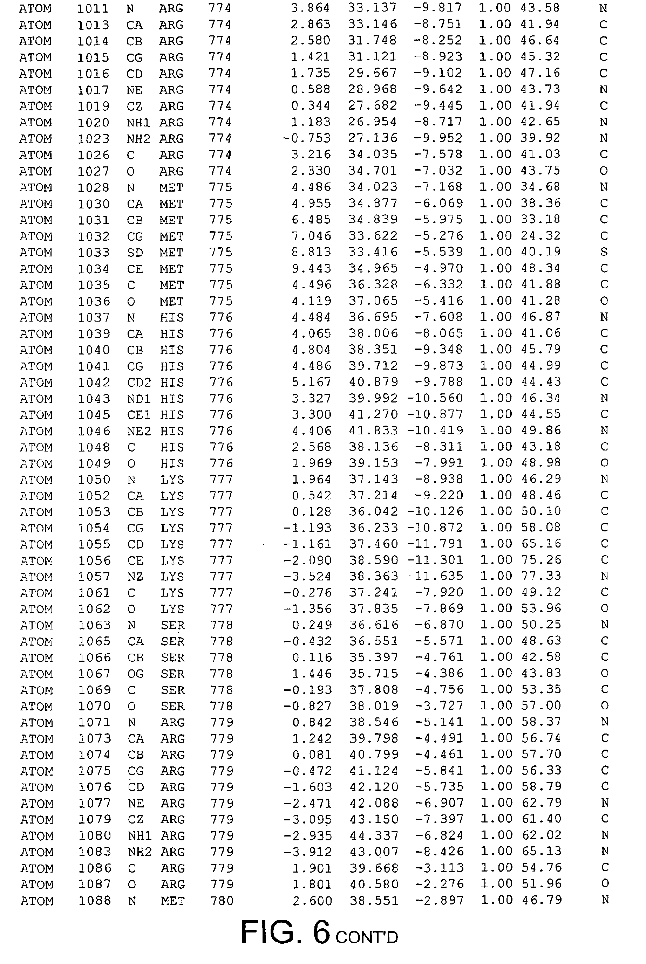

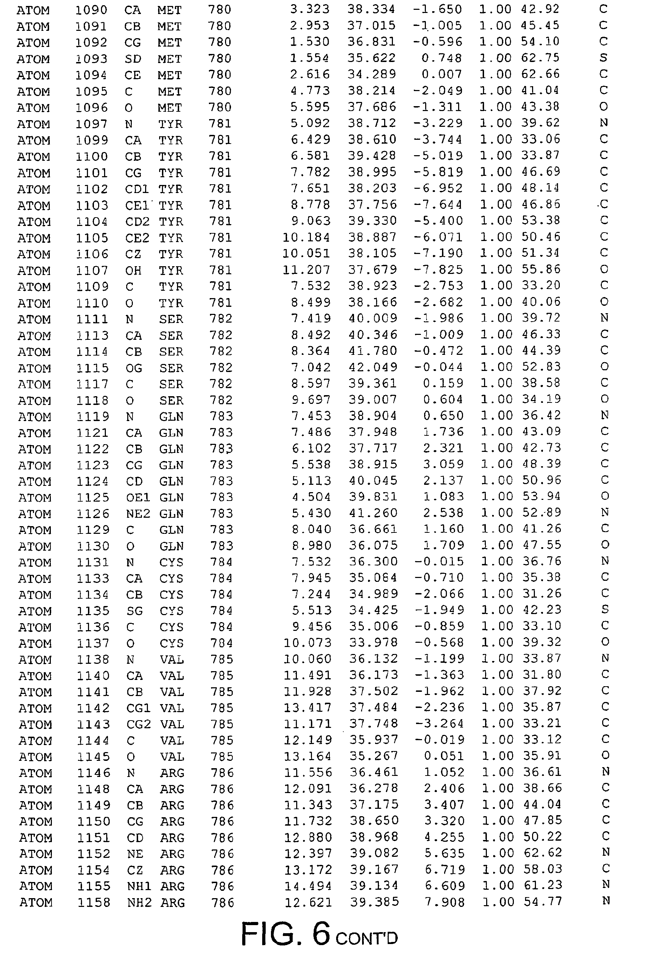

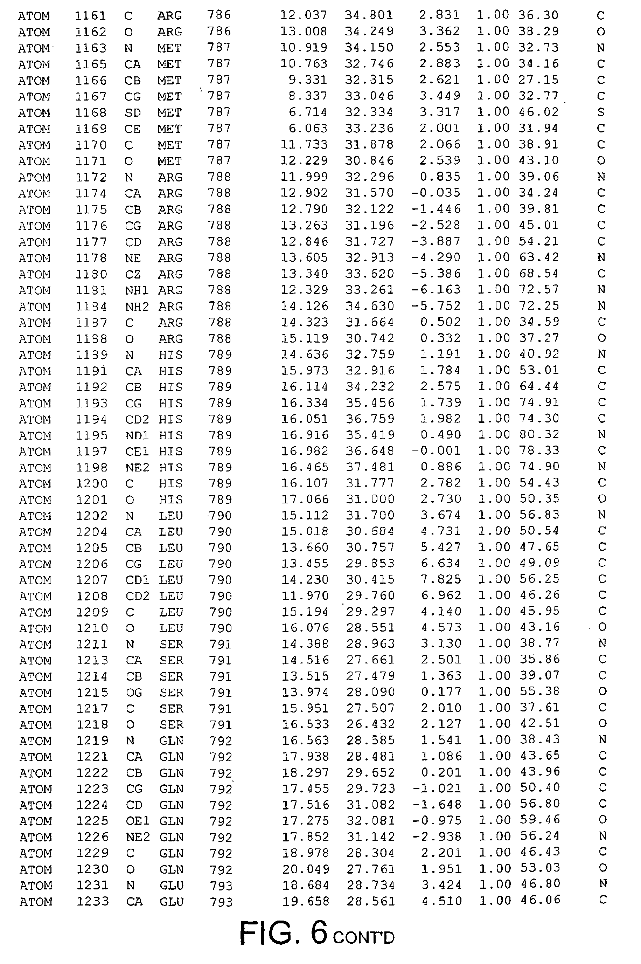

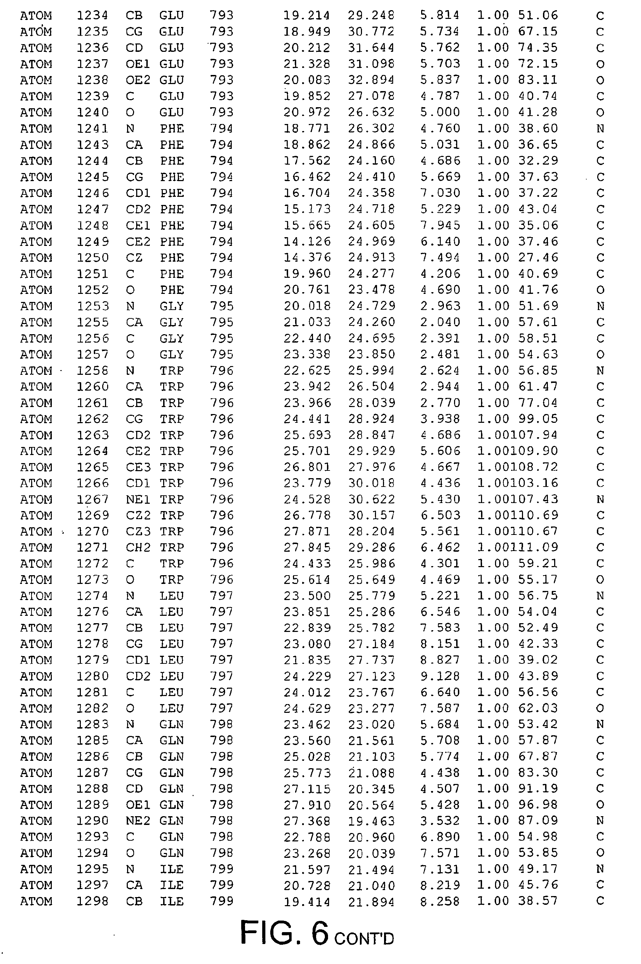

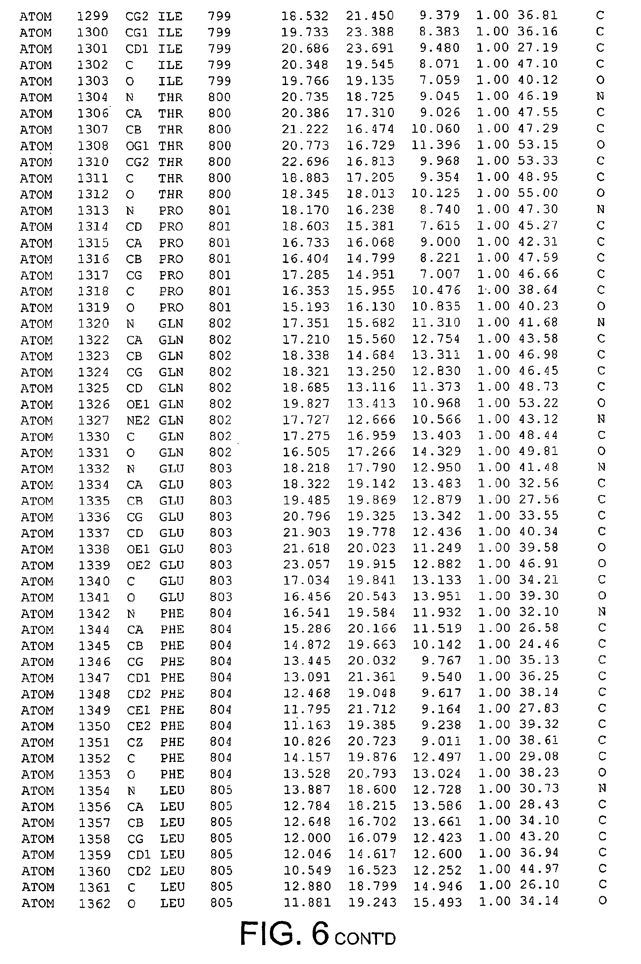

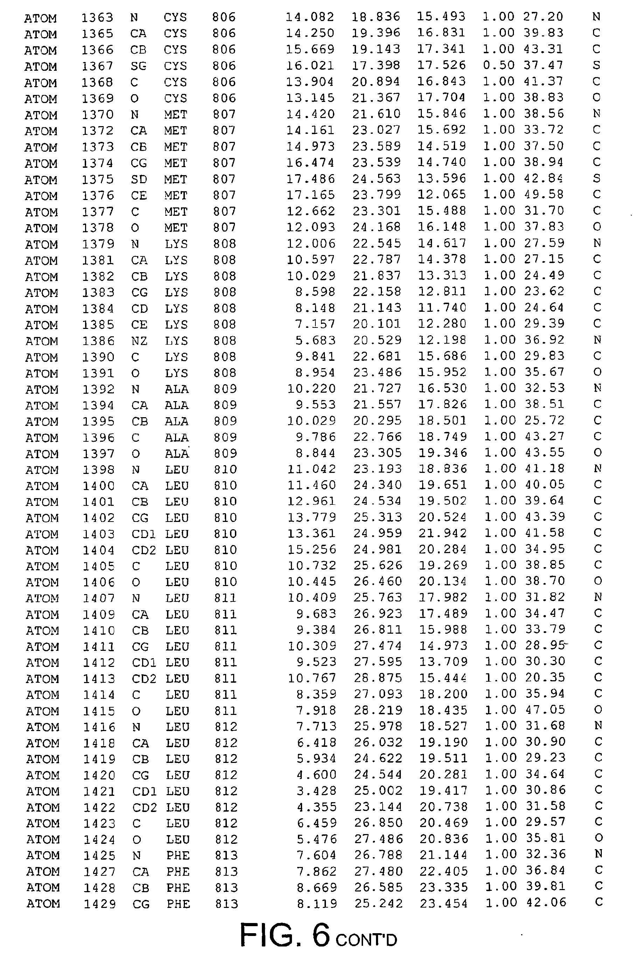

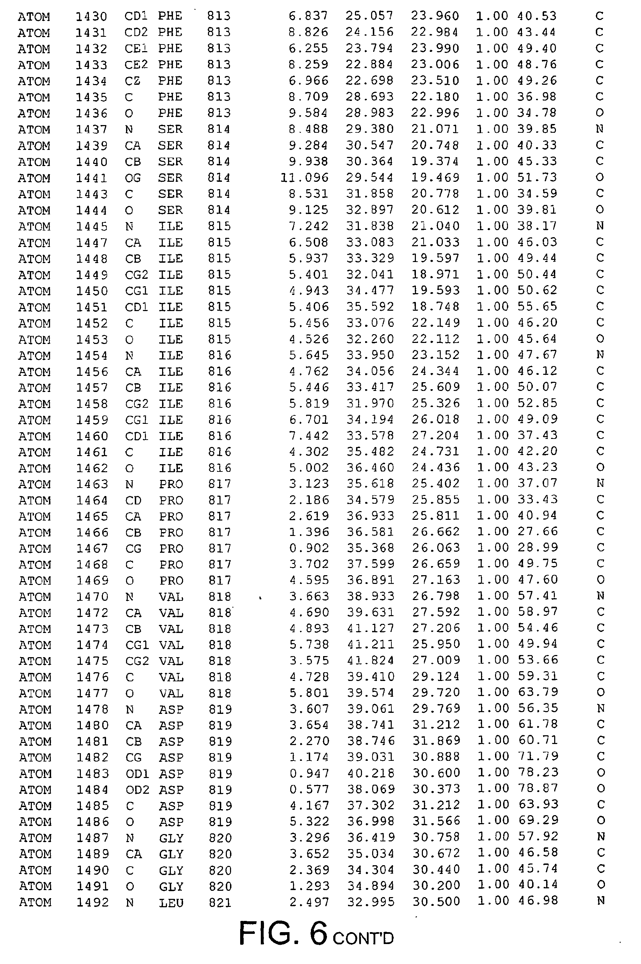

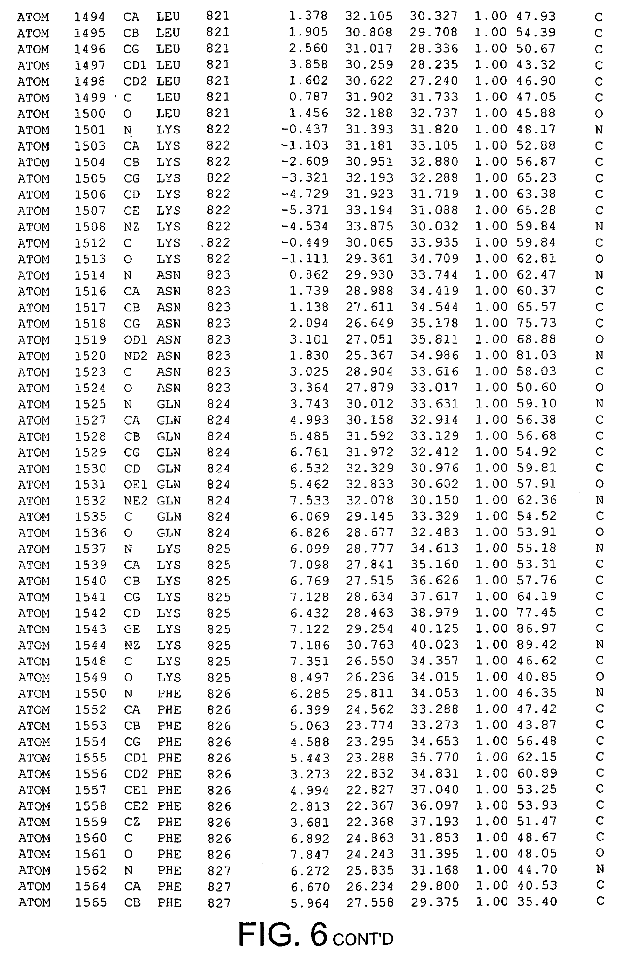

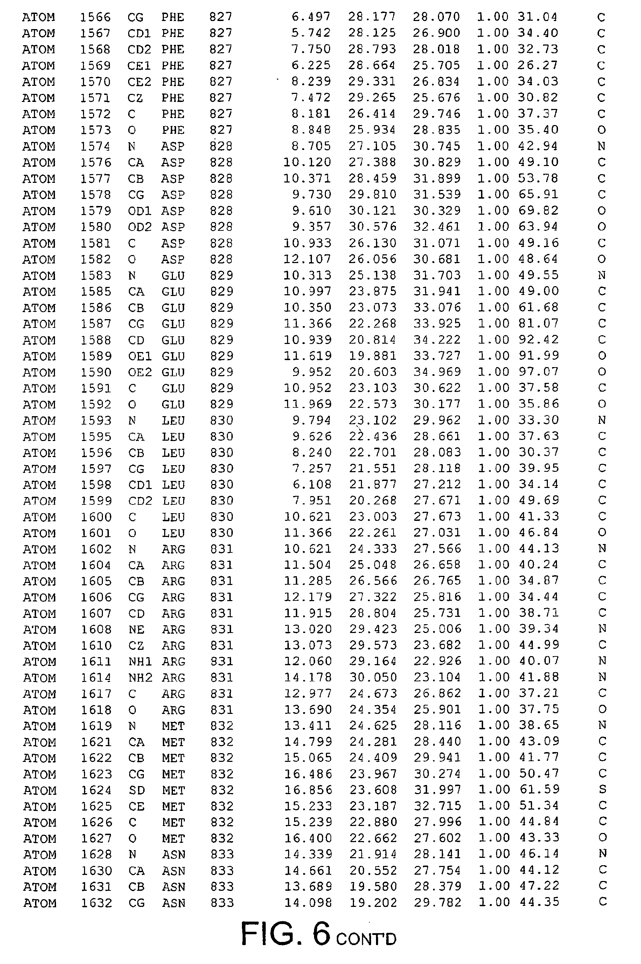

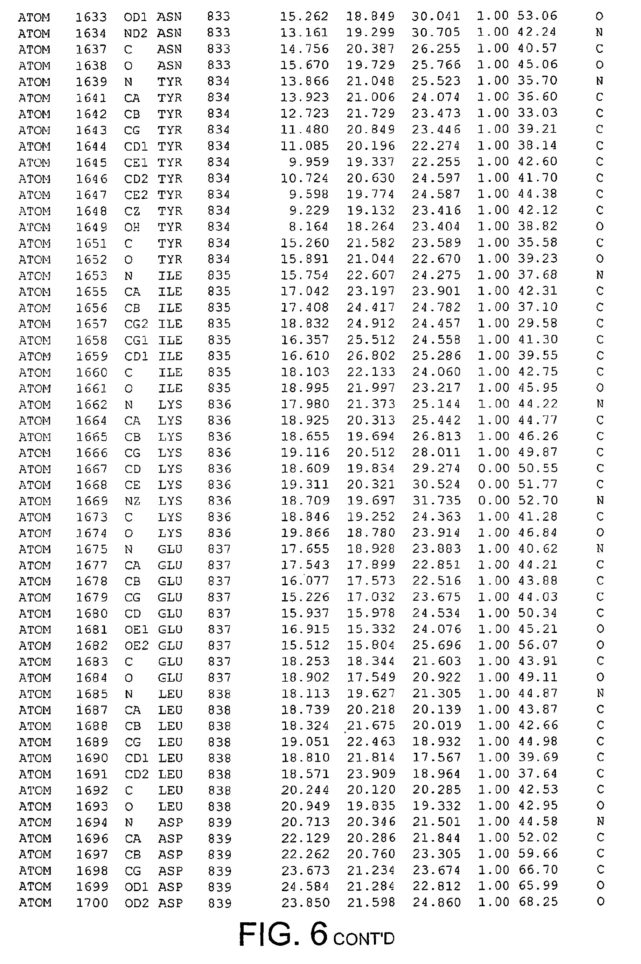

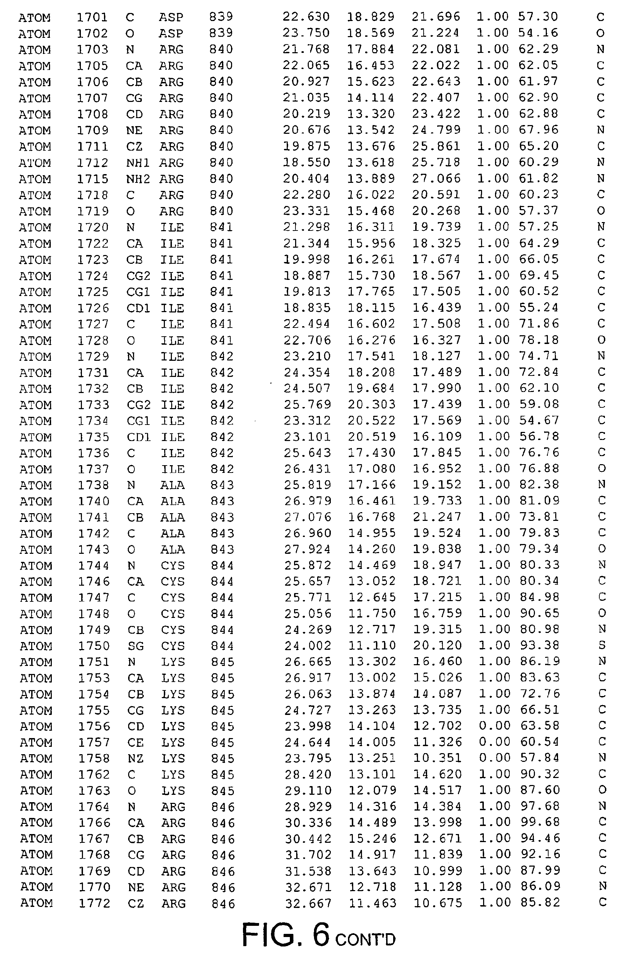

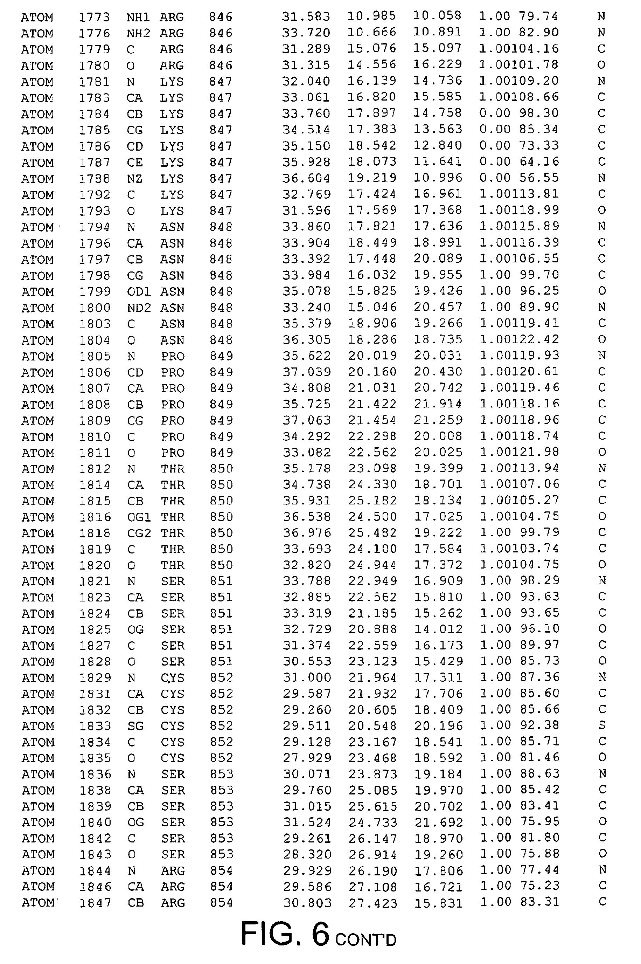

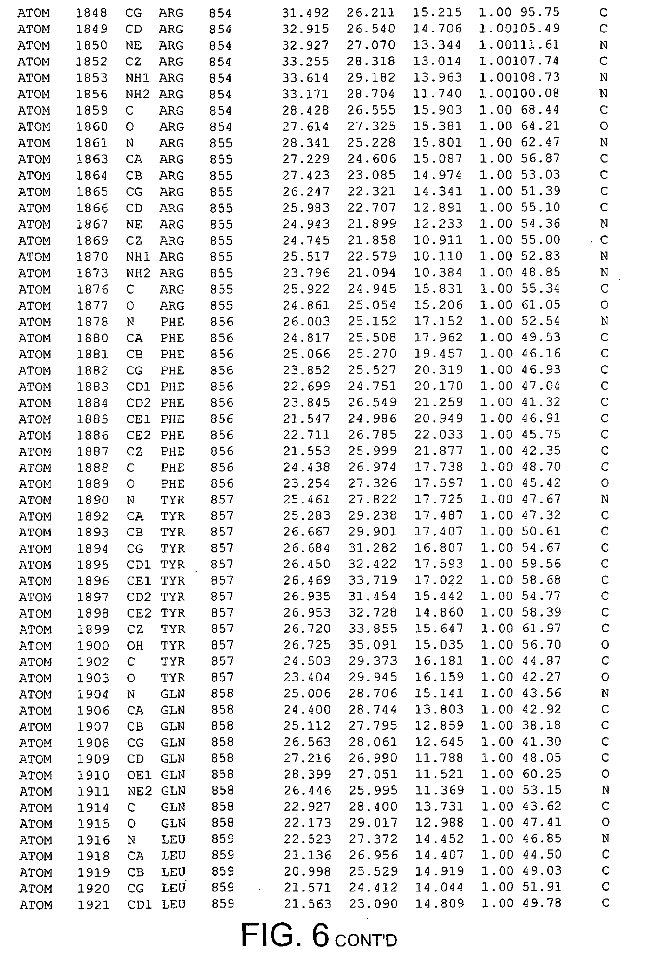

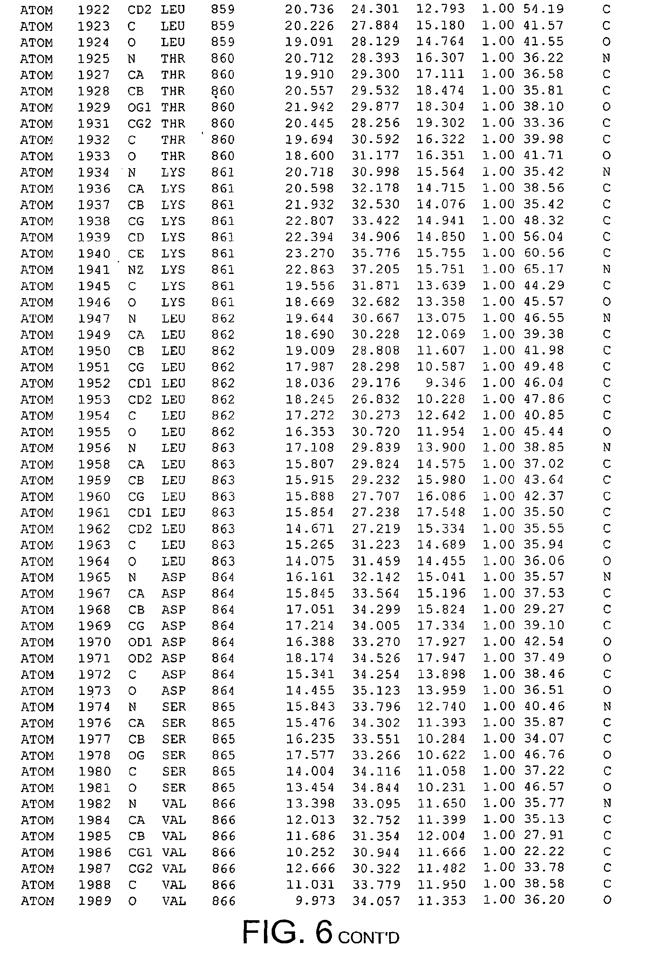

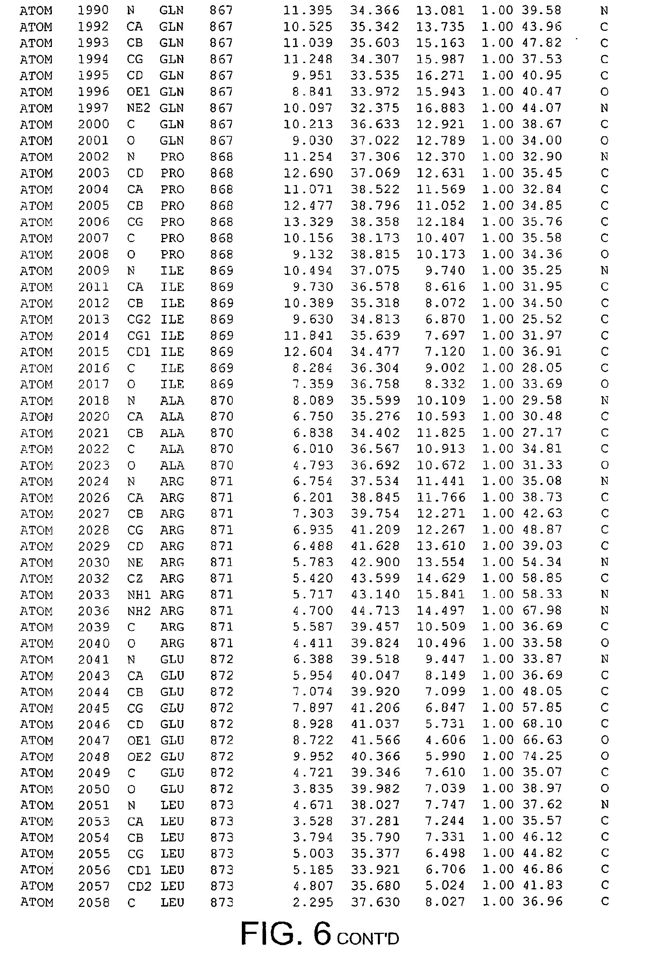

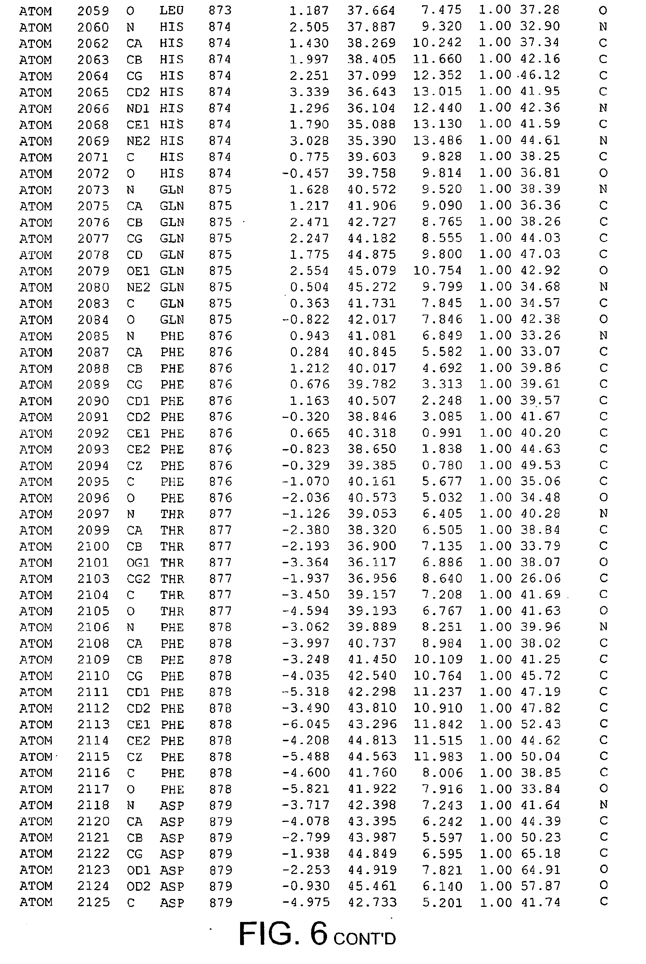

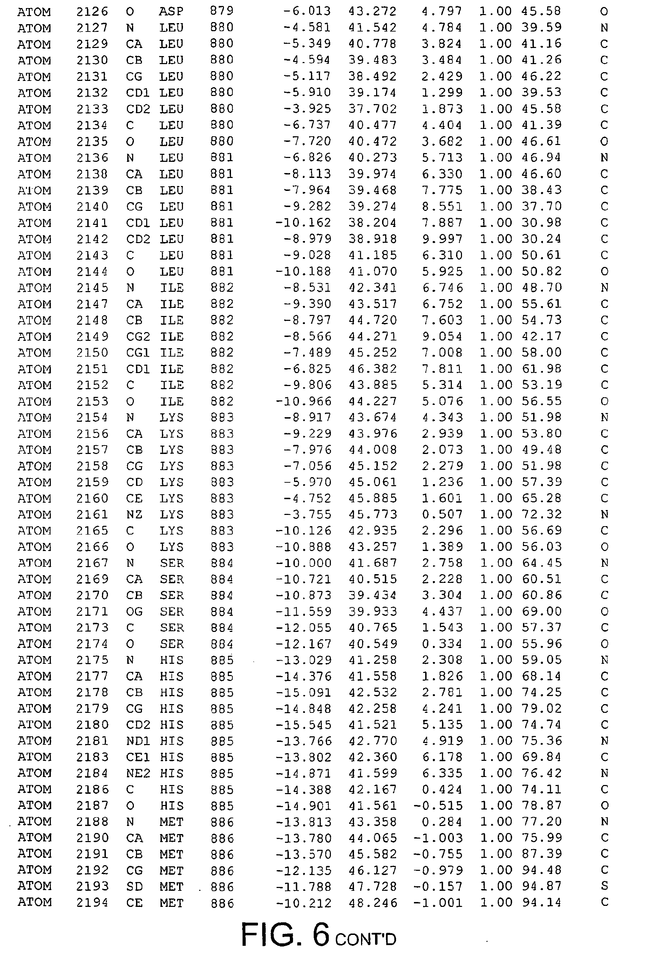

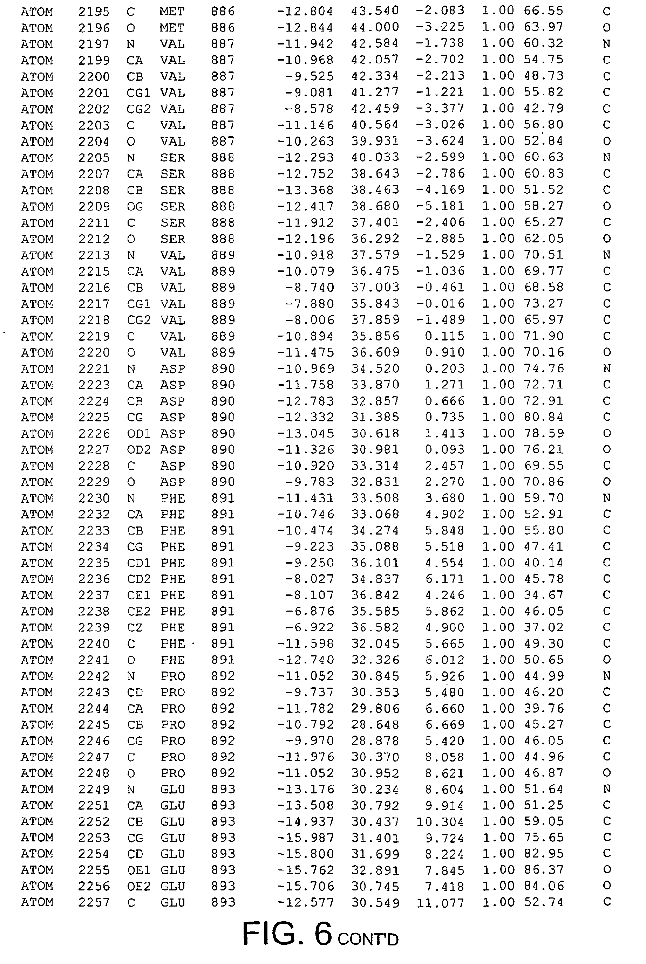

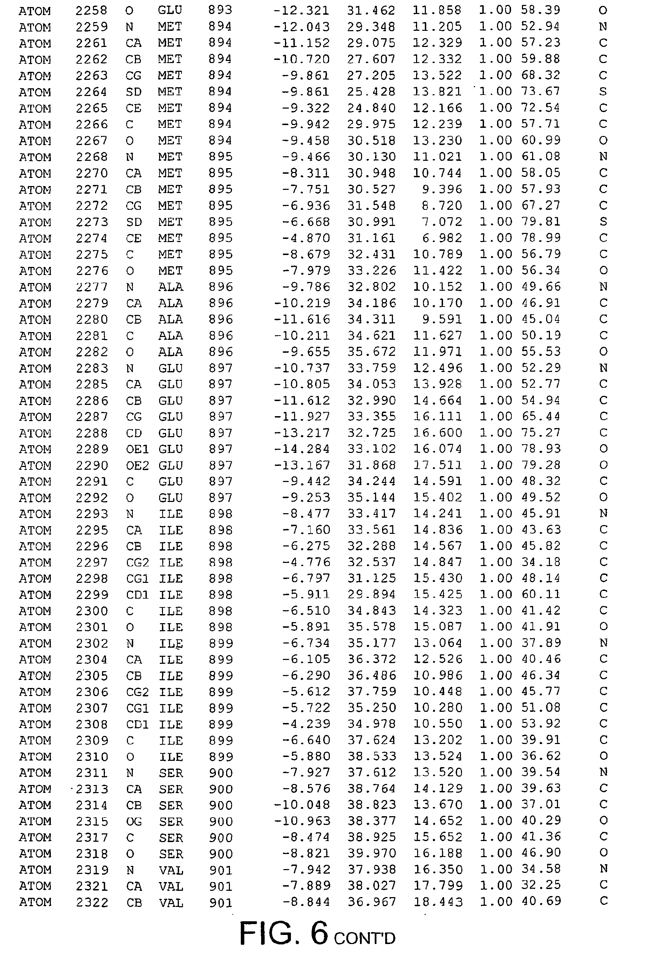

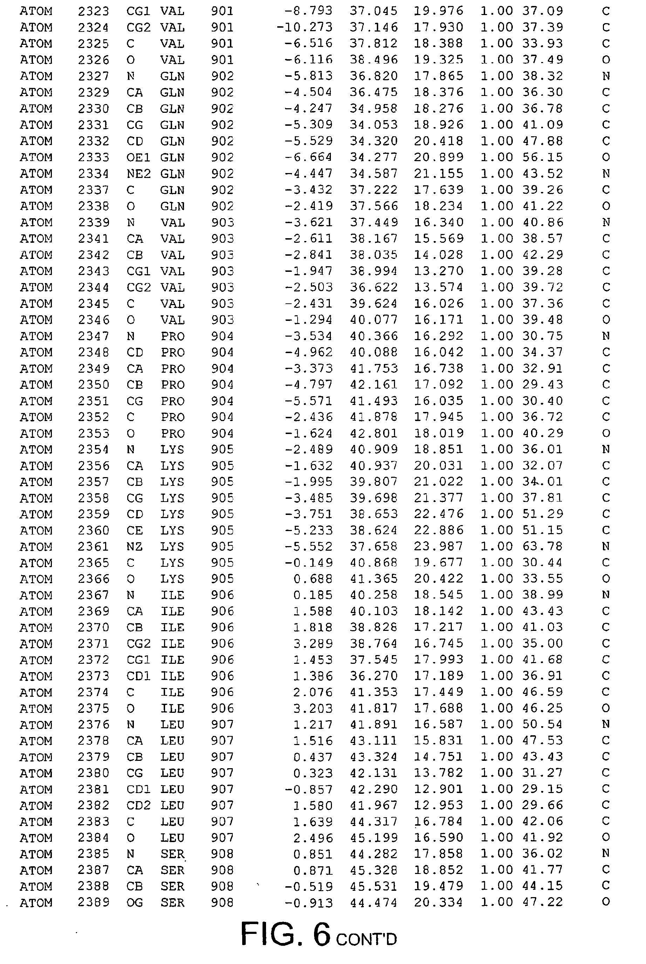

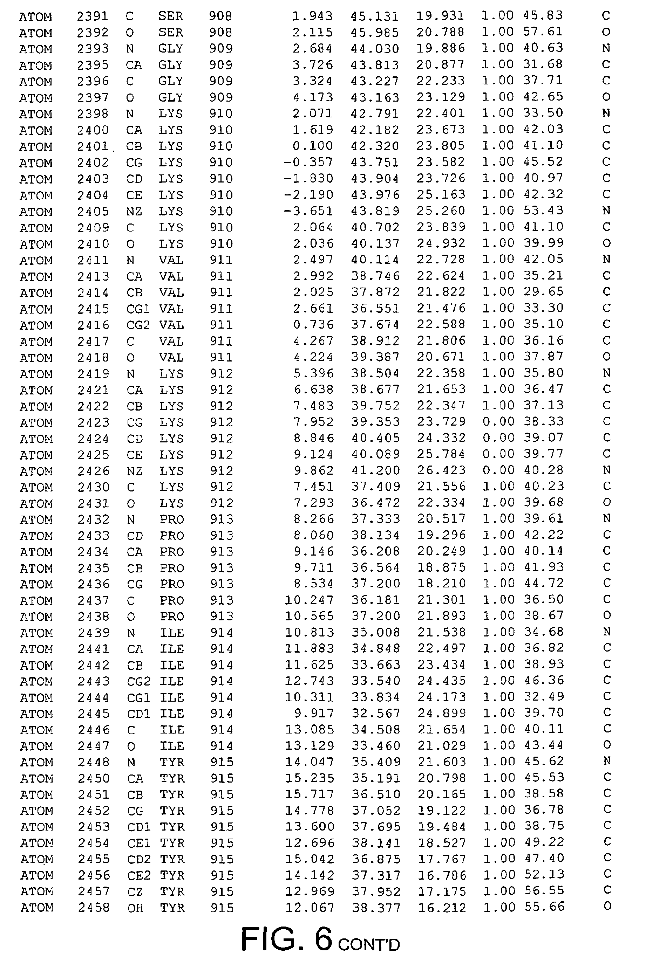

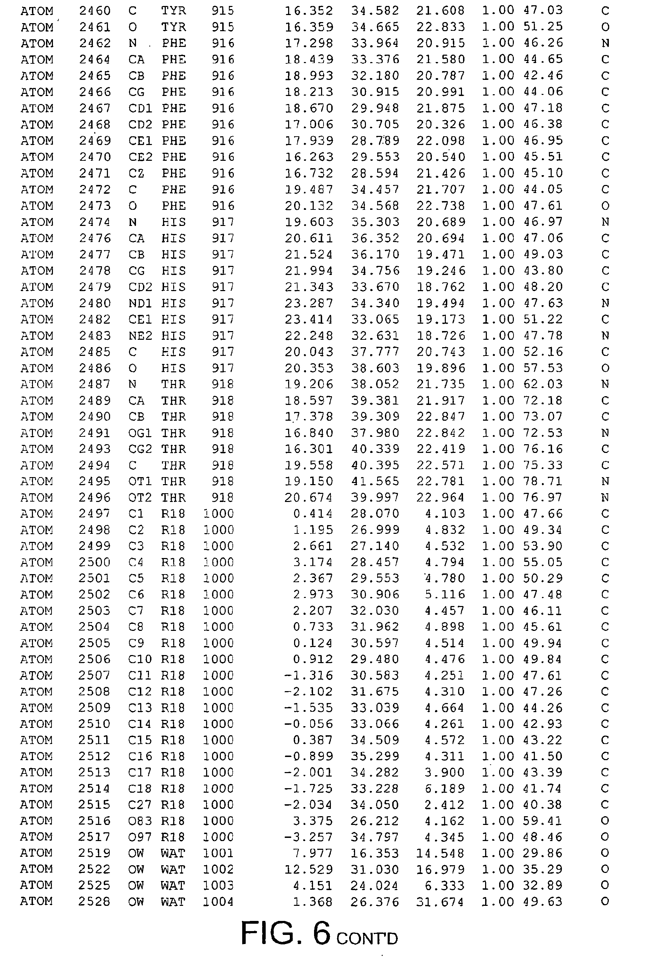

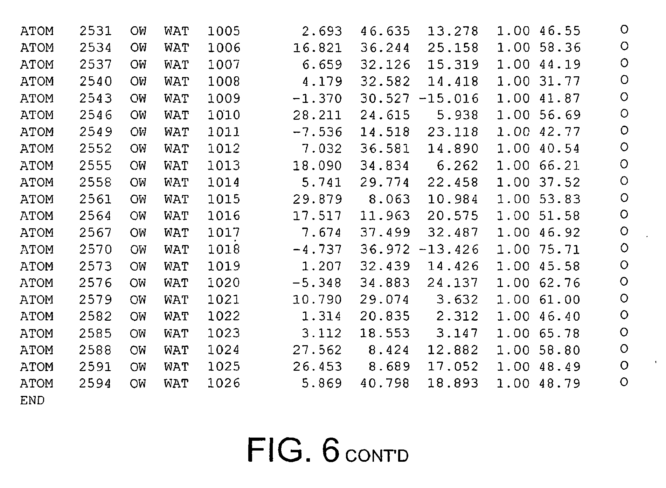

In a highly preferred embodiment, the crystal has the structural co-ordinates as provided in Table 4 (Figure 6) which may be used for the identification of a ligand capable of binding to the AR-LBD.

As used herein, the term "structural co-ordinates" refer to a set of values that define the position of one or more amino acid residues with reference to a system of axes. The term refers to a data set that defines the three dimensional structure of a molecule or molecules (e.g. Cartesian coordinates, temperature factors, and occupancies). Structural coordinates can be slightly modified and still render nearly identical three dimensional structures. A measure of a unique set of structural coordinates is the root-mean-square deviation of the resulting structure. Structural coordinates that render three dimensional structures (in particular a three dimensional structure of an SGC domain) that deviate from one another by a root-mean-square deviation of less than 5 Å, 4 Å, 3 Å, 2 Å, or 1.5 Å may be viewed by a person of ordinary skill in the art as very similar.

According to one aspect of the present invention, there is provided a crystal comprising a complex between an androgen receptor ligand-binding domain and a ligand. In other words the androgen receptor ligand binding domain may be associated with a ligand in the crystal. The ligand may be any compound which is capable of interacting stably and specifically with the androgen receptor ligand binding domain. The ligand may, for example, be an inhibitor of the AR-LBD.

### LIGAND-BINDING DOMAIN

As used herein, the term "ligand binding domain (LBD)" means the C-terminal ligand binding region of a steroid receptor which is responsible for ligand binding. The term "ligand binding domain (LBD)" also includes a homologue of the ligand binding domain or a portion thereof. The LBD of the present invention comprises a ligand binding pocket (LBP).With reference to the crystal of the present invention residues in the LBD may be defined by their spatial proximity to the ligand in the crystal structure. The term "ligand binding domain (LBD)" also includes a homologue of the ligand binding domain or a portion thereof.

As used herein, the term "portion thereof' means the structural co-ordinates corresponding to a sufficient number of amino acid residues of AR-LBD (or homologues thereof) that are capable of interacting with a test compound capable of binding to the LBD. This term includes AR- ligand binding domain amino acid residues having an amino acid residues from about 4Å to about 5Å of a bound compound or fragment thereof. Thus, for example, the structural co-ordinates provided in the crystal structure may contain a subset of the amino acid residues in the LBD which may be useful in the modelling and design of compounds that bind to the LBD.

The ligand binding domain may be defined by its association with the ligand.

Preferably the ligand binding domain comprises one or more amino acid residues as determined from the crystal structure or a homologue thereof. Examples of such amino acid residues are presented herein.

### LIGAND BINDING POCKET (LBP)

According to one aspect of the present invention, there is provided a crystal structure comprising a ligand binding pocket (LBP); wherein the LBP is defined by the following amino acid residue structural co-ordinates: L701; L704; N705; L707; Q711; M742; L744; M745; M749; R752; F764; Q783; M787; F876; T877; L880; F891; M895; or a homologue thereof.

As used herein, the term "ligand binding pocket (LBP)" refers to the cavity or hollow in a structure - typically a three-dimensional (3D) structure - in which a ligand binds and in which is located the ligand binding domain (LBD). The LBP is sometimes referred to as a "binding niche". In particular, preferaby, the term AR-LBP refers to the 18-20 known amino acid residues in the hAR-LBD which are known to interact with bound ligand (either R1881 or progesterone). These residues are highlighted in Figure 1 and included in Figure 4. Most of these residues are hydrophobic and interact mainly with the steroid scaffold, while a few are polar and may form hydrogen bonds to the polar atoms in the ligand.

### POLAR AMINO ACIDS

As used herein, the term "polar" includes positively and negatively charged amino acids. In this respect, negatively charged amino acids include aspartic acid (D) and glutamic acid (E); positively charged amino acids include lysine (K) and arginine (R); and amino acids with uncharged polar head groups having similar hydrophilicity values include leucine (L), isoleucine (I), valine (V), glycine (G), alanine (A), asparagine (N), glutamine (Q), serine (S), threonine (T), phenylalanine (F), and tyrosine (Y). The classification of these amino acid residues is set out in the Table below.

### HOMOLOGUE

As used herein, the term "homologue" refers to an AR-LBD or a portion thereof which may have deletions, insertions or substitutions of amino acid residues as long as the binding specificity of the AR-LBD is retained. In this regard, deliberate amino acid substitutions may be made on the basis of similarity in polarity, charge, solubility, hydrophobicity, hydrophilicity, and/or the amphipathic nature of the residues as long as the binding specificity of the AR-LBD is retained. Here, a conservative substitution which may produce a silent change which may result in a functionally equivalent AR-LBD.

As used herein, the term "homologue" also means a homologue of the crystal structure of the AR-LBD wherein the homologue has a root mean square (r.m.s) deviation from the backbone atoms of amino acid residues in secondary structural elements of less than 3.0Å. Preferably the r.m.s deviation from the backbone atoms of amino acid residues in the secondary structural elements is less than 2.0Å.

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

Abbreviations for amino acid residues are the standard 3-letter and/or 1-letter codes used in the art to refer to one of the 20 common L-amino acids.

### SECONDARY STRUCTURE

The AR-LBD of the present invention is arranged in an α-helical sandwich. The AR-LBD comprises preferably eleven α-helices (H1, H3, H4, H5, H6, H7, H8, H9, H10, H11, H12). There is no H2 helix. Because both helices H4 and H5 and helices H11 and H12 are contiguous helices, the α-helical sandwich is regarded as comprising 9 α-helices and not 11 α-helices. The α- helices designated by the letter H in Figure 1. The helix number (such as H1) is indicated in black above the relevant helical sequence. The α-helical sandwich fold may further comprise preferably 3₁₀ helices and preferably four short β strands (S1, S2, S3 and S4) associated in two anti-parallel β-sheets. The β strands are indicated by the letter E in Figure 1. The strand number (such as S1) is indicated in black above the relevant β sheet.

### ALPHA HELIX (α-Helix)

As used herein, the term "α-helix" means a helical or spiral configuration of a polypeptide chain in which successive turns of the helix are held together by hydrogen bonds between the amide (peptide) links, the carbonyl group of any given residue being hydrogen-bonded to the amino group of the third residue behind it in the chain. This is the case for all of the carbonyl and amide groups of the peptide bonds of the main chain. Typically, the α-helix has 3, 6 residues per turn and the translation or pitch along the helical axis is 1.5Å per residue and 5.4Å per turn. The helix may be left- or right-handed, the latter being much more common. The α-helix is one of the two basic elements of the secondary structure adopted by the polypeptide chain within the hydrophobic core of a globular protein. The other basic element is the β strand.

The AR-LBD of the present invention comprises a helix in the region of helix H6 which is an α-helix.

The AR-LBD of the present invention comprises contiguous helices. In this respect, helices H4 and H5 and helices H10 and H11 are contiguous. In contrast only the H10 and H11 sequences of the progesterone receptor were found to be contiguous (see Williams 1998).

### CONTIGUOUS

As used herein, the term "contiguous helices" means helices which are connected to each other such as connected in line with each other.

### BETA SHEET (β-SHEET) and BETA STRANDS (β STRANDS)

As used herein, the term "beta sheet (β-sheet) structure means a combination of several regions of a polypeptide chain. In contrast, the α helix, is built up from one continuous region. These regions, β strands, are usually from 5 to 10 residues long and are in an almost fully extended conformation with φ, ψ angles within the broad structurally allowed region in the upper left quadrant of the Ramachandran plot. These β strands are aligned adjacent to each other such that hydrogen bonds can form between C'O groups of one β strand and NH groups on an adjacent β strand and vice versa. The β sheets that are formed from several such β strands are "pleated" with C_{α} atoms successively a little above and below the plane of the β sheet. The side chains follow this pattern such that within a β strand they also point alternatively above and below the β sheet.

### PARALLEL AND ANTI-PARALLEL β-SHEET

β strands can interact in two ways to form a pleated sheet. Either the amino acids in the aligned β strands can all run in the same biochemical direction, amino terminal to carboxy terminal, in which case the sheet is described as parallel, or the amino acids in successive strands can have alternating directions, amino terminal to carboxy terminal followed by carboxy terminal to amino terminal, followed by amino terminal to carboxy terminal, and so on, in which case the sheet is called antiparallel. Each of the two forms has a distinctive pattern of hydrogen bonding. The antiparallel β sheet has narrowly spaced hydrogen bond pairs that alternate with widely spaced pairs. Parallel β sheets have evenly spaced hydrogen bonds that angle across between the β strands. Within both types of β sheets all possible main chain hydrogen bonds are formed, except for the two flanking strands of the β sheet that only have one neighboring β strand.

The AR-LBD of the present invention comprises beta strands (β strands), designated by the letter E, which are make up sheets. These strands (S1, S2, S3 and S4) are arranged in the order in which they appear in the secondary structure as set out in Figure 1. These strands are arranged in two β-sheets.

### KEY RESIDUES

As used herein the term "key residues" refers to one or more amino acid residues in an AR-LBD, capable of modulating ligand binding. The residues may be any one of the key residues within the AR-LBD as described herein or mutants thereof or they may be residues with homology to the residues or mutants thereof. The key amino acid residues of the AR-LBD may be any one or more of the amino acid residues selected from the group consisting of: L701; L704; N705; L707; Q711; M742; L744; M745; M749; R752; F764; Q783; M787; F876; T877; L880; F891; M895 or a homologue or mutant thereof.

### CONFORMATIONALLY CONSTRAINED RESIDUES

Preferably binding of the ligand to the AR-LBD causes conformational changes to the AR-LBD thereby inhibiting further binding thereto.
Preferably the ligand produced in accordance with the invention fills at least the LBP of the AR without perturbing the remainder of the AR structure.

Preferably the ligand interacts with conformationally constrained residue of the AR-LBD.
As used herein, the term "conformationally constrained residue" refers to a residue, such as an amino acid residue whose binding properties may be modulated through a mutation in that residue. The mutation in the amino acid residue may result in a change in the conformation of that residue. In particular, the mutation may result in a restricted/constrained conformation which may affect the interaction of a ligand with the hAR-LBD.

### BINDING AFFINITY

Preferably the ligands of the present invention bind more effectively to the AR-LBD than androgen.

Preferably the ligands of the present invention bind with twice the binding affinity of androgen.

Preferably the ligands of the present invention bind with three times the affinity of androgen.

Preferably the ligands of the present invention bind with ten or more times the affinity of androgen.

Preferably the improvements in the interaction of a ligand with the AR-LBD are manifested as increases in binding affinity but may also include increases in receptor selectivity and/or modulation of efficacy.

Preferably the ligand inhibits the action of androgen and androgen mimetics by binding tightly to the AR-LBD but by not up-regulating androgen receptor gene expression.

### MODEL

One aspect of the present invention is related to a model.
The crystal structure of the present invention can be used to generate a structural model such as a three dimensional (3D) structural model (or a representation thereof) comprising an AR-LBD or portion thereof. Alternatively, the crystal structure may be used to generate a computer model for the structure.

Preferably the crystal model comprising the AR-LBD is built from all or part of the X-ray diffraction data presented in Table 1 and/or the refinement statistics presented in Table 2.

Preferably the crystal model comprising the AR-LBD is built from all or part of the crystal co-ordinate data as shown in Table 4 (see Figure 6).

Thus, for example, the structural co-ordinates provided in the crystal structure and/or model structure may comprise the amino acid residues of the AR-LBD, or a portion of the AR-LBD or a homologue thereof useful in the modelling and design of test compounds capable of binding to the AR-LBD.

As used herein, the term "modelling" includes the quantitative and qualitative analysis of molecular structure and/or function based on atomic structural information and interaction models. The term "modelling" includes conventional numeric-based molecular dynamic and energy minimization models, interactive computer graphic models, modified molecular mechanics models, distance geometry and other structure-based constraint models.

In another aspect of the present invention, the structural coordinates comprising the AR-LBD or a portion thereof may be applied to a model screening system.

As used herein, the term "model screening system" may be a solid 3D screening system or a computational screening system. Using this model, Test compounds can be modelled that fit spatially and preferentially into the AR-LBD.

In one preferred aspect, the test compounds are positioned in the AR-IBD through computational docking.

In another preferred aspect, the test compounds are positioned in the AR-BD through manual docking.

As used herein, the term "fits spatially" means that the three-dimensional structure of a ligand is accommodated geometrically in a cavity or pocket of an AR-IBD.

Preferably, modelling is performed using a computer and may be further optimized using known methods. This is called modelling optimisation. Overlays and super positioning with a three dimensional model of the AR-LBD, and/or a portion thereof, can also be used for modelling optimisation.

Alignment and/or modelling can be used as a guide for the placement of mutations on the AR-LBD surface to characterise the nature of the site in the context of a cell.

The structure coordinates of an AR-LBD structure described herein can be used as a model for determining the secondary or three-dimensional structures of additional native or mutated AR-LBD with unknown structure, as well as the structures of co-crystals of AR-LBD with compounds such as substrates and modulators (e.g. stimulators or inhibitors). The structure coordinates and models of an AR-LBD structure can also be used to determine solution-based structures of native or mutant AR-LBD.

Secondary or three-dimensional structure may be determined by applying the structural coordinates of an AR-LBD structure to other data such as an amino acid sequence, X-ray crystallographic diffraction data, or nuclear magnetic resonance (NMR) data. Homology modeling, molecular replacement, and nuclear magnetic resonance methods using these other data sets are described below.

Homology modeling (also known as comparative modeling or knowledge-based modeling) methods develop a three dimensional model from a polypeptide sequence based on the structures of known proteins (e.g. native or mutated AR-LBD). In the present invention the method utilizes a computer representation of an AR-LBD structure or a complex of same, a computer representation of the amino acid sequence of a polypeptide with an unknown structure (additional native or mutated AR-LBD), and standard computer representations of the structures of amino acids. The method in particular comprises the steps of; (a) identifying structurally conserved and variable regions in the known structure; (b) aligning the amino acid sequences of the known structure and unknown structure (c) generating coordinates of main chain atoms and side chain atoms in structurally conserved and variable regions of the unknown structure based on the coordinates of the known structure thereby obtaining a homology model; and (d) refining the homology model to obtain a three dimensional structure for the unknown structure. This method is well known to those skilled in the art (Greer, 1985, Science 228, 1055; Bundell et al 1988, Eur. J. Biochem. 172, 513; Knighton et al., 1992, Science 258:130-135, http://biochem.vt.edu/courses/modeling/homology.htn). Computer programs that can be used in homology modeling are Quanta and the Homology module in the Insight II modeling package distributed by Molecular Simulations Inc, or MODELLER (Rockefeller University, www.iucr.ac:uk/sinris-top/logical/prg-modeller.html).

In step (a) of the homology modeling method, the known AR-LBD structure is examined to identify the structurally conserved regions (SCRs) from which an average structure, or framework, can be constructed for these regions of the protein. Variable regions (VRs), in which known structures may differ in conformation, also must be identified. SCRs generally correspond to the elements of secondary structure, such as alpha-helices and beta-sheets, and to ligand- and substrate-binding sites (e.g. acceptor and donor binding sites). The VRs usually lie on the surface of the proteins and form the loops where the main chain turns.

Many methods are available for sequence alignment of known structures and unknown structures. Sequence alignments generally are based on the dynamic programming algorithm of Needleman and Wunsch [J. Mol. Biol. 48: 442-453, 1970]. Current methods include FASTA, Smith-Waterman, and BLASTP, with the BLASTP method differing from the other two in not allowing gaps. Scoring of alignments typically involves construction of a 20x20 matrix in which identical amino acids and those of similar character (i.e., conservative substitutions) may be scored higher than those of different character. Substitution schemes which may be used to score alignments include the scoring matrices PAM (Dayhoff et al., Meth. Enzymol. 91: 524-545, 1983), and BLOSUM (Henikoff and Henikoff, Proc. Nat. Acad. Sci. USA 89: 10915-10919, 1992), and the matrices based on alignments derived from three-dimensional structures including that of Johnson and Overington (JO matrices) (J. Mol. Biol. 233: 716-738, 1993).

Alignment based solely on sequence may be used; however, other structural features also may be taken into account. In Quanta, multiple sequence alignment algorithms are available that may be used when aligning a sequence of the unknown with the known structures. Four scoring systems (i.e. sequence homology, secondary structure homology, residue accessibility homology; CA-CA distance homology) are available, each of which may be evaluated during an alignment so that relative statistical weights may be assigned.

When generating coordinates for the unknown structure, main chain atoms and side chain atoms, both in SCRs and VRs need to be modeled. A variety of approaches known to those skilled in the art may be used to assign coordinates to the unknown. In particular, the coordinates of the main chain atoms of SCRs will be transferred to the unknown structure. VRs correspond most often to the loops on the surface of the polypeptide and if a loop in the known structure is a good model for the unknown, then the main chain coordinates of the known structure may be copied. Side chain coordinates of SCRs and VRs are copied if the residue type in the unknown is identical to or very similar to that in the known structure. For other side chain coordinates, a side chain rotamer library may be used to define the side chain coordinates. When a good model for a loop cannot be found fragment databases may be searched for loops in other proteins that may provide a suitable model for the unknown. If desired, the loop may then be subjected to conformational searching to identify low energy conformers if desired.

Once a homology model has been generated it is analyzed to determine its correctness. A computer program available to assist in this analysis is the Protein Health module in Quanta which provides a variety of tests. Other programs that provide structure analysis along with output include PROCHECK and 3D-Profiler [Luthy R. et al, Nature 356: 83-85, 1992; and Bowie, J.U. et al, Science 253: 164-170, 1991]. Once any irregularities have been resolved, the entire structure may be further refined. Refinement may consist of energy minimization with restraints, especially for the SCRs. Restraints may be gradually removed for subsequent minimizations. Molecular dynamics may also be applied in conjunction with energy minimization.

Using the structure coordinates of the crystal complexes provided by this invention, molecular replacement may be used to determine the structure coordinates of a crystalline mutant or homologue of AR-LBD or of a related protein.

Molecular replacement involves applying a known structure to solve the X-ray crystallographic data set of a polypeptide of unknown structure (e.g. native or mutated AR-LBD). The method can be used to define the phases describing the X-ray diffraction data of a polypeptide of unknown structure when only the amplitudes are known. Commonly used computer software packages for molecular replacement are X-PLOR (Brunger 1992, Nature 355: 472-475), AMoRE (Navaza, 1994, Acta Crystallogr. A50:157-163), the CCP4 package (Collaborative Computational Project, Number 4, "The CCP4 Suite: Programs for Protein Crystallography", Acta Cryst., Vol. D50, pp. 760-763, 1994), and the MERLOT package (P.M.D. Fitzgerald, J. Appl. Cryst., Vol. 21, pp. 273-278, 1988). It is preferable that the resulting structure not exhibit a root-mean-square deviation of more than 3 Å.

Molecular replacement computer programs generally involve the following steps: (1) determining the number of molecules in the unit cell and defining the angles between them (self rotation function); (2) rotating the known structure (e.g. AR-LBD) against diffraction data to define the orientation of the molecules in the unit cell (rotation function); (3) translating the known structure in three dimensions to correctly position the molecules in the unit cell (translation function); (4) determining the phases of the X-ray diffraction data and calculating an R-factor calculated from the reference data set and from the new data wherein an R-factor between 30-50% indicates that the orientations of the atoms in the unit cell have been reasonably determined by the method; and (5) optionally, decreasing the R-factor to about 20% by refining the new electron density map using iterative refinement techniques known to those skilled in the art (refinement).

In an embodiment of the invention, a method is provided for determining three dimensional structures of polypeptides with unknown structure (e.g. additional native or mutated AR-LBD) by applying the structural coordinates of an AR-LBD structure to provide an X-ray crystallographic data set for a polypeptide of unknown structure, and (b) determining a low energy conformation of the resulting structure.

The structural coordinates of an AR-LBD structure may be applied to nuclear magnetic resonance (NMR) data to determine the three dimensional structures of polypeptides (e.g. additional native or mutated AR-LBD). (See for example, Wuthrich, 1986, John Wiley and Sons, New York: 176-199; Pflugrath et al., 1986, J. Molecular Biology 189: 383-386; Kline et al., 1986 J. Molecular Biology 189:377-382). While the secondary structure of a polypeptide may often be determined by NMR data, the spatial connections between individual pieces of secondary structure are not as readily determined. The structural coordinates of a polypeptide defined by X-ray crystallography can guide the NMR spectroscopist to an understanding of the spatial interactions between secondary structural elements in a polypeptide of related structure. Information on spatial interactions between secondary structural elements can greatly simplify Nuclear Overhauser Effect (NOE) data from two-dimensional NMR experiments. In addition, applying the structural coordinates after the determination of secondary structure by NMR techniques simplifies the assignment of NOE's relating to particular amino acids in the polypeptide sequence and does not greatly bias the NMR analysis of polypeptide structure.

This, in turn, can be subject to any of the several forms of refinement to provide a final, accurate structure of the unknown crystal. Lattman, E., "Use of the Rotation and Translation Functions", in Methods in Enzymology, 115, pp. 55-77 (1985); M. G. Rossmann, ed., "The Molecular Replacement Method", Int. Sci. Rev. Ser., No. 13, Gordon & Breach, New York, (1972).

Other molecular modelling techniques may also be employed in accordance with this invention. See, e.g., Cohen, N. C. et al, "Molecular Modelling Software and Methods for Medicinal Chemistry", J. Med. Chem., 33, pp. 883-894 (1990). See also, Navia, M. A. and M. A. Murcko, "The Use of Structural Information in Drug Design", Current Opinions in Structural Biology, 2, pp. 202-210 (1992).

The present invention also relates to a method of screening for a ligand capable of binding to the AR-LBD and/or which are capable of modulating the binding capacity of the AR-LBD wherein said method comprises the use of the crystal or model according to the invention.
The method may employ a solid 3D screening system or a computational screening system. Using these systems, test compounds may be screened to find those which interact spatially and preferentially with the AR-LBD, through either computational or manual docking.

### TEST COMPOUNDS

In one aspect, the invention relates to a method of screening for a ligand capable of binding to an AR-LBD, wherein the AR-LBD is defined by the amino acid residue structural coordinates given above, the method comprising contacting the AR-LBD with a test compound and determining if said test compound binds to said AR-LBD.

As used herein, the term "test compound" includes, but is not limited to, a compound which may be obtainable from or produced by any suitable source, whether natural or not. The test compound may be designed or obtained from a library of compounds which may comprise peptides, as well as other compounds, such as small organic molecules and particularly new lead compounds. By way of example, the test compound may be a natural substance, a biological macromolecule, or an extract made from biological materials such as bacteria, fungi, or animal (particularly mammalian) cells or tissues, an organic or an inorganic molecule, a synthetic test compound, a semi-synthetic test compound, a structural or functional mimetic, a peptide, a peptidomimetics, a derivatised test compound, a peptide cleaved from a whole protein, or a peptides synthesised synthetically (such as, by way of example, either using a peptide synthesizer or by recombinant techniques or combinations thereof, a recombinant test compound, a natural or a non-natural test compound, a fusion protein or equivalent thereof and mutants, derivatives or combinations thereof.

### MODULATING

The term "modulating" means inducing an increase or a decrease in the activity of the androgen receptor through binding of a test compound to an AR-LBD. The term also encompasses removal of the activity of the receptor.

### MIMETIC

As used herein, the term "mimetic" relates to any chemical which includes, but is not limited to, a peptide, polypeptide, antibody or other organic chemical which has the same qualitative activity or effect as a known test compound. That is, the mimetic is a functional equivalent of a known test compound (such as a known ligand capable of binding to the AR-LBD).

### DERIVATIVE

The term "derivative" or "derivatised" as used herein includes chemical modification of a test compound. Illustrative of such chemical modifications would be replacement of hydrogen by a halo group, an alkyl group, an acyl group or an amino group.

Typically the test compound will be prepared by recombinant DNA techniques and/or chemical synthesis techniques.

Once a test compound capable of interacting with a key amino acid residue in the AR-LBD has been identified, further steps may be carried out either to select and/or to modify compounds and/or to modify existing compounds, to modulate the interaction with the key amino acid residues in the AR-LBD.

### BIOLOGICAL SCREENS

Test compounds and ligands which are identified with the crystal of the present invention can be screened in assays such as are well known in the art. Screening can be, for example *in vitro,* in cell culture, and/or *in vivo.* Biological screening assays preferably center on activity-based response models, binding assays (which measure how well a compound binds to the receptor), and bacterial, yeast and animal cell lines (which measure the biological effect of a compound in a cell). The assays can be automated for high capacity-high throughput screening (HTS) in which large numbers of compounds can be tested to identify compounds with the desired activity. The biological assay, may also be an assay for ligand binding activity a compound that selectively binds to the LBD compared to other nuclear receptors.

In one embodiment, the present invention provides a method of screening for a test compound capable of interacting with a key amino acid residue of the AR-LBD.

Another preferred aspect of the invention provides a process comprising the steps of:
(a) performing the method of screening for a ligand as described above;
(b) identifying one or more ligands capable of binding to a ligand binding domain; and
(c) preparing a quantity of said one or more ligands.

A further preferred aspect of the invention provides a process comprising the steps of:
(a) performing the method of screening for a ligand as described above;
(b) identifying one or more ligands capable of binding to an AR-LBD; and
(c) preparing a pharmaceutical composition comprising said one or more ligands.

Yet another preferred aspect of the invention provides a process comprising the steps of:
(a) performing the method of screening for a ligand as described above;
(b) identifying one or more ligands capable of binding to an AR-LBD;
(c) modifying said one or more ligands capable of binding to an AR-LBD;
(d) performing said method of screening for a ligand as described above;
(e) optionally preparing a pharmaceutical composition comprising said one or more ligands.

Thus, the structural information from the crystal structure of the present invention is useful in the design of potential ligands capable of interacting with the AR-LBD and/or capable of modulating the DNA binding capacity of the AR-LBD, and the models of the present invention are useful to examine the effect such a ligand is likely to have on the structure and/or function of the AR-LBD.

In one aspect the present invention relates to a ligand identified using such screening methods.

### LIGAND

As used herein, the term "ligand" refers to a test compound capable of binding to one or more key residues in the LBD. Such a ligand may also be referred to as an androgen receptor binding compound. Preferably the ligand is capable of modulating the activity of AR-LBD.

### IDENTIFICATION OF MODULATORS OF AR-LBD

Modulators (e.g. inhibitors) of a AR-LBD may be designed and identified that may modify a AR-LBD involved in a clinical disorder. The rational design and identification of modulators of AR-LBD can be accomplished by utilizing the atomic structural coordinates that define an AR-LBD structure, or a part thereof. Structure-based modulator design identification methods are powerful techniques that can involve searches of computer data bases containing a variety of potential modulators and chemical functional groups. (See. Kuntz et al., 1994, Acc. Chem. Res. 27:117; Guida, 1994, Current Opinion in Struc. Biol. 4: 777; and Colman, 1994, Current Opinion in Struc. Biol. 4: 868, for reviews of structure-based drug design and identification;and Kuntz et al 1982, J. Mol. Biol. 162:269; Kuntz et al., 1994, Ace. Chem. Res. 27: 117; Meng et al., 1992, J. Compt. Chem. 13: 505; Bohm, 1994, J. Comp. Aided Molec. Design 8: 623 for methods of structure-based modulator design).

The AR-LBD structures, and parts thereof described herein, and the structures of other polypeptides determined by the homology modeling, molecular replacement, and NMR techniques described herein can also be applied to modulator design and identification methods.

Modulators of AR-LBD may be identified by docking the computer representation of compounds from a data base of molecules. Data bases which may be used include ACD (Molecular Designs Limited), NCI (National Cancer Institute), CCDC (Cambridge Crystallographic Data Center), CAST (Chemical Abstract Service), Derwent (Derwent Information Limited), Maybridge (Maybridge Chemical Company Ltd), Aldrich (Aldrich Chemical Company), DOCK (University of California in San Francisco), and the Directory of Natural Products (Chapman & Hall). Computer programs such as CONCORD (Tripos Associates) or DB-Converter (Molecular Simulations Limited) can be used to convert a data set represented in two dimensions to one represented in three dimensions.

The computer programs may comprise the following steps:
(a) docking a computer representation of a structure of a compound into a computer representation of an AR-LBD defined in accordance with the invention using the computer program, or by interactively moving the representation of the compound into the representation of the binding site;
(b) characterizing the geometry and the complementary interactions formed between the atoms of the binding site and the compound; optionally
(c) searching libraries for molecular fragments which can fit into the empty space between the compound and binding site and can be linked to the compound; and
(d) linking the fragments found in (c) to the compound and evaluating the new modified compound.

Methods are also provided for identifying a potential modulator of an AR-LBD function by docking a computer representation of a compound with a computer representation of a structure of an AR-LBD that is defined by atomic interactions, atomic contacts, or atomic structural coordinates described herein. In an embodiment the method comprises the following steps:
(a) docking a computer representation of a compound from a computer data base with a computer representation of a selected site (e.g. the inhibitor binding site) on a AR-LBD structure defined in accordance with the invention to obtain a complex;
(b) determining a conformation of the complex with a favourable geometric fit and favourable complementary interactions; and
(c) identifying compounds that best fit the selected site as potential modulators of the AR-LBD.

"Docking" refers to a process of placing a compound in close proximity with an active site of a polypeptide (i.e. an AR-LBD), or a process of finding low energy conformations of a compound/polypeptide complex (i.e. compound/AR-LBD complex).

Examples of other computer programs that may be used for structure-based modulator design are CAVEAT (Bartlett et al., 1989, in "Chemical and Biological Problems in Molecular Recognition", Roberts, S.M. Ley, S.V.; Campbell, N.M. eds; Royal Society of Chemistry: Cambridge, pp 182-196); FLOG (Miller et al., 1994, J. Comp. Aided Molec. Design 8:153); PRO Modulator (Clark et al., 1995 J. Comp. Aided Molec. Design 9:13); MCSS (Miranker and Karplus, 1991, Proteins: Structure, Fuction, and Genetics 8:195); and, GRID (Goodford, 1985, J. Med. Chem. 28:849).

In an embodiment of the invention, a method is provided for identifying potential modulators of AR-LBD function. The method utilizes the structural coordinates of an AR-LBD three dimensional structure, or binding site thereof. The method comprises the steps of (a) generating a computer representation of an AR-LBD structure, and docking a computer representation of a compound from a computer data base with a computer representation of the AR-LBD to form a complex; (b) determining a conformation of the complex with a favourable geometric fit or favorable complementary interactions; and (c) identifying compounds that best fit the AR-LBD as potential modulators of AR-LBD function. The initial AR-LBD structure may or may not have compounds bound to it. A favourable geometric fit occurs when the surface areas of a compound in a compound-AR-LBD complex is in close proximity with the surface area of the AR-LBD without forming unfavorable interactions. A favourable complementary interaction occurs where a compound in a compound-AR-LBD complex interacts by hydrophobic, aromatic, ionic, or hydrogen donating and accepting forces, with the AR-LBD without forming unfavorable interactions. Unfavourable interactions may be steric hindrance between atoms in the compound and atoms in the AR-LBD.

In another embodiment, potential modulators are identified utilizing an AR-LBD structure with or without compounds bound to it. The method comprises the steps of (a) modifying a computer representation of an AR-LBD having one or more compounds bound to it, where the computer representations of the compound or compounds and AR-LBD are defined by atomic structural coordinates; (b) determining a conformation of the complex with a favorable geometric fit and favorable complementary interactions; and (c) identifying the compounds that best fit the AR-LBD as potential modulators. A computer representation may be modified by deleting or adding a chemical group or groups. Computer representations of the chemical groups can be selected from a computer database.

Another way of identifying potential modulators is to modify an existing modulator in a polypeptide binding site. The computer representation of modulators can be modified within the computer representation of an AR-LBD. This technique is described in detail in Molecular Simulations User Manual, 1995 in LUDI. The computer representation of a modulator may be modified by deleting a chemical group or groups, or by adding a chemical group or groups. After each modification to a compound, the atoms of the modified compound and binding site can be shifted in conformation and the distance between the modulator and the binding site atoms may be scored on the basis of geometric fit and favourable complementary interactions between the molecules. Compounds with favourable scores are potential modulators.

Compounds designed by modulator building or modulator searching computer programs may be screened to identify potential modulators. Examples of such computer programs include programs in the Molecular Simulations Package (Catalyst), ISIS/HOST, ISIS/BASE, and ISIS/DRAW (Molecular Designs Limited), and UNITY (Tripos Associates). A building program may be used to replace computer.representations of chemical groups in a compound complexed with an AR-LBD with groups from a computer database. A searching program may be used to search computer representations of compounds from a computer database that have similar three dimensional structures and similar chemical groups as a compound that binds to an AR-LBD. The programs may be operated on the structure of the AR-LBD structure.

A typical program may comprise the following steps:
(a) mapping chemical features of a compound such as by hydrogen bond donors or acceptors, hydrophobic/lipophilic sites, positively ionizable sites, or negatively ionizable sites;
(b) adding geometric constraints to selected mapped features;
(c) searching data bases with the model generated in (b).

In an embodiment of the invention a method of identifying potential modulators of an AR-LBD is provided using the three dimensional conformation of the AR-LBD in various modulator construction or modulator searching computer programs on compounds complexed with the AR-LBD. The method comprises the steps of (a) generating a computer representation of one or more compounds complexed with an AR-LBD; (b) (i) searching a data base for a compound with a similar geometric structure or similar chemical groups to the generated compounds using a computer program that searches computer representations of compounds from a database that have similar three dimensional structures and similar chemical groups, or (ii) replacing portions of the compounds complexed with the AR-LBD with similar chemical structures (i.e. nearly identical shape and volume) from a database using a compound construction computer program that replaces computer representations of chemical groups with groups from a computer database, where the representations of the compounds are defined by structural coordinates.

A compound that interacts with an AR-LBD identified using a method of the invention may be used as a modulator of any AR-LBD or composition bearing the interacting binding domain. Therefore, the invention features a modulator of an AR-LBD identified by a method of the invention.

The invention further contemplates a method for designing potential inhibitors of an AR-LBD comprising the step of using the structural coordinates of an inhibitor or substrate or parts thereof, defined in relation to its spatial association with an AR-LBD structure to generate a compound that is capable of associating with the AR-LBD.

In an embodiment of the invention, a method is provided for designing potential inhibitors of an AR-LBD comprising the step of using the structural coordinates of AR-LBD in Table 4 to generate a compound for associating with the active site of an AR-LBD. The following steps are employed in a particular method of the invention: (a) generating a computer representation of AR-LBD defined by its structural coordinates listed in Table 4; (b) searching for molecules in a data base that are structurally or chemically similar to the defined AR-LBD, using a searching computer program, or replacing portions of the compound with similar chemical structures from a database using a compound building computer program.

It will be appreciated that a modulator of an AR-LBD may be identified by generating an actual three-dimensional model of a binding cavity, synthesizing a compound, and examining the components to find whether the required interaction occurs.

Potential modulators of AR-LBD identified using the above-described methods may be prepared using methods described in standard reference sources utilized by those skilled in the art. For example, organic compounds may be prepared by organic synthetic methods described in references such as March, 1994, Advanced Organic Chemistry: Reactions, Mechanisms, and Structure, New York, McGraw Hill.

The invention also relates to a potential modulator identified by the methods of the invention. In particular, classes of modulators of AR-LBD are provided that are based on the three-dimensional structure of an inhibitor's or modulator's spatial association with an AR-LBD structure.

The invention contemplates all optical isomers and racemic forms of the modulators of the invention.

"Modulator" refers to a molecule which changes or alters the biological activity of a AR-LBD. A modulator may increase or decrease AR-LBD activity, or change its characteristics, or functional or immunological properties. It may be an inhibitor that decreases the biological or immunological activity of the protein. A modulator may enhance or inhibit a biological activity of AR-LBD.

Modulators include but are not limited to peptides, members of random peptide libraries and combinatorial chemistry-derived molecular libraries, phosphopeptides (including members of random or partially degenerate, directed phosphopeptide libraries), antibodies, carbohydrates, nucleosides or nucleotides or parts thereof, and small organic or inorganic molecules. A modulator may be an endogenous physiological compound, or it may be a natural or synthetic compound.

### LIGAND

The term "ligand" includes, but is not limited to, steroidal and non-steroidal ligands. The ligands may be natural or synthetic. The ligands may be structurally novel AR-LBD ligands. Alternatively, the ligands may be analogues of known AR-LBD ligands but with improved properties. The ligand may be an androgen mimetic. The ligand may be capable of modulating (e.g. upregulating) androgen receptor gene expression. Alternatively, the ligand may be capable of blocking the activity of androgens by binding to an AR-LBD with a high affinity. The ligand may be capable of down regulating androgen receptor gene expression. The term "ligand" also refers to a chemically modified ligand.

The ligand may act, for example, as an agonist, a partial agonist, an antagonist, and/or a competitive antagonist of the androgen receptor.

For some embodiments, the ligand is in a purified and/or isolated form.

### DESIGNER LIGANDS

As used herein, the term means "designer ligands" refers to test compounds which are likely to bind to the AR-LBD based on their three dimensional shape compared to that of the androgen receptor and in particular the AR-LBD.

Preferably, those compounds have a structure which is complementary to that of the AR-LBD.

Preferably the ligands comprise ligand substituents which compensate for the structural changes in the ligand binding pocket (LBP) between the wild type and mutant AR-LBDs.

The test compound may be tested for its interaction with an interacting amino acid residue in the AR-LBD. Alternatively, the test compound may affect ligand binding by acting either as an agonists or an antagonists.

### AGONIST

As used herein, the term "agonist" means any ligand, which is capable of binding to an AR-LBD and which is capable of increasing a proportion of the AR that is in an active form, resulting in an increased biological response. The term includes partial agonists and inverse agonists.

### PARTIAL AGONIST

As used herein, the term "partial agonist" means an agonist that is unable to evoke the maximal response of a biological system, even at a concentration sufficient to saturate the specific receptors.

### INVERSE AGONIST

As used herein, the term "partial inverse agonist" is an inverse agonist that evokes a submaximal response to a biological system, even at a concentration sufficient to saturate the specific receptors. At high concentrations, it will diminish the actions of a full inverse agonist.

### ANTAGONIST

As used herein, the term "antagonist" means any agent that reduces the action of another agent, such as an agonist. The antagonist may act at the same receptor as the agonist. The antagonistic action may result from a combination of the substance being antagonised (chemical antagonism) or the production of an opposite effect through a different receptor (functional antagonism or physiological antagonism) or as a consequence of competition for the binding site of an intermediate that links receptor activation to the effect observed (indirect antagonism).

### COMPETITIVE ANTAGONIST

As used herein, the term "competitive antagonism" refers to the competition between an agonist and an antagonist for a receptor that occurs when the binding of agonist and antagonist becomes mutually exclusive. This may be because the agonist and antagonist compete for the same binding site or combine with adjacent but overlapping sites. A third possibility is that different sites are involved but that they influence the receptor macromolecules in such a way that agonist and antagonist molecules cannot be bound at the same time. If the agonist and antagonist form only short lived combinations with the receptor so that equilibrium between agonist, antagonist and receptor is reached during the presence of the agonist, the antagonism will be surmountable over a wide range of concentrations. In contrast, some antagonists, when in close enough proximity to their binding site, may form a stable covalent bond with it and the antagonism becomes insurmountable when no spare receptors remain.

In one aspect, the identified ligand may act as a ligand model (for example, a template) for the development of other compounds.

### LIGAND MODEL

The term "ligand model" refers to the structural coordinates of a compound that fits into the AR-ligand binding domain (LBD) and which may be used for modeling to identify and/or design ligands (designer ligands) capable of binding to the AR-LBD, such as for the subsequent modulation thereof.

One skilled in the art may use one of several methods to test compounds for their ability to associate with AR-LBD. This process may begin by visual inspection of, for example, a target site on the computer screen based on the structure coordinates given in Table 4. Selected test compounds may then be positioned in a variety of orientations, or docked, within an individual target site of AR-LBD as defined supra. Docking may be accomplished using software such as Quanta and Sybyl, followed by energy minimization and molecular dynamics with standard molecular mechanics forcefields, such as CHARMM and AMBER. Specialized computer programs may also assist in the process of selecting potential ligands. These include:
1. GRID (Goodford, P. J., "A Computational Procedure for Determining Energetically Favorable Binding Sites on Biologically Important Macromolecules", J. Med. Chem., 28, pp. 849-857 (1985)). GRID is available from Oxford University, Oxford, UK.
2. MCSS (Miranker, A. and M. Karplus, "Functionality Maps of Binding Sites: A Multiple Copy Simultaneous Search Method." Proteins: Structure. Function and Genetics, 11, pp. 29-34 (1991)). MCSS is available from Molecular Simulations, Burlington, Mass.
3. AUTODOCK (Goodsell, D. S. and A. J. Olsen, "Automated Docking of Substrates to Proteins by Simulated Annealing", Proteins: Structure. Function, and Genetics, 8, pp. 195-202 (1990)). AUTODOCK is available from Scripps Research Institute, La Jolla, Calif.
4. DOCK (Kuntz, I. D. et al., "A Geometric Approach to Macromolecule-Ligand Interactions", J. Mol. Biol., 161, pp. 269-288 (1982)). DOCK is available from University of California, San Francisco, Calif.
   Once a ligand has been optimally selected or designed, substitutions may then be made in some of its atoms or side groups in order to improve or modify its binding properties. Generally, initial substitutions are conservative, i.e., the replacement group will have approximately the same size, shape, hydrophobicity and charge as the original group. It should, of course, be understood that components known in the art to alter conformation should be avoided. Such substituted chemical compounds may then be analyzed for efficiency of fit to AR-LBD by the same computer methods described above.
   Preferably, positions for substitution are selected based on the predicted binding orientation of a test compound to the AR-LBD.
   The ligands of the present invention may be natural or synthetic. The term "ligand" also refers to a chemically modified ligand.

### SYNTHESIS METHODS

The ligand of the present invention or mimetics thereof may be produced using chemical methods to synthesize the ligand in whole or in part. For example, peptides can be synthesized by solid phase techniques, cleaved from the resin, and purified by preparative high performance liquid chromatography (e.g., Creighton (1983) Proteins Structures And Molecular Principles, WH Freeman and Co, New York NY). The composition of the synthetic peptides may be confirmed by amino acid analysis or sequencing (e.g., the Edman degradation procedure; Creighton, *supra*).

Direct synthesis of the ligand or mimetics thereof can be performed using various solid-phase techniques (Roberge JY et al (1995) Science 269: 202-204) and automated synthesis may be achieved, for example, using the ABI 43 1 A Peptide Synthesizer (Perkin Elmer) in accordance with the instructions provided by the manufacturer. Additionally, the amino acid sequences obtainable from the ligand, or any part thereof, may be altered during direct synthesis and/or combined using chemical methods with a sequence from other subunits, or any part thereof, to produce a variant ligand.

In an alternative embodiment of the invention, the coding sequence of the ligand or mimetics thereof may be synthesized, in whole or in part, using chemical methods well known in the art (see Caruthers MH et al (1980) Nuc Acids Res Symp Ser 215-23, Horn T et al (1980) Nuc Acids Res Symp Ser 225-232).

Hence, the ligands may be chemically synthesised or they may be prepared using recombinant techniques.

In one aspect, preferably, the ligand is prepared by the use of chemical synthesis techniques.

### RECOMBINANT METHODS

In another aspect, preferably the ligands of the present invention may be produced from host cells using recombinant techniques.

A wide variety of host cells can be employed for expression of the nucleotide sequences encoding the ligands of the present invention. These cells may be both prokaryotic and eukaryotic host cells. Suitable host cells include bacteria such as *E. coli,* yeast, filamentous fungi, insect cells, mammalian cells, typically immortalized, e.g., mouse, CHO, human and monkey cell lines and derivatives thereof. Preferred host cells are able to process the expression products to produce an appropriate mature polypeptide. Processing includes but is not limited to glycosylation, ubiquitination, disulfide bond formation and general post-translational modification.

### CHEMICAL MODIFICATION

In one embodiment of the present invention, the ligand may be a chemically modified ligand.

The chemical modification of a ligand and/or a key amino acid residue of the present invention may either enhance or reduce hydrogen bonding interaction, charge interaction, hydrophobic interaction, Van Der Waals interaction or dipole interaction between the ligand and the key amino acid residue(s) of the AR-LBD. By way of example, steric hinderance is a common means of changing the interaction of the AR-LBD binding domain with the activation domain.

Preferably such modifications involve the addition of substituents onto a test compound such that the substituents are positioned to collide or to bind preferentially with one or more amino acid residues that correspond to the key amino acid residues of AR-LBD of the present invention.

### COMPARATIVE MODELS

The unique features involved in AR selective ligand binding can be identified by comparing crystal structures of different steroid receptors, such as the AR and the progesterone (PR) receptors and/or isoforms of the same type of receptor.

In a seventh aspect the present invention provides the use of a ligand identified by a method of screening which comprises the use of a crystal structure comprising an AR-LBD in the preparation of a medicament to prevent and/or treat androgen related disorders.

### DISORDERS

The term androgen related disorders relates to disorder such as prostrate cancer (PC), androgen insensitivity syndrome (AIS), partial androgen insensitivity syndrome (PAIS), mild androgen insensitivity syndrome (MAIS) and complete androgen insensitivity syndrome (CAIS).

### PHARMACEUTICAL COMPOSITIONS

In a further aspect, the present invention provides a pharmaceutical composition, which comprises a ligand according to the present invention and optionally a pharmaceutically acceptable carrier, diluent or excipient (including combinations thereof). The pharmaceutical composition may comprise or may be used in conjunction with an additional pharmaceutically active compound or composition.

The pharmaceutical compositions may be for human or animal usage in human and veterinary medicine and will typically comprise any one or more of a pharmaceutically acceptable diluent, carrier, or excipient. Acceptable carriers or diluents for therapeutic use are well known in the pharmaceutical art, and are described, for example, in Remington's Pharmaceutical Sciences, Mack Publishing Co. (A. R. Gennaro edit. 1985). The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s), solubilising agent(s).

Preservatives, stabilizers, dyes and even flavouring agents may be provided in the pharmaceutical composition. Examples of preservatives include sodium benzoate, sorbic acid and esters of p-hydroxybenzoic acid. Antioxidants and suspending agents may be also used.

There may be different composition/formulation requirements dependent on the different delivery systems. By way of example, the pharmaceutical composition of the present invention may be formulated to be delivered using a mini-pump or by a mucosal route, for example, as a nasal spray or aerosol for inhalation or ingestable solution, or parenterally in which the composition is formulated by an injectable form, for delivery, by, for example, an intravenous, intramuscular or subcutaneous route. Alternatively, the formulation may be designed to be delivered by both routes.

Where the pharmaceutical composition is to be delivered mucosally through the gastrointestinal mucosa, it should be able to remain stable during transit though the gastrointestinal tract; for example, it should be resistant to proteolytic degradation, stable at acid pH and resistant to the detergent effects of bile.

Where appropriate, the pharmaceutical compositions can be administered by inhalation, in the form of a suppository or pessary, topically in the form of a lotion, solution, cream, ointment or dusting powder, by use of a skin patch, orally in the form of tablets containing excipients such as starch or lactose or chalk, or in capsules or ovules either alone or in admixture with excipients, or in the form of elixirs, solutions or suspensions containing flavouring or colouring agents, or they can be injected parenterally, for example intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

### ADMINISTRATION

The invention further provides a method of preventing and/or treating an androgen related disorder in a mammal, the method comprising administering to a mammal a ligand which binds to at least the AR-LBD with high affinity, and in some cases to such an extent so as to modulate said AR-LBD. In one aspect, the block binding of further ligands to at least the AR-LBD. Such ligands may be useful in, for example, the treatment of AR mediated disorders in males or females.

Typically, a physician will determine the actual dosage which will be most suitable for an individual subject and it will vary with the age, weight and response of the particular patient and severity of the condition. The dosages below are exemplary of the average case. There can, of course, be individual instances where higher or lower dosage ranges are merited.

The compositions (or component parts thereof) of the present invention may be administered orally. In addition or in the alternative the compositions (or component parts thereof) of the present invention may be administered by direct injection. In addition or in the alternative the compositions (or component parts thereof) of the present invention may be administered topically. In addition or in the alternative the compositions (or component parts thereof) of the present invention may be administered by inhalation. In addition or in the alternative the compositions (or component parts thereof) of the present invention may also be administered by one or more of: parenteral, mucosal, intramuscular, intravenous, subcutaneous, intraocular or transdermal administration means, and are formulated for such administration.

By way of further example, the pharmaceutical composition of the present invention may be administered in accordance with a regimen of 1 to 10 times per day, such as once or twice per day. The specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

The term "administered" also includes but is not limited to delivery by a mucosal route, for example, as a nasal spray or aerosol for inhalation or as an ingestable solution; a parenteral route where delivery is by an injectable form, such as, for example, an intravenous, intramuscular or subcutaneous route.

Hence, the pharmaceutical composition of the present invention may be administered by one or more of the following routes: oral administration, injection (such as direct injection), topical, inhalation, parenteral administration, mucosal administration, intramuscular administration, intravenous administration, subcutaneous administration, intraocular administration or transdermal administration.

### STRUCTURAL STUDIES

One aspect of the invention provides to a method of determining the secondary and/or tertiary structures of polypeptides with unknown structures comprising the step of using a crystal structure or model of the invention.

The polypeptide under investigation is preferably structurally or functionally related to the androgen receptor ligand binding domain. For example, the polypeptide may show a degree of homology over some or all parts of the primary amino acid sequence.

As applied to polypeptides, the term "substantial sequence identity" means that two peptide sequences, when optimally aligned, such as by the programs GAP or BESTFIT using default gap, share at least 40%, 50%, 60%, 65%, 70%, 75%, 80%, or 85% sequence identity, preferably at least 90 percent sequence identity, more preferably at least 95 percent sequence identity or more. Preferably, residue positions which are not identical differ by conservative amino acid substitutions. For example, the substitution of amino acids having similar chemical properties such as charge or polarity are not likely to effect the properties of a protein. Examples include glutamine for asparagine or glutamic acid for aspartic acid.

In a further embodiment, the invention relates to a method of determining three dimensional structures of polypeptides with unknown structures, preferably a native or mutated AR-LBD by applying the structural coordinates of an AR-LBD structure of the invention to nuclear magnetic resonance (NMR) data of the unknown structure. This method comprises the steps of: (a) determining the secondary structure of an unknown structure using NMR data; and (b) simplifying the assignment of through-space interactions of amino acids. The term " through-space interactions" defines the orientation of the secondary structural elements in the three dimensional structure and the distances between amino acids from different portions of the amino acid sequence. The term "assignment" defines a method of analyzing NMR data and identifying which amino acids give rise to signals in the NMR spectrum.

The polypeptide may, for example be a mutant form of an AR-LBD. The term "mutant" refers to a polypeptide that is obtained by replacing at least one amino acid residue in a native AR-LBD with a different amino acid residue. Mutation can also be accomplished by adding and/or deleting amino acid residues within the native AR-LBD or part thereof. A mutant may or may not be functional.

Alternatively, the polypeptide may be an AR-LBD from a different species.

Alternatively, the polypeptide may perform an analogous function or be suspected to show a similar binding mechanism to the AR-LBD.

### ANDROGEN RECEPTOR LIGAND BINDING DOMAIN STRUCTURES

The present invention provides a secondary or three-dimensional structure of an AR-LBD or part thereof. In an embodiment the structure is a crystalline form An AR-LBD structure may comprise an AR-LBD unit cell.

An AR-LBD structure includes the secondary or three-dimensional structure of a native AR-LBD, a derivative AR-LBD, or a mutant AR-LBD. Thus, a crystalline form includes native crystals, derivative crystals, and co-crystals. The crystals generally comprise a substantially pure AR-LBD in crystalline form. It is understood that the AR-LBD structures of the invention are not limited to a naturally occurring or native AR-LBD but include polypeptides with substantial sequence identity to an AR-LBD. An AR-LBD structure also includes mutants of a native AR-LBD obtained by replacing at least one amino acid residue in a native AR-LBD with a different amino acid residue, or by adding or deleting amino acid residues within the native polypeptide, and having substantially the same secondary or three-dimensional structure as the native AR-LBD from which the mutant is derived i.e. having a set of atomic structural coordinates that have a root mean square deviation of less than or equal to about 5, 4, 3, 2, or 1.5 Å when superimposed with the atomic structure coordinates of the native AR-LBD from which the mutant is derived when at least 50% to 100% of the atoms of the native AR-LBD are included in the superimposition. It should be noted that the AR-LBD structures contemplated herein need not exhibit AR-LBD activity.

A derivative AR-LBD structure of the invention comprises an AR-LBD structure in association with one or more moieties that are heavy metal atoms. For example, derivative crystals of the invention generally comprise a crystalline AR-LBD in covalent association with one or more heavy metal atoms. The AR-LBD may correspond to a native or mutated AR-LBD. Heavy metal atoms useful for providing derivative AR-LBD structures include by way of example, and not limitation, gold, mercury, etc.

The invention features an AR-LBD structure in association with one or more moieties that are ligands. The association may be covalent or non-covalent. Crystalline forms of this type are referred to herein as co-crystals. The compound may be any organic molecule, and it may modulate the function of an AR-LBD by for example inhibiting or enhancing its function, or it may be a substrate for the AR-LBD. It is preferred that the geometry of the compound and the interactions formed between the compound and the AR-LBD provide high affinity binding between the two molecules.

The secondary or three-dimensional structures of the particular AR-LBD described herein provide useful models for the secondary or three-dimensional structures of AR-LBD from any species, particularly mammalian, including bovine, ovine, porcine, murine, equine, preferably human, from any source whether natural, synthetic, semi-synthetic, or recombinant.

In a particular embodiment of the invention, a secondary or three-dimensional crystal structure of an AR-LBD that associates with an inhibitor of an AR-LBD is provided comprising at least two or three atomic contacts of atomic interactions in Figure 4, each atomic interaction defined therein by an atomic contact (more preferably, a specific atom where indicated) on the inhibitor, and an atomic contact (more preferably, a specific amino acid residue where indicated) on the AR-LBD (i.e. ligand atomic contact). The binding domain may be defined by the ligand atomic contacts of atomic interactions in Figure 4. Preferably, the binding domain is defined by the atoms of the ligand atomic contacts having the structural coordinates for the atoms listed in Table 4.

### IDENTIFICATION OF HOMOLOGUES

The knowledge of an AR-LBD structure of the invention enables one skilled in the art to identify homologues of AR-LBD. This is achieved by searches of three-dimensional databases. Since structural folds are conserved to a greater extent than sequence, one may identify homologues with very little sequence identity or similarity. Programs that provide this type of database searching are known in the art and include Dali. The structural coordinates of a protein structure are submitted and the program performs a multiple structural alignment with proteins in the protein data bank. Homologues identified in accordance with the present invention may be used in the methods of the invention described herein.

### PROGESTERONE (PR) RECEPTOR

The present invention also provides experimentally isolated crystals for the PR-LBD in complex with the ligand metribolone (R1881). From these experimentally isolated crystals, a three dimensional (3-D) structure for the PR receptor has been produced to medium resolution. The PR-LBD comprises a LBD which is substantially the same as the LBD of the AR-LBD except that the LBD comprises a stronger bending of the helices H10 and H11 and helix H9 has a length which is at least one helical turn shorter than the AR-LBD. The sequence for the wild type PR-LBD site comprises at least SEQ ID No 3 (see Figure 1). The PR-LBD-R1881 crystal complex belongs to the space group P2₁ and having the unit dimensions a = 58.40Å, b = 65.0Å, c = 71.18Å and an angle β of 95.7° and with the unit cell dimensions as presented in Table 1.

The present invention also demonstrates the surprising finding that the two independent molecules in the crystal structure of hPR LBD-R1881 exhibit different modes of ligand binding. One orientation pf R1881 in one monomer resembles that of R1881 in the hAR LBD complex while in the second monomer R1881 is orientated similar to progesterone in the hPR LBD-progesterone complex. Thus it may be possible to design ligands that selectively bind to either one or both of the monomers in the hPR-LBD-ligand complex, thereby dissociating desirable preventative and/or therapeutic effects from undesirable side effects of PR ligands.

A partial homology model of the AR receptor has been created based on the experimentally derived hPR-LBD-progesterone crystal complex. This homology model captures the essential difference in binding between the AR-LBD crystal and AR-LBD model structures. This homology model also highlights the differences with respect to the secondary structure alignment between the model structure of the present invention and that from other published models.

By way of example, the model structure of the present invention differs from other published models [Yong, 1998] with respect to the secondary structure alignment. Yong [1998] based their model on the crystal structure of the RARα LBD [Bourguet, 1995]. The secondary structure assignment by Yong *et al.* as compared to the hAR LBD crystal structure is similar between helices H3 and H10, but the assignment differs most for helices H11, H12 and the additional helix at the C-terminal end.

The ligand binding pocket interactions of the present invention have been determined using the hAR LBD-R1881 crystal structure and the hPR LBD-R1881 complex.

Based on a comparison of the LBP interactions, the differences in ligand binding specificities between the AR and PR can be determined. Using these differences, the ability of a ligand to bind to either or both of the AR and PR may be predicted. Hence, if it is known that one tissue possesses solely one form of an AR and/or PR receptor, then it may be possible to confer a degree of tissue specificity to a ligand by designing a ligand to bind the predominant form of the AR and/or PR present in that tissue.

Thus, the present invention also provides an understanding of the differences between R1881 and progesterone binding to AR and PR receptors and therefore a means to design AR and PR ligands with the desired degree of efficacy.

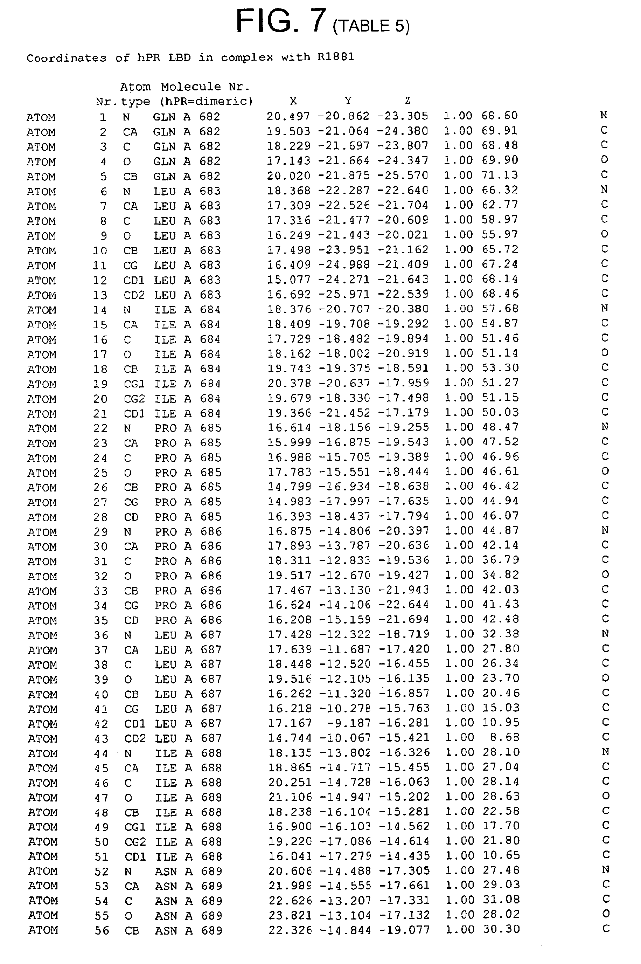

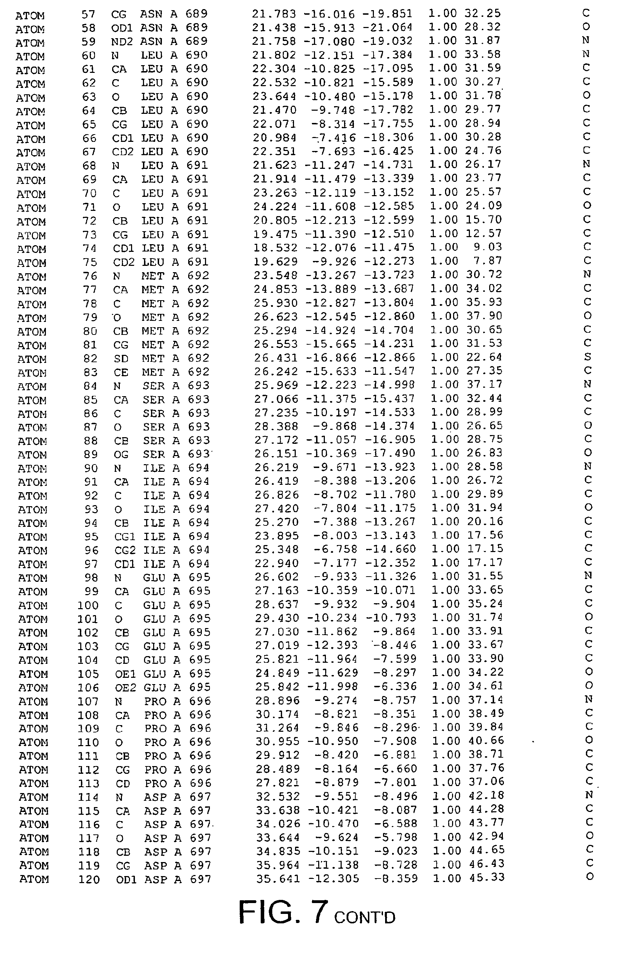

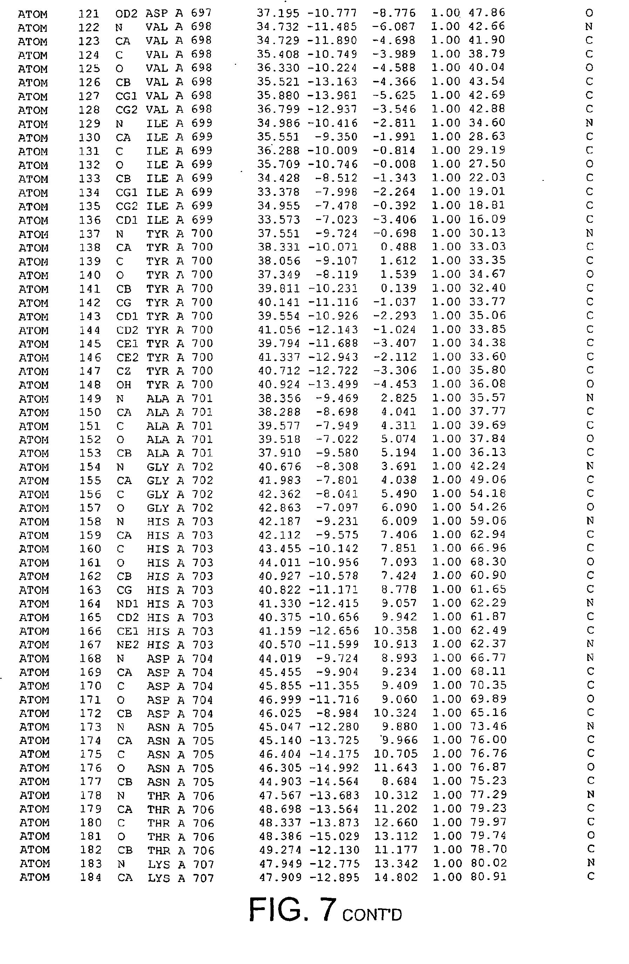

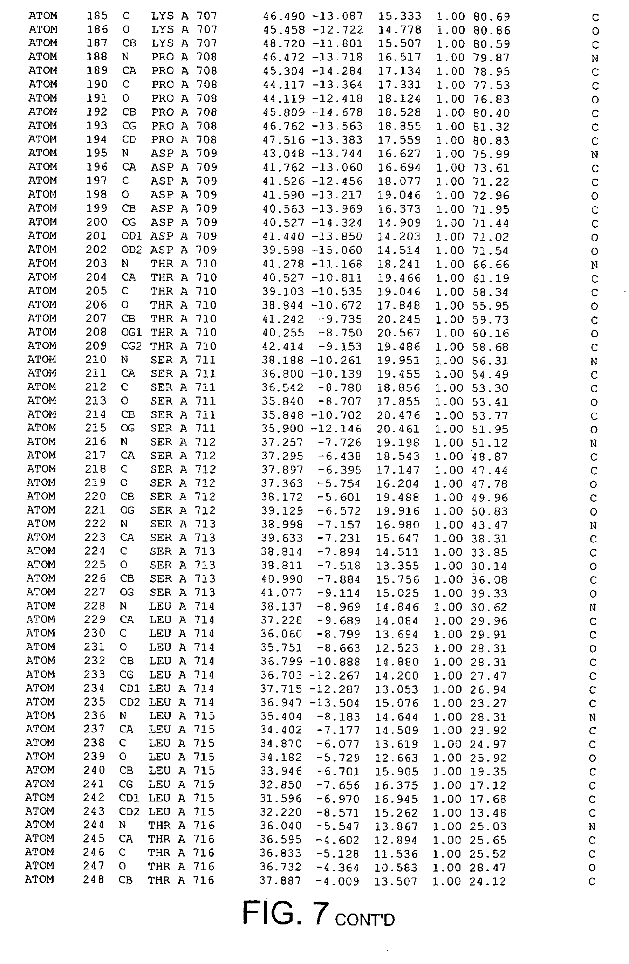

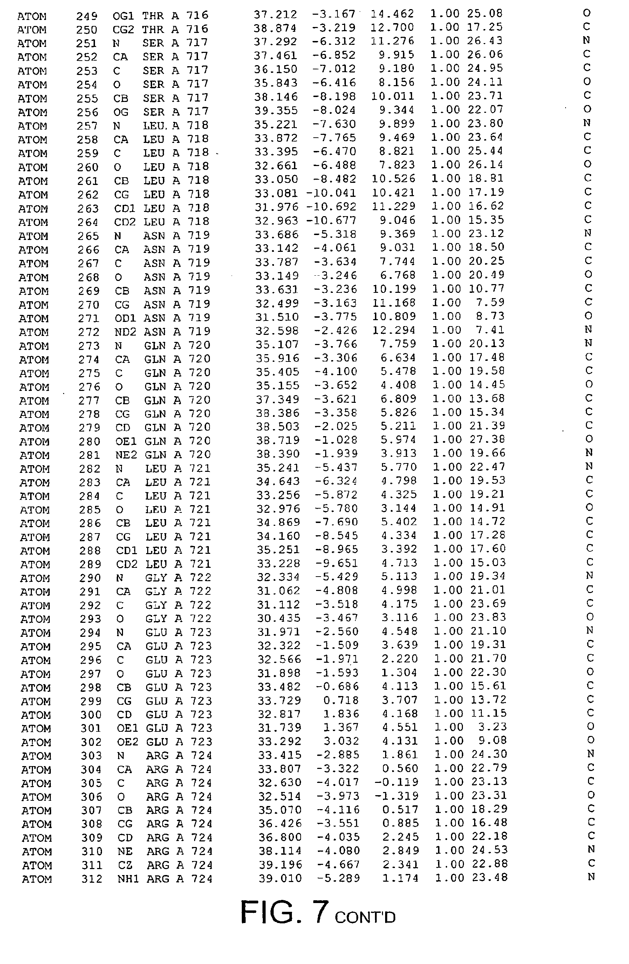

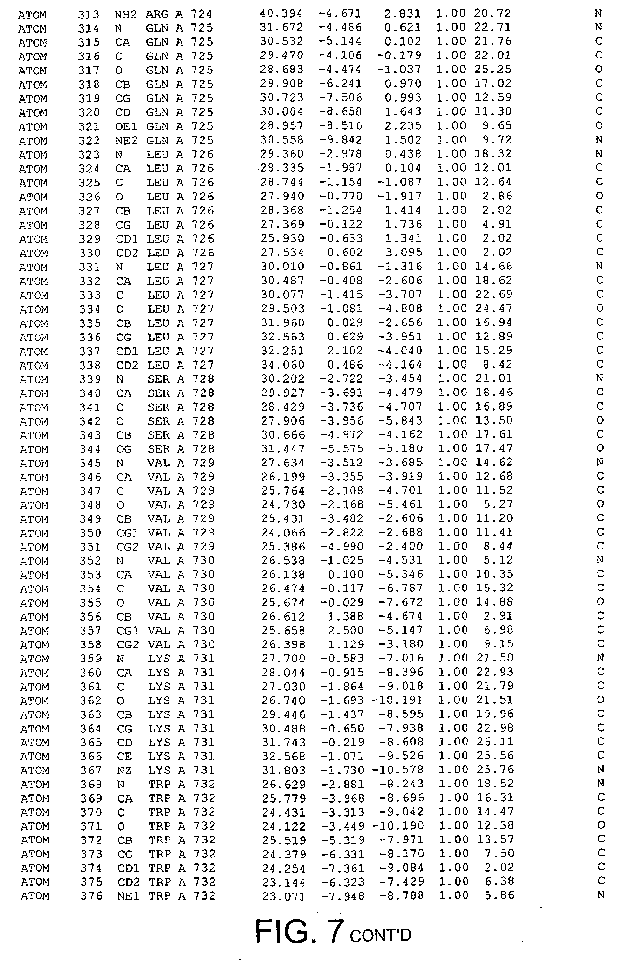

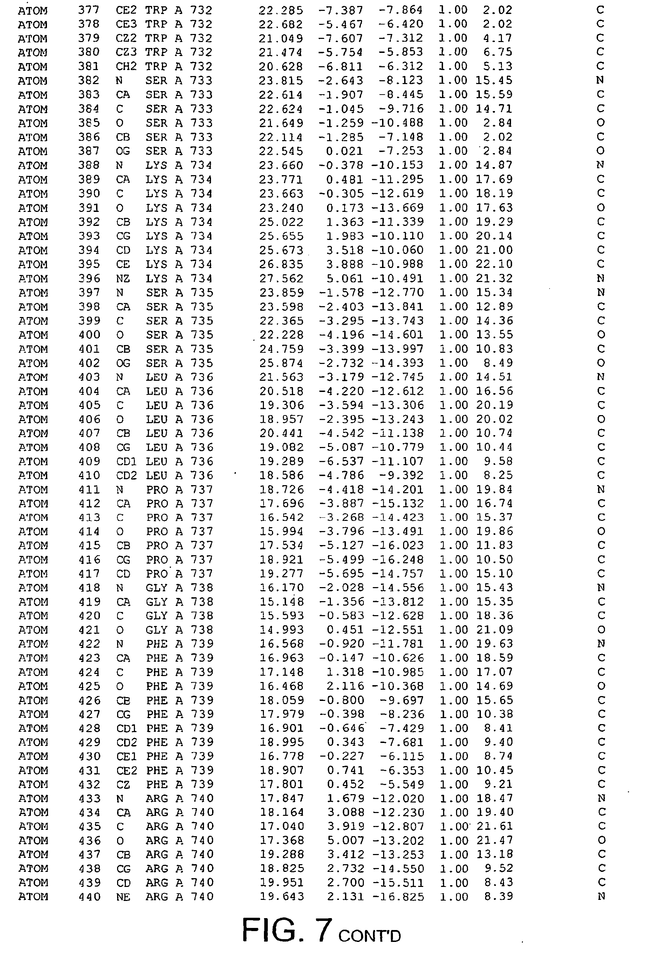

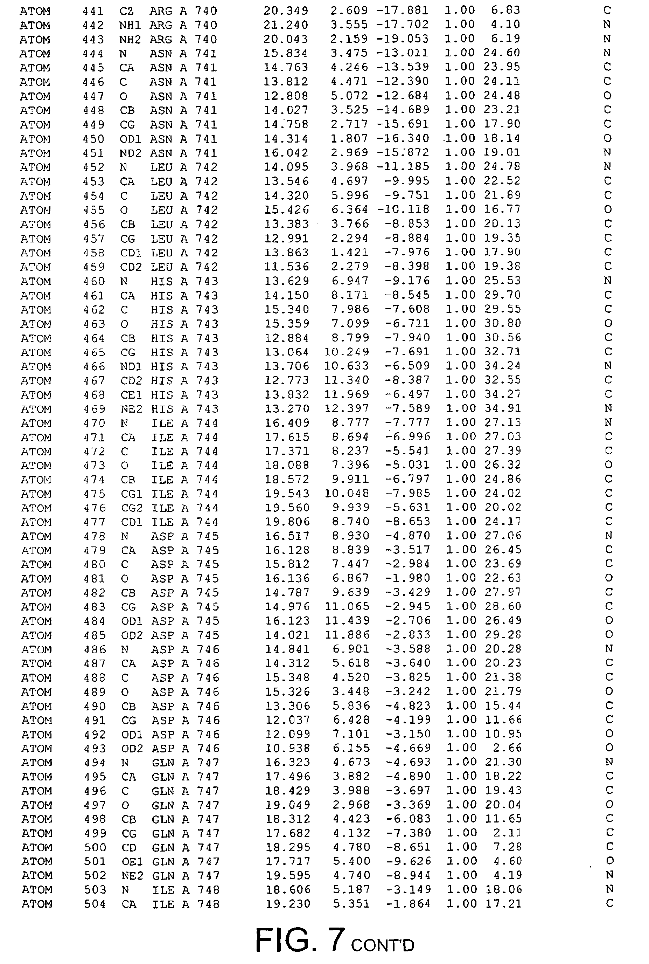

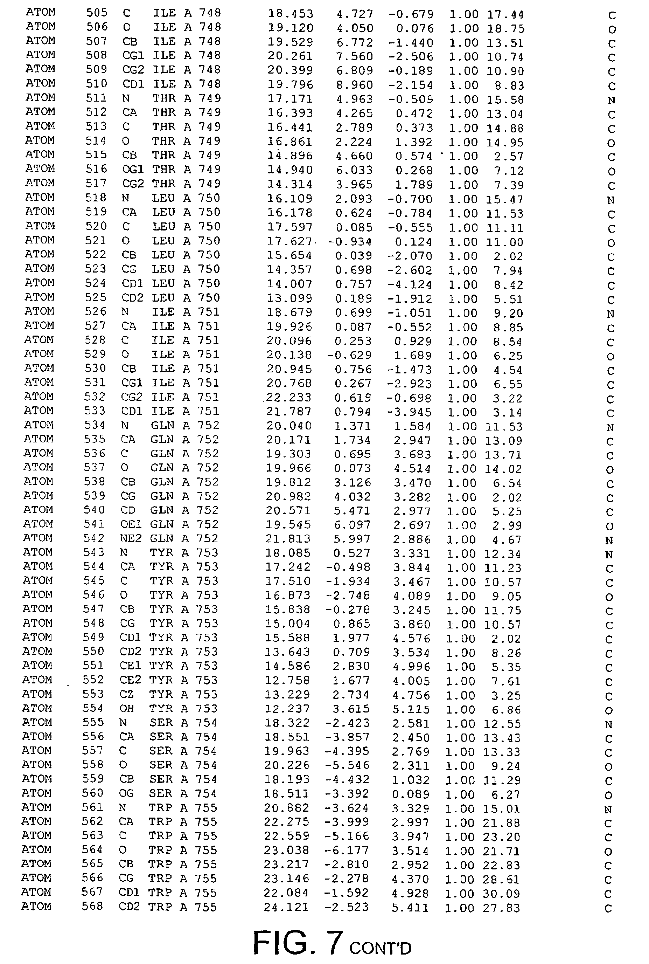

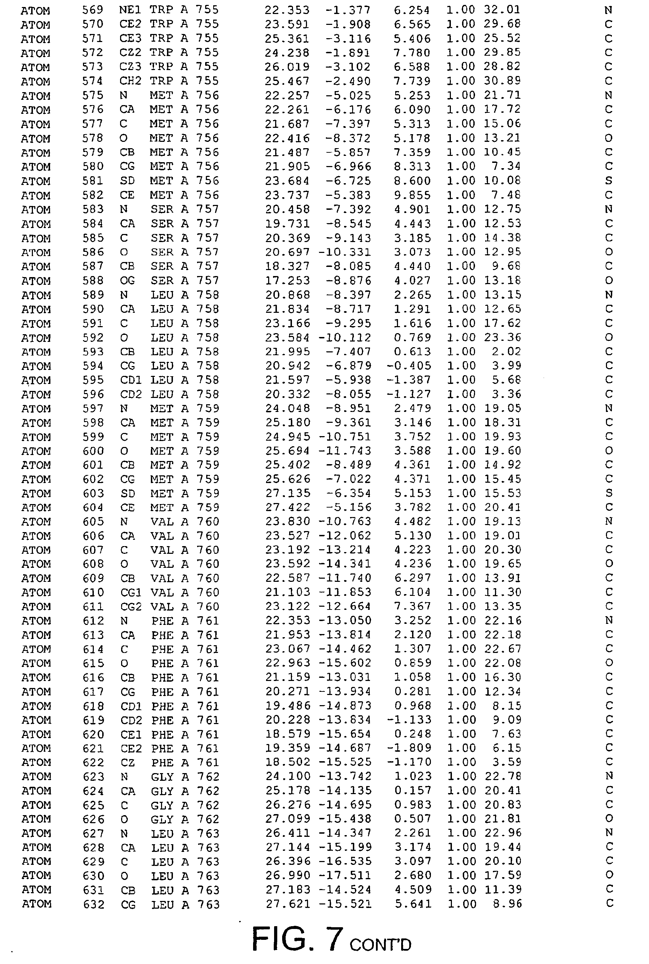

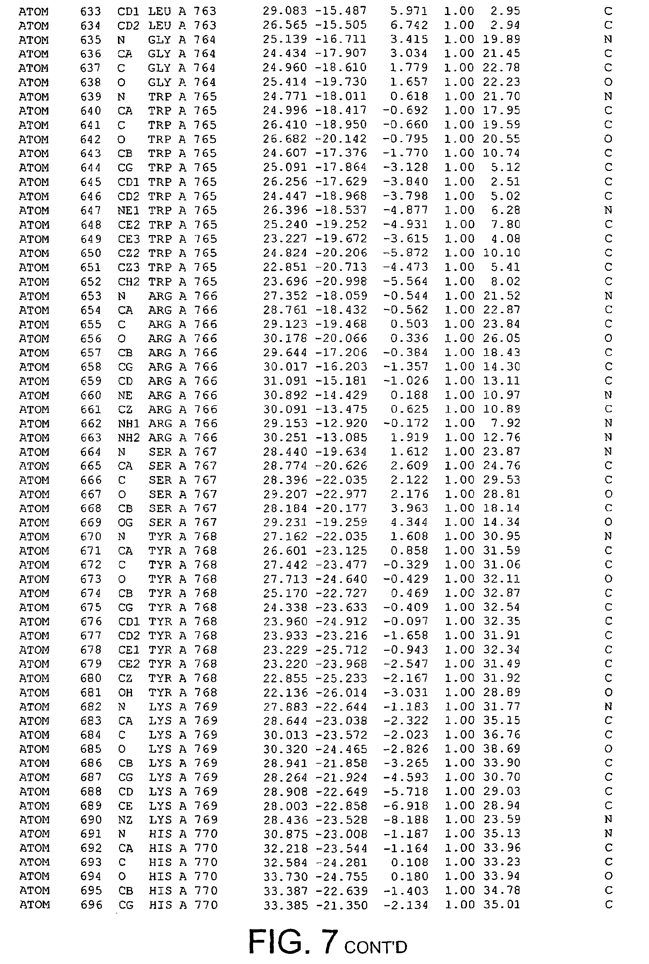

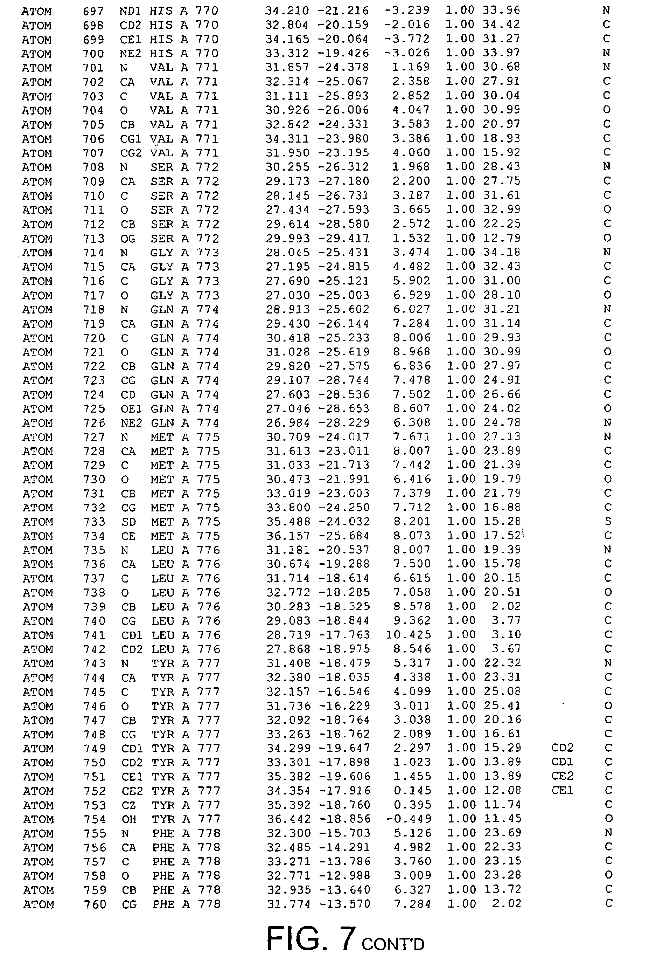

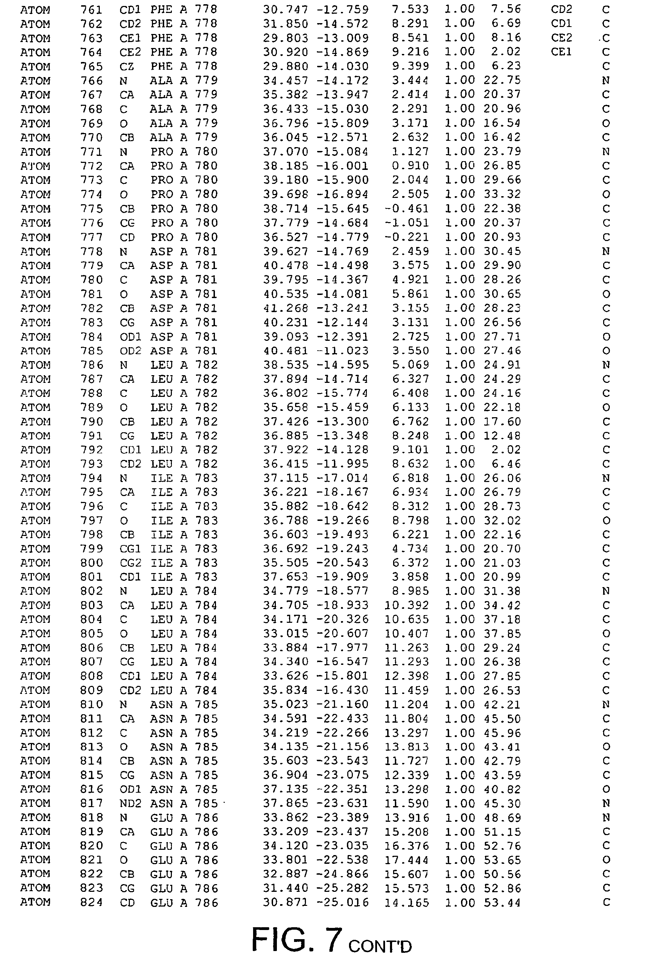

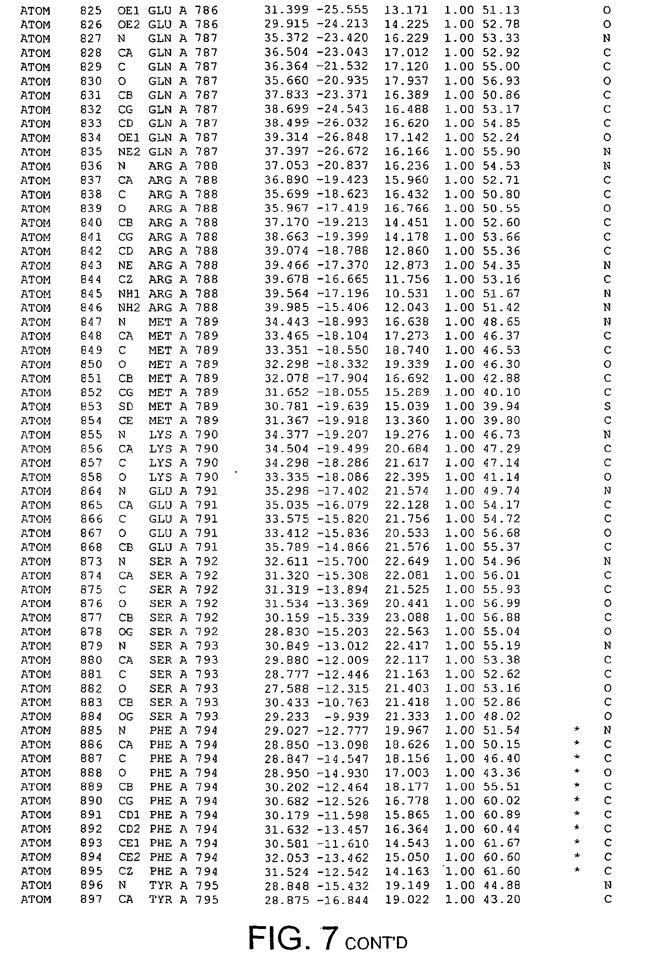

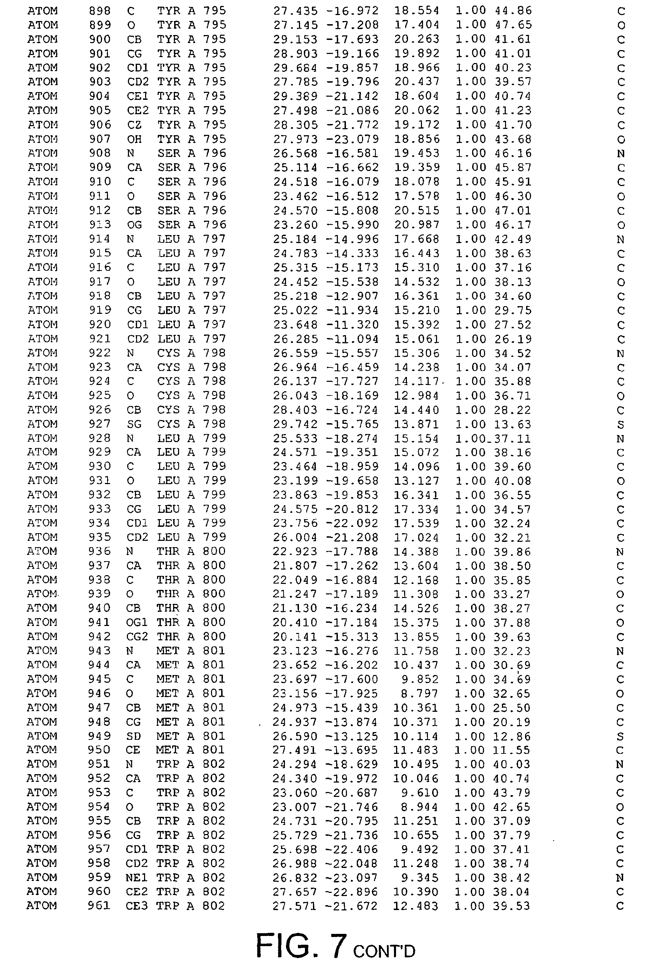

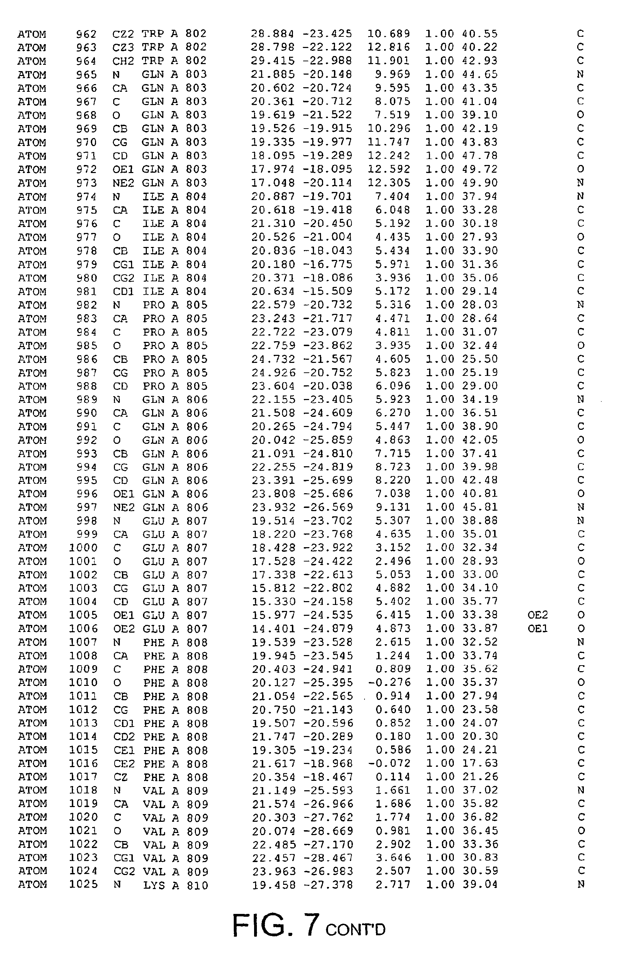

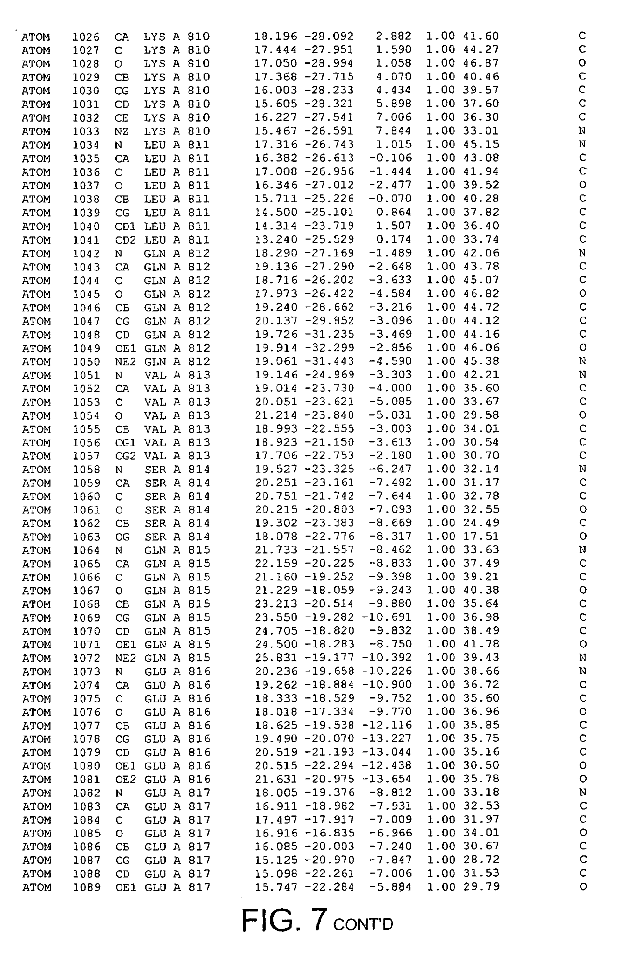

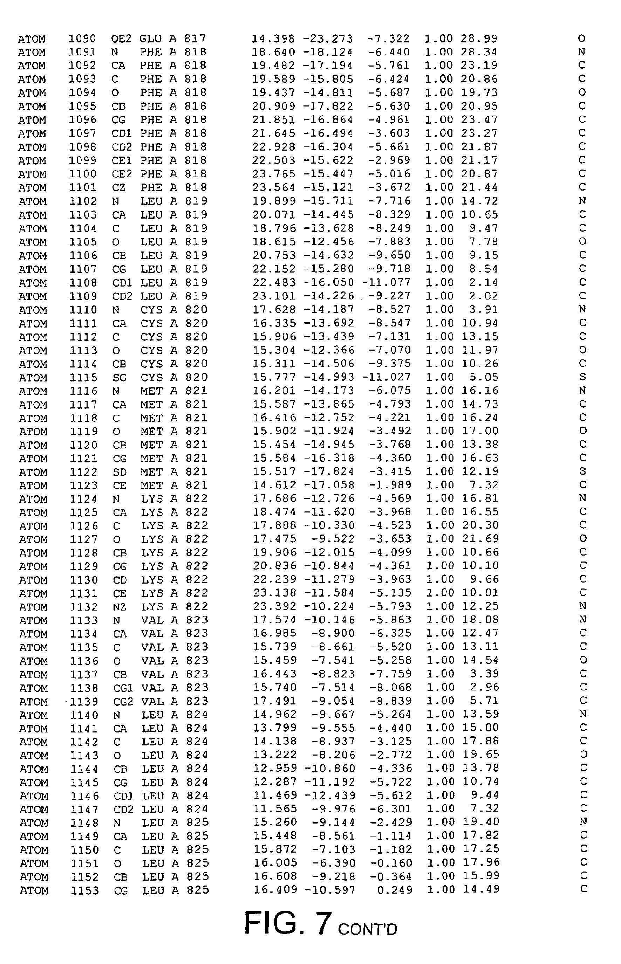

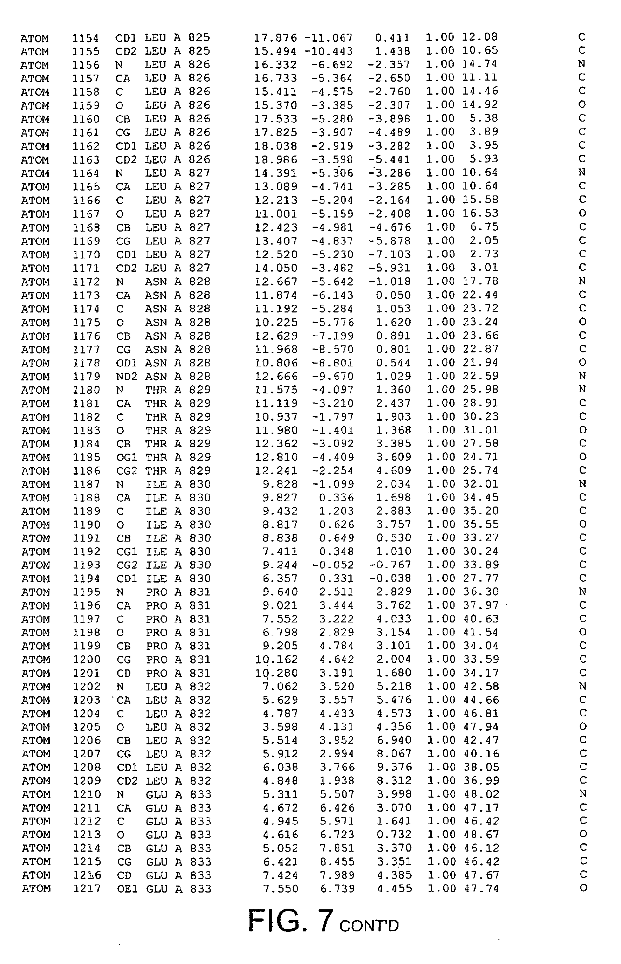

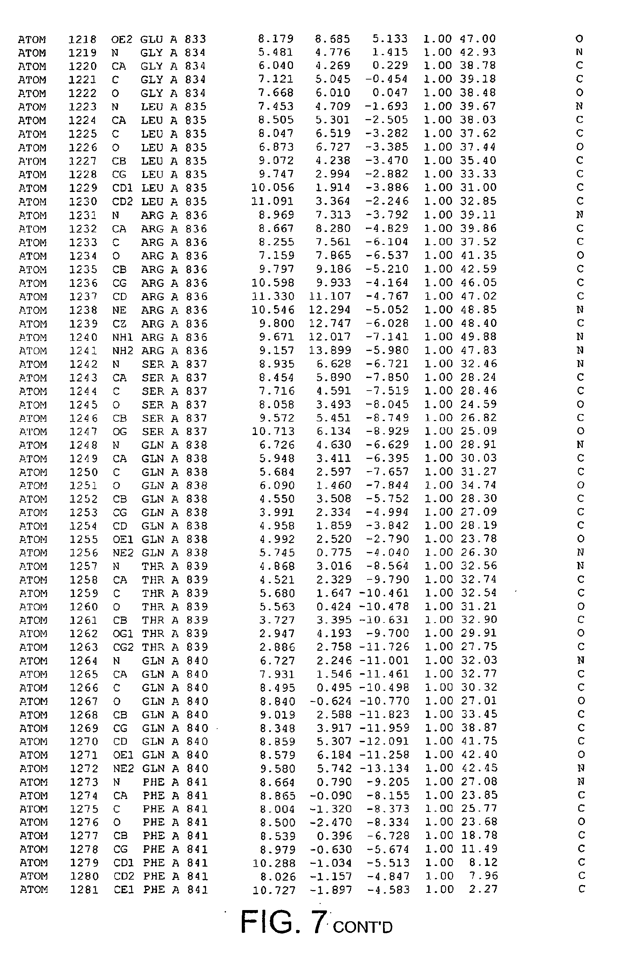

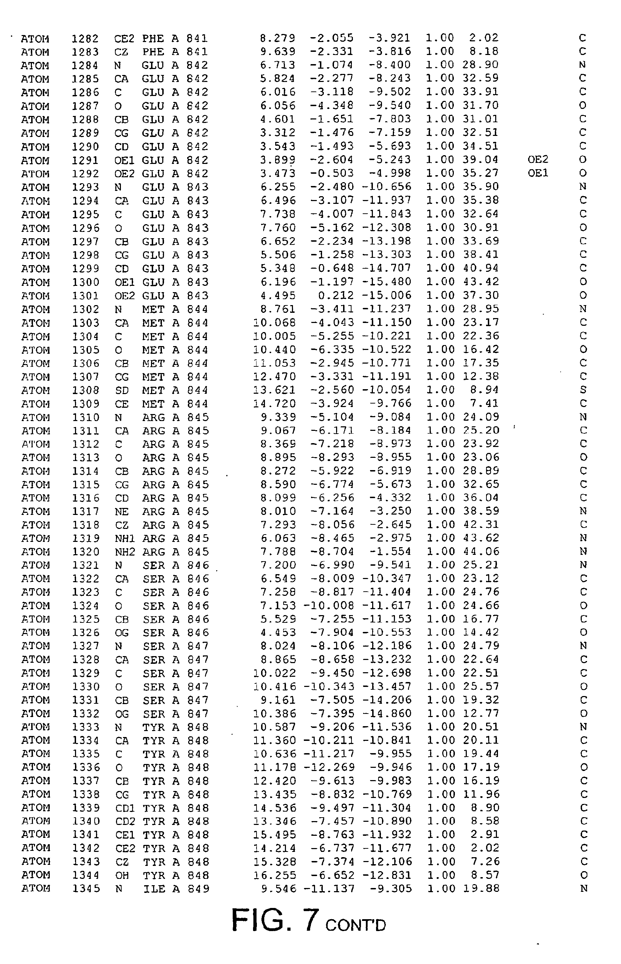

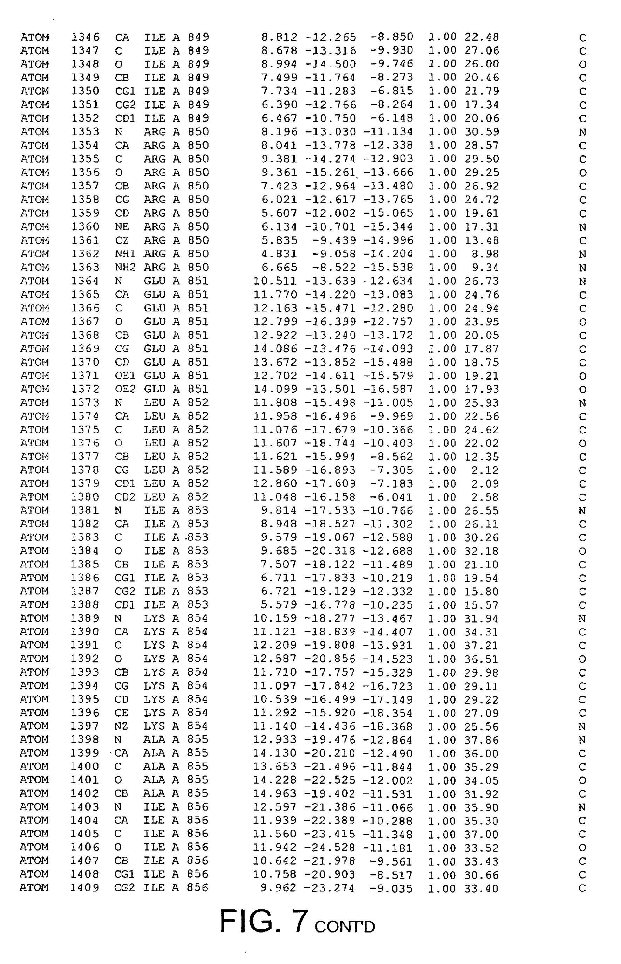

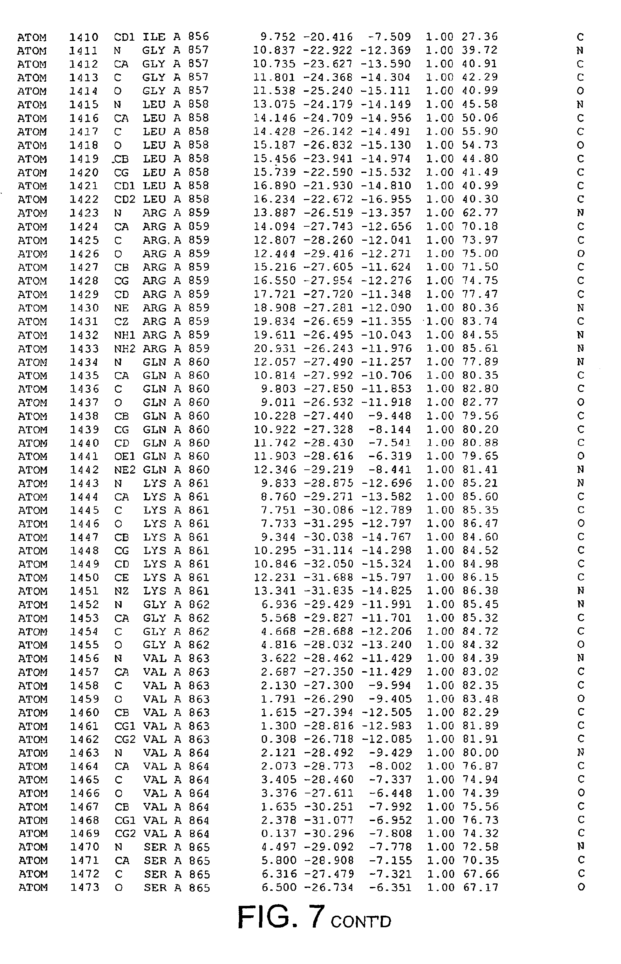

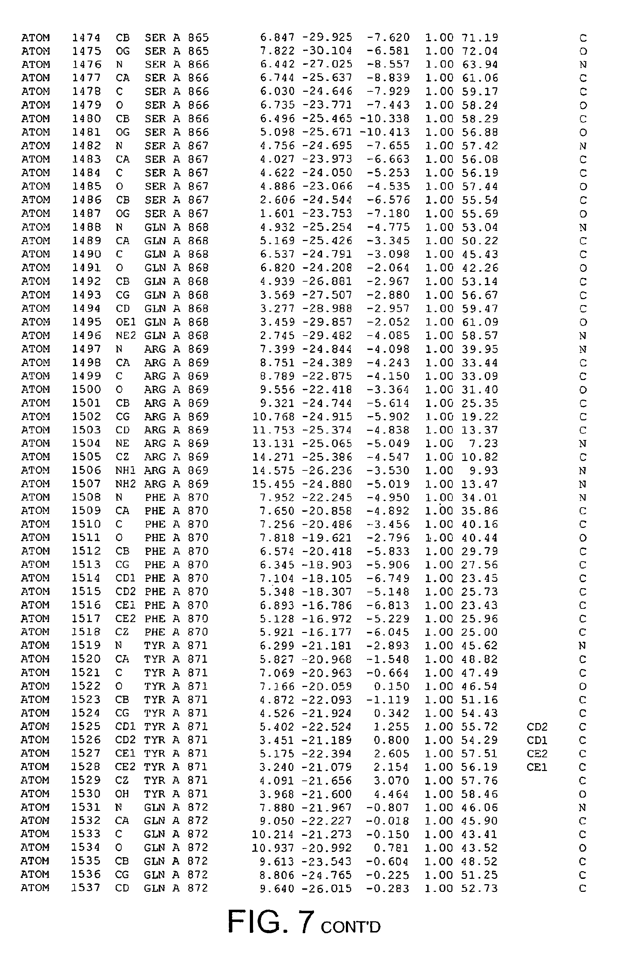

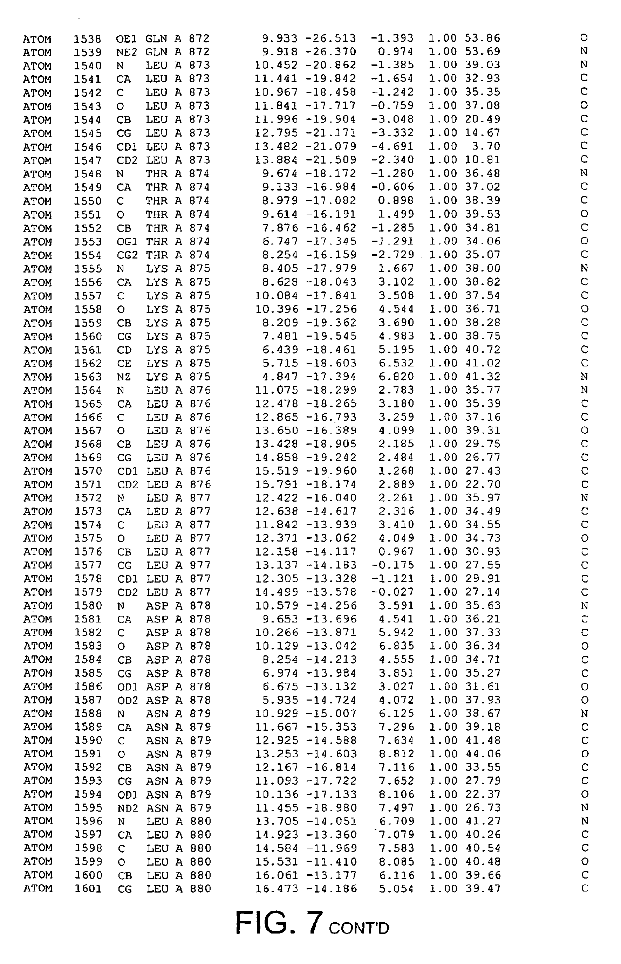

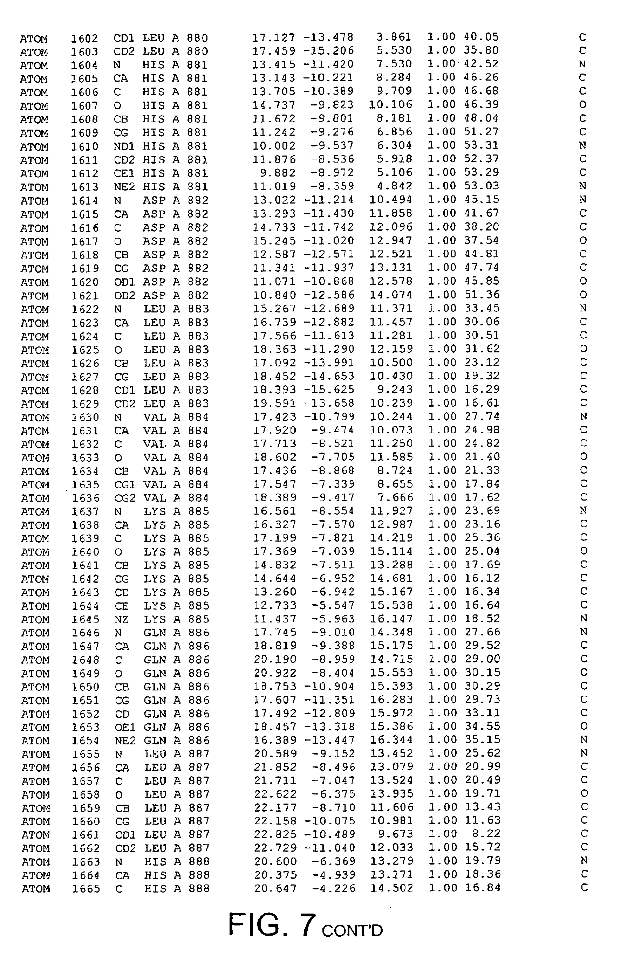

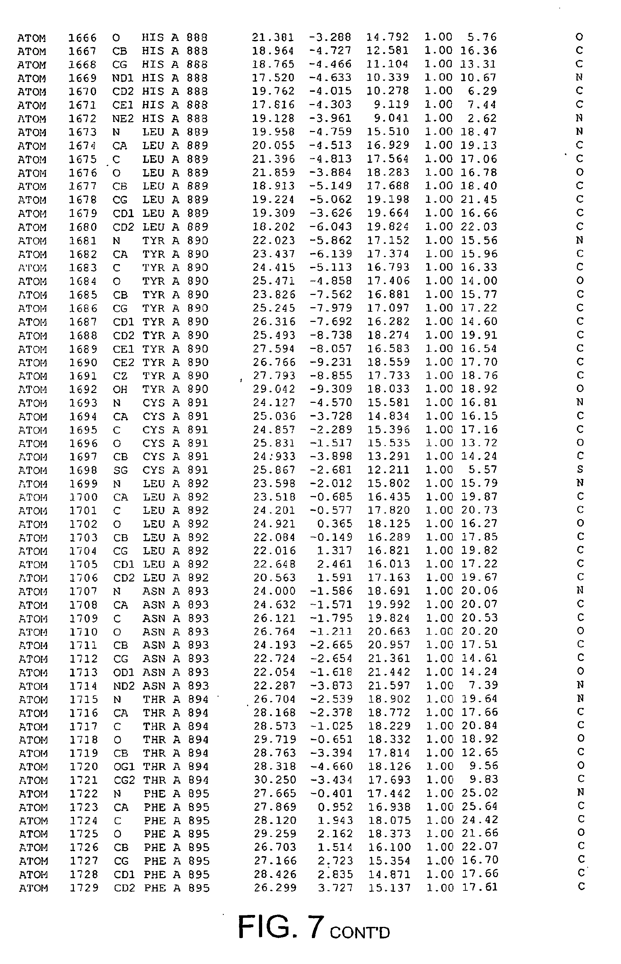

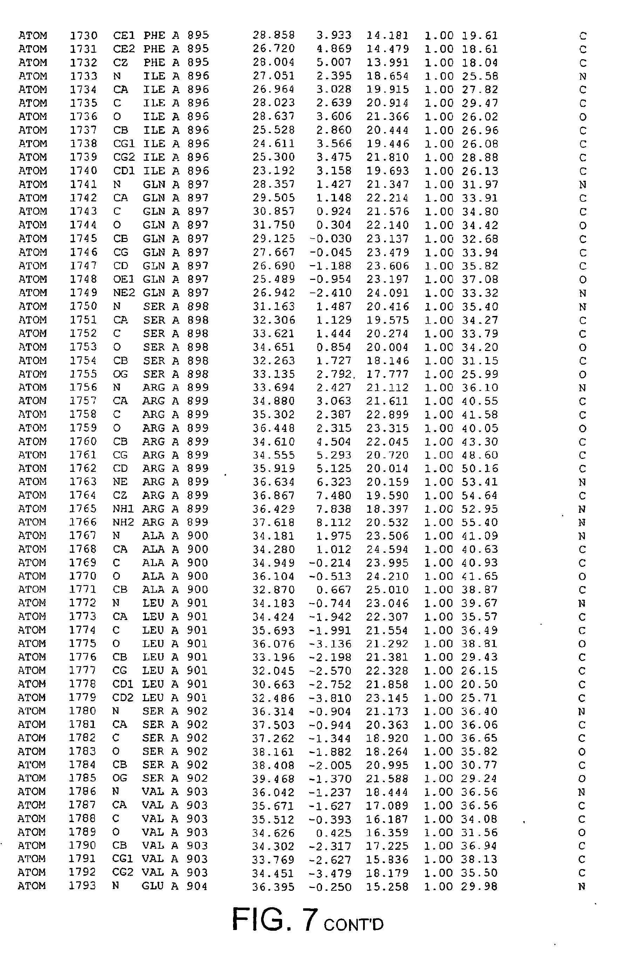

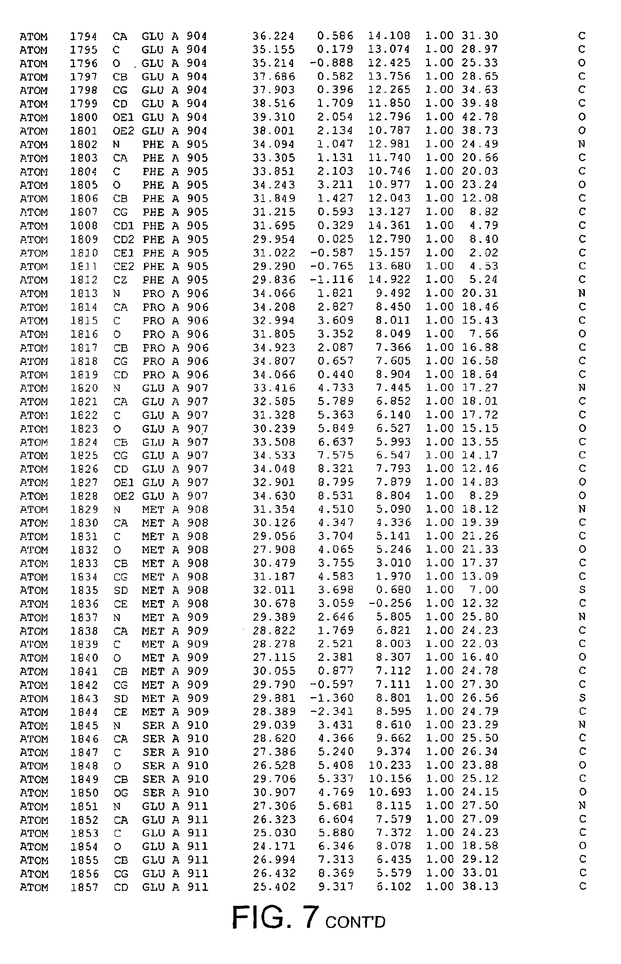

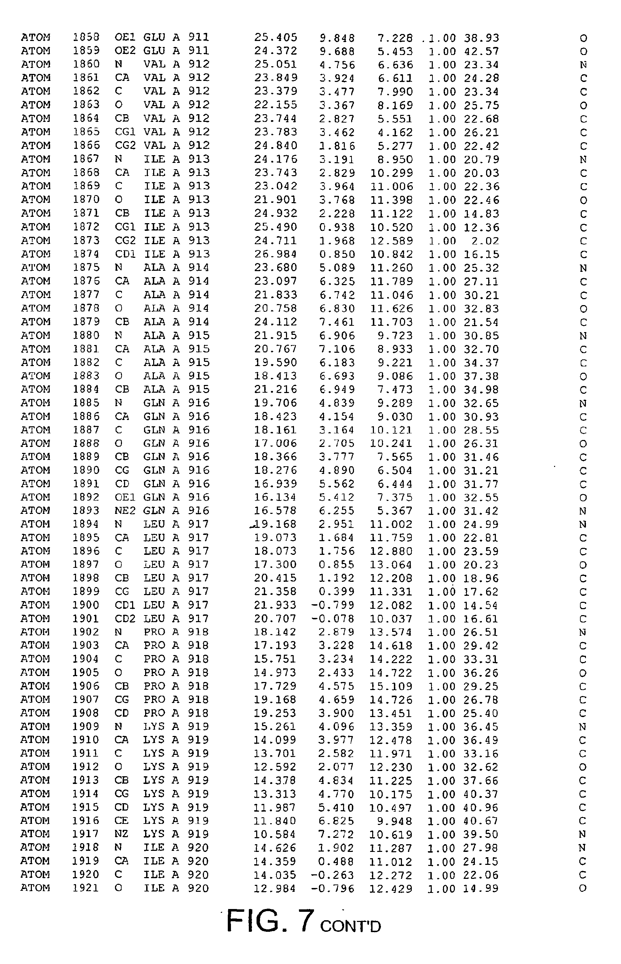

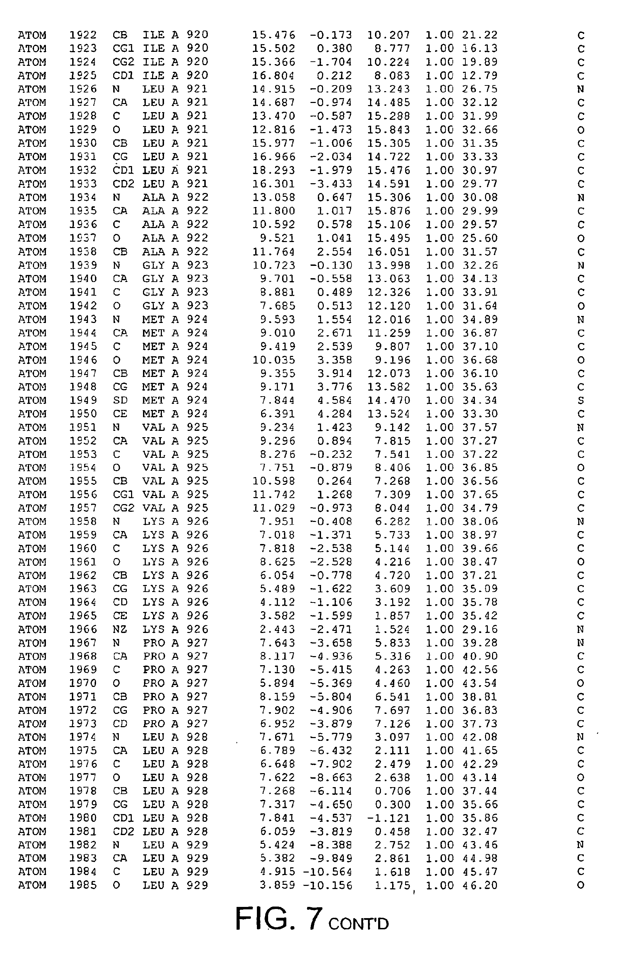

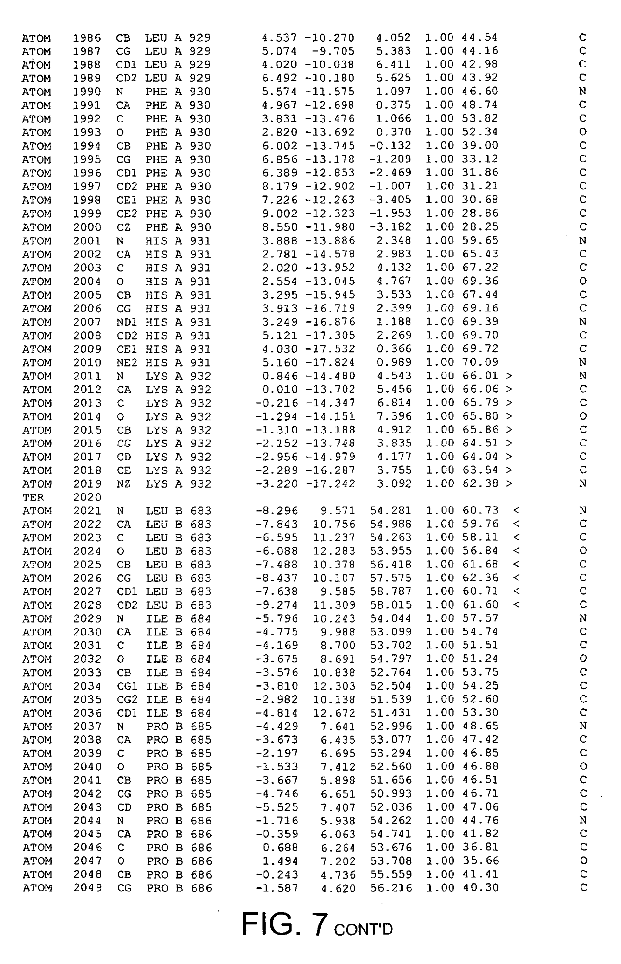

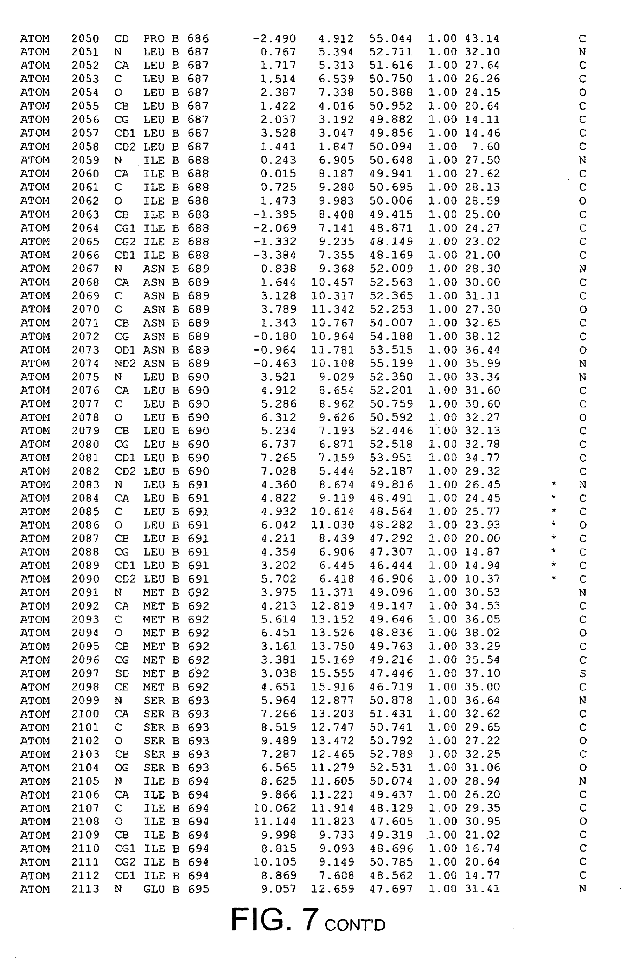

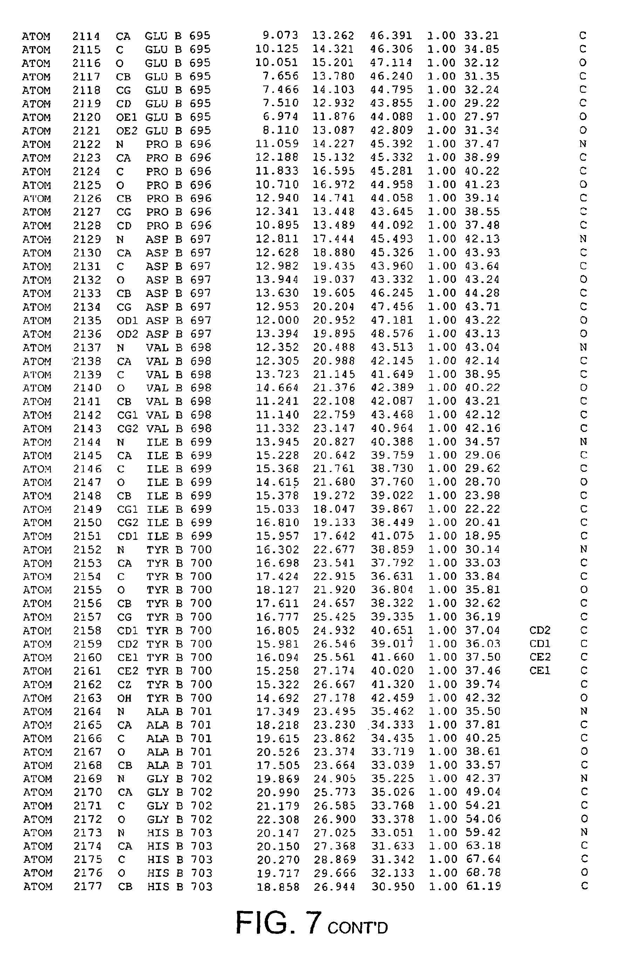

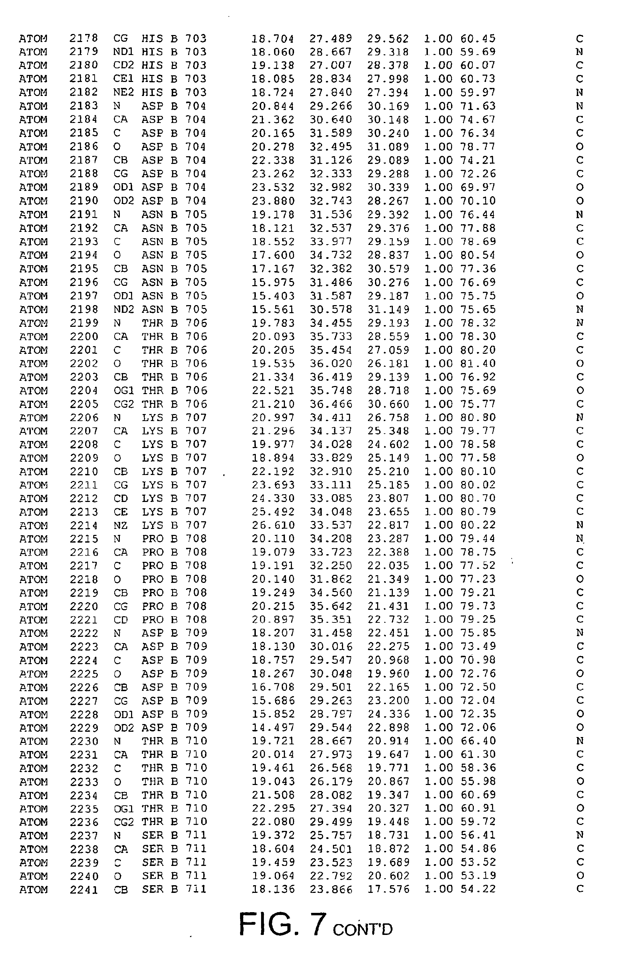

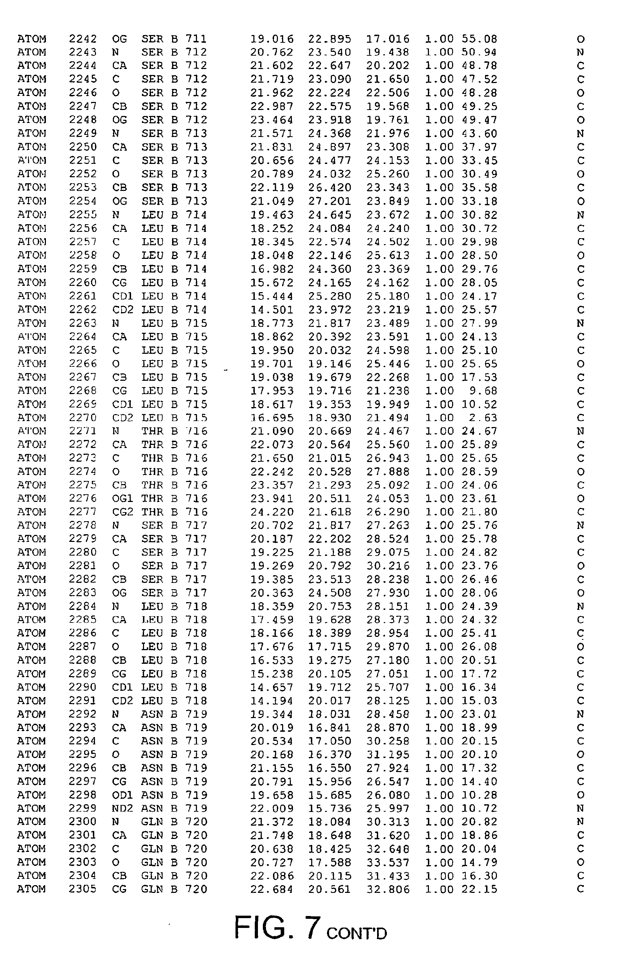

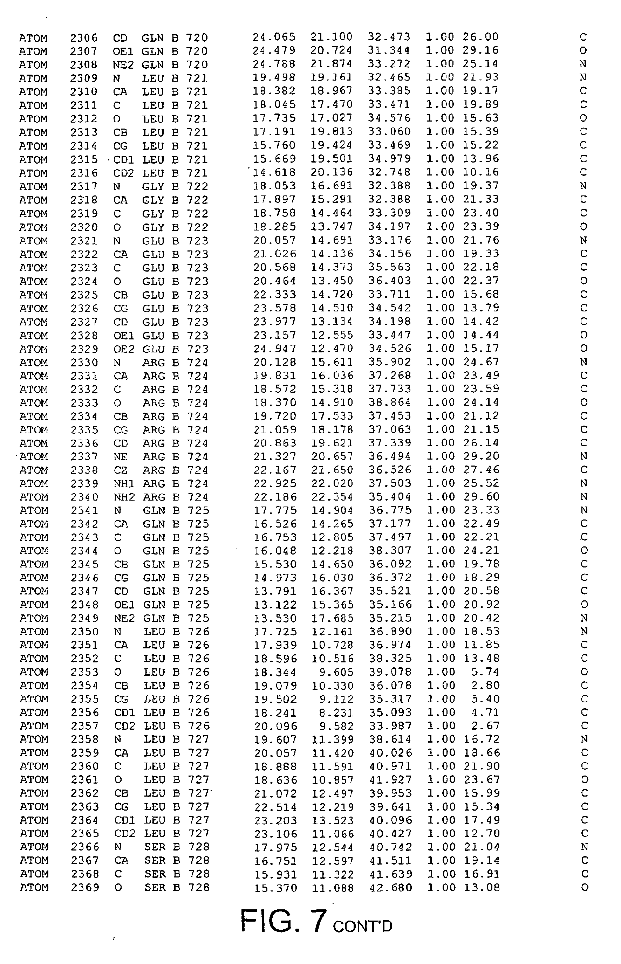

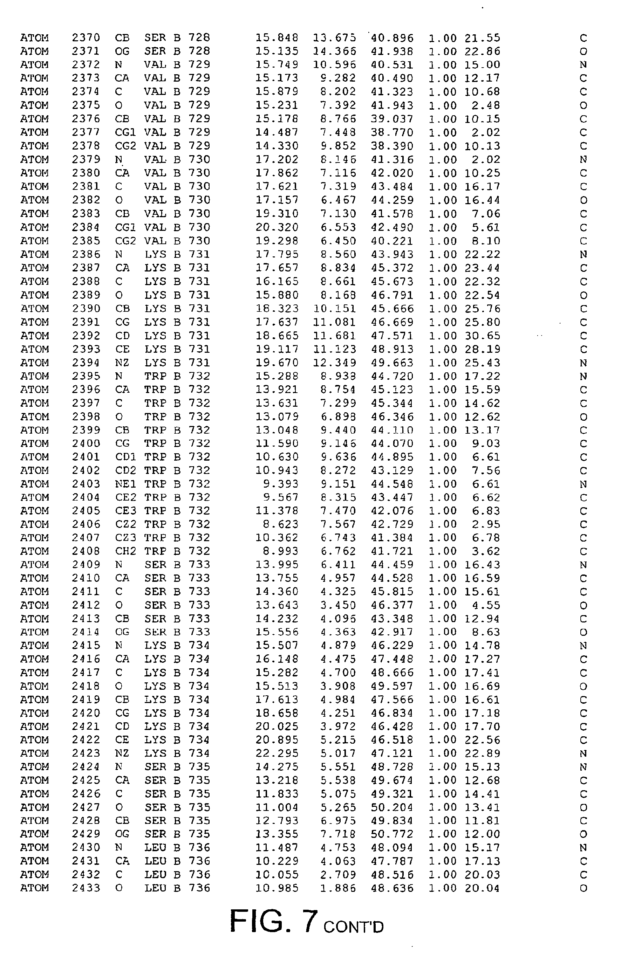

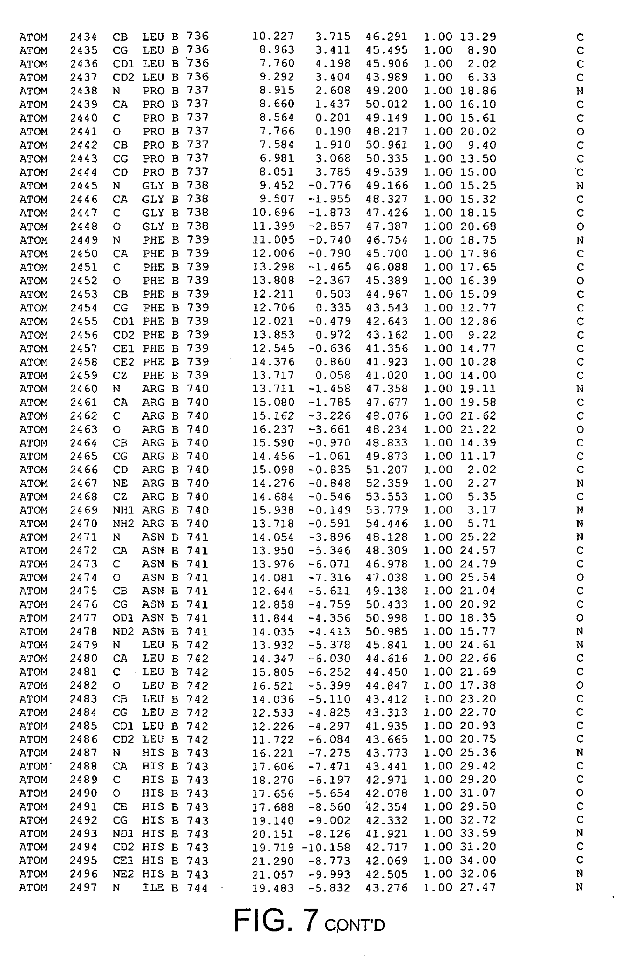

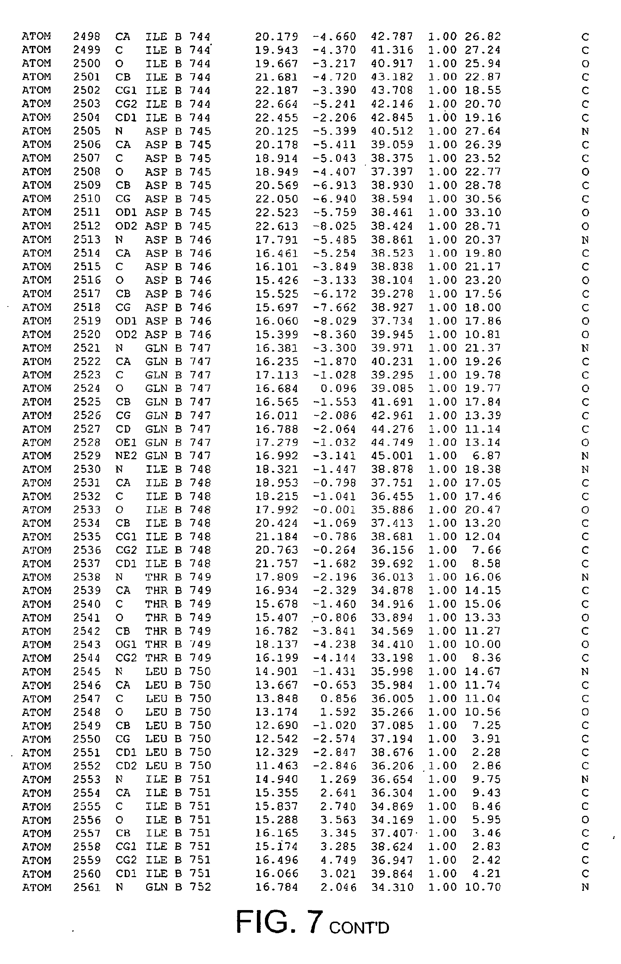

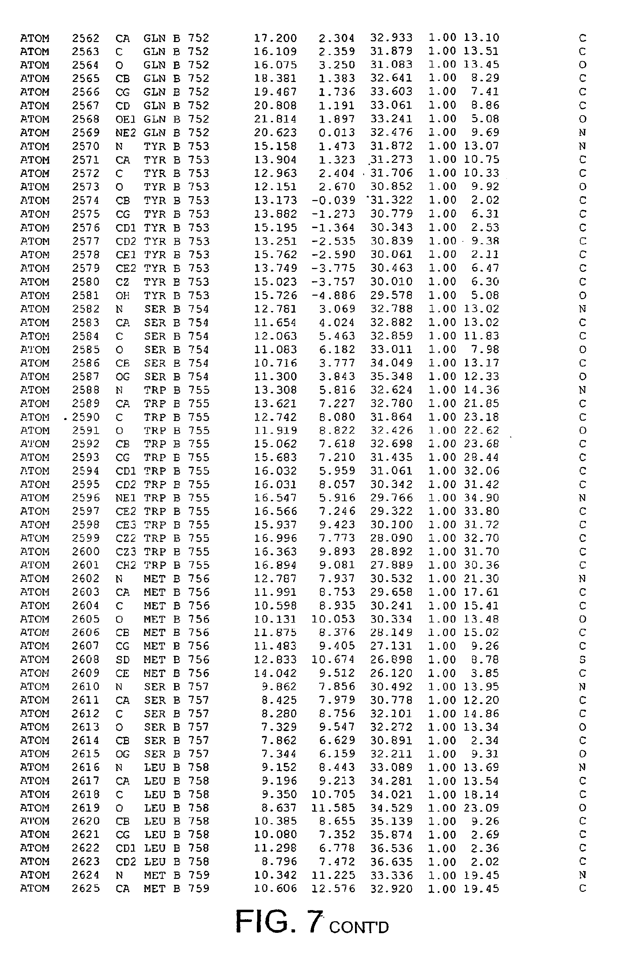

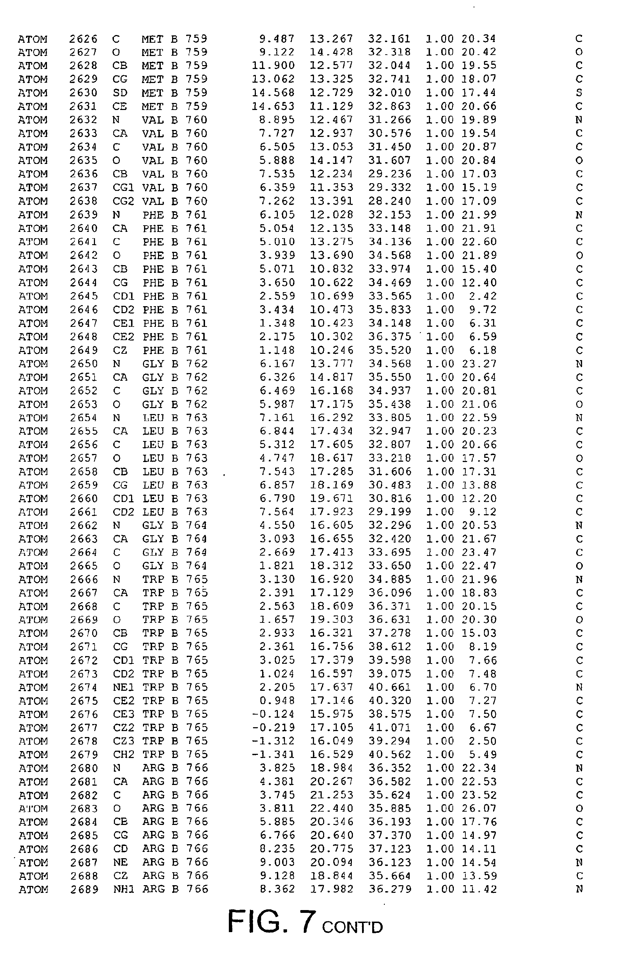

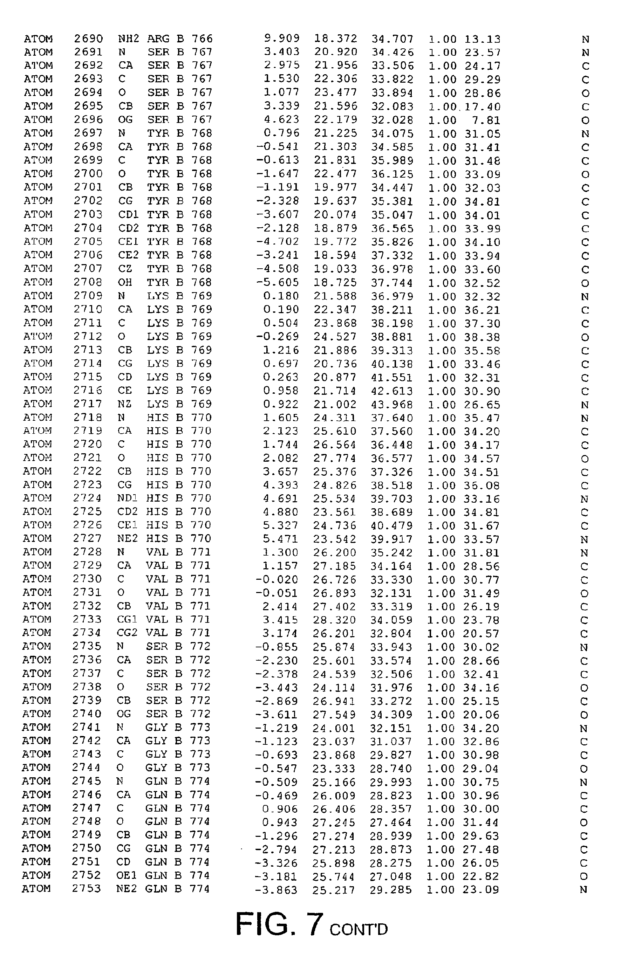

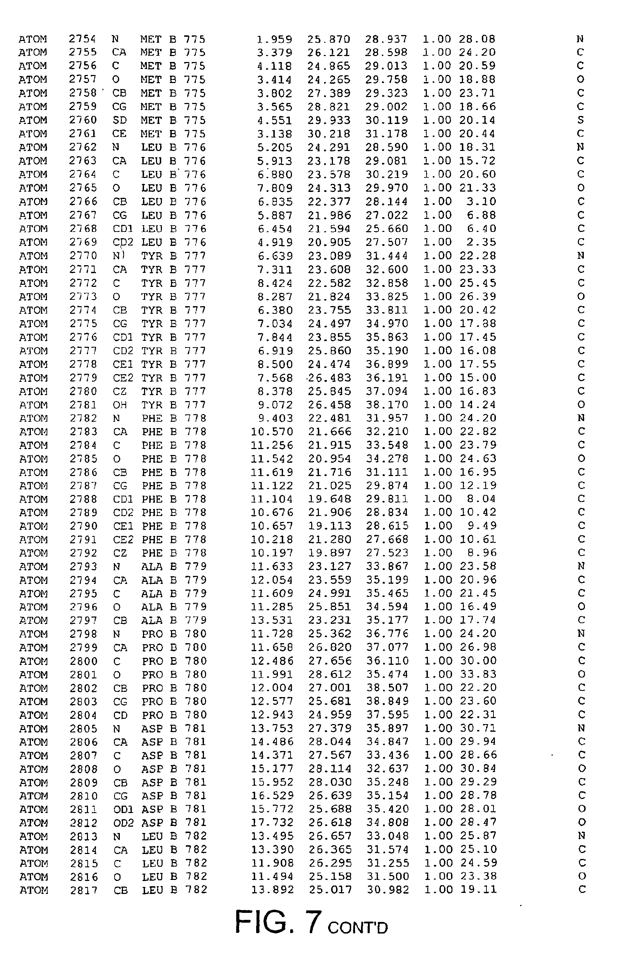

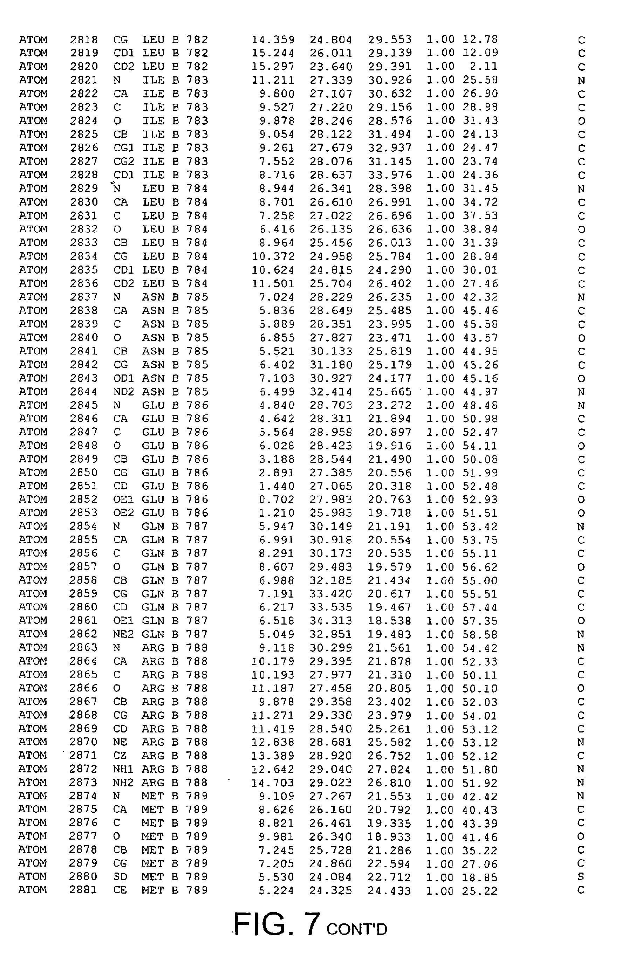

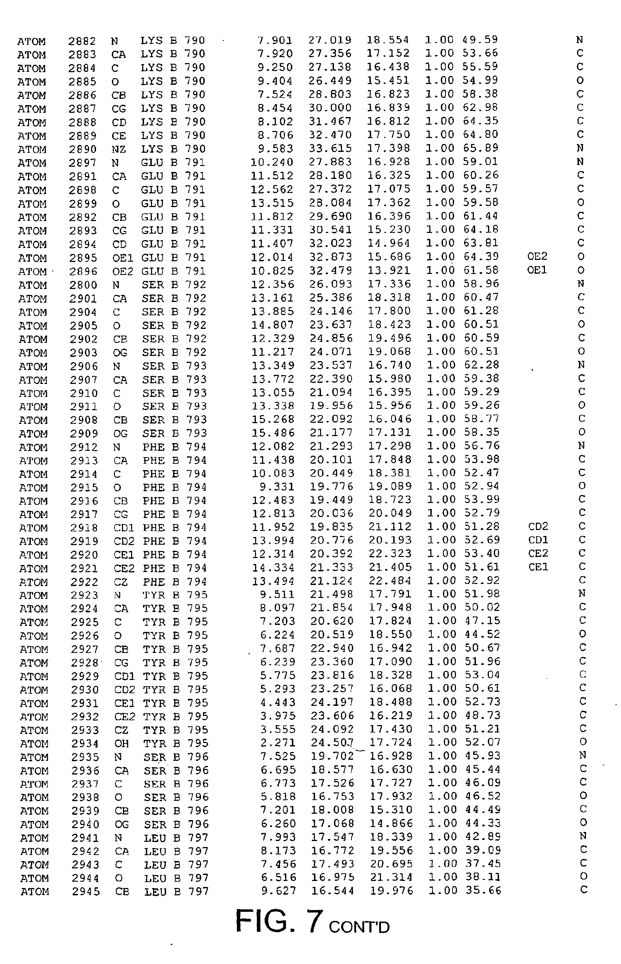

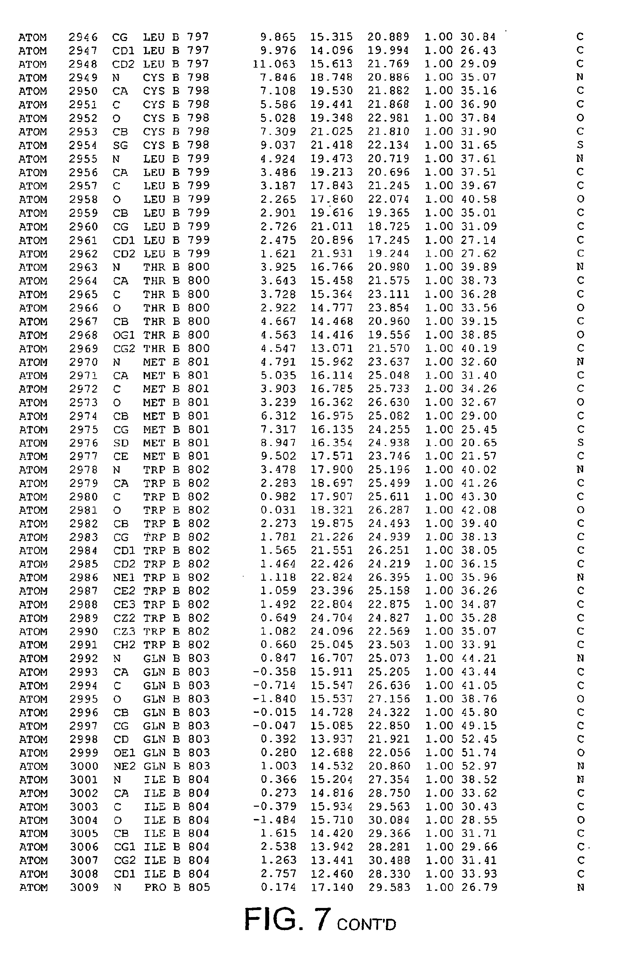

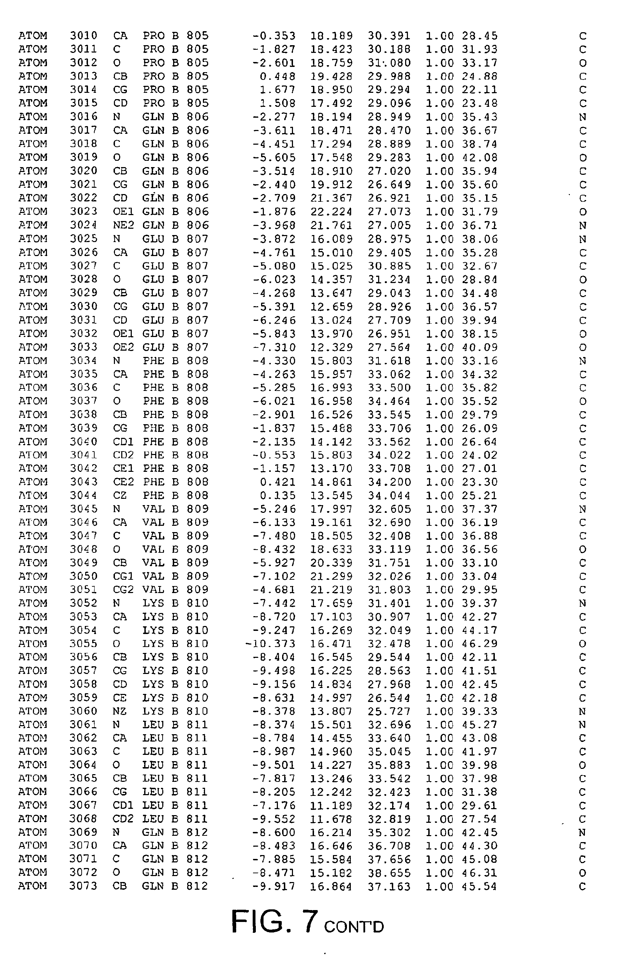

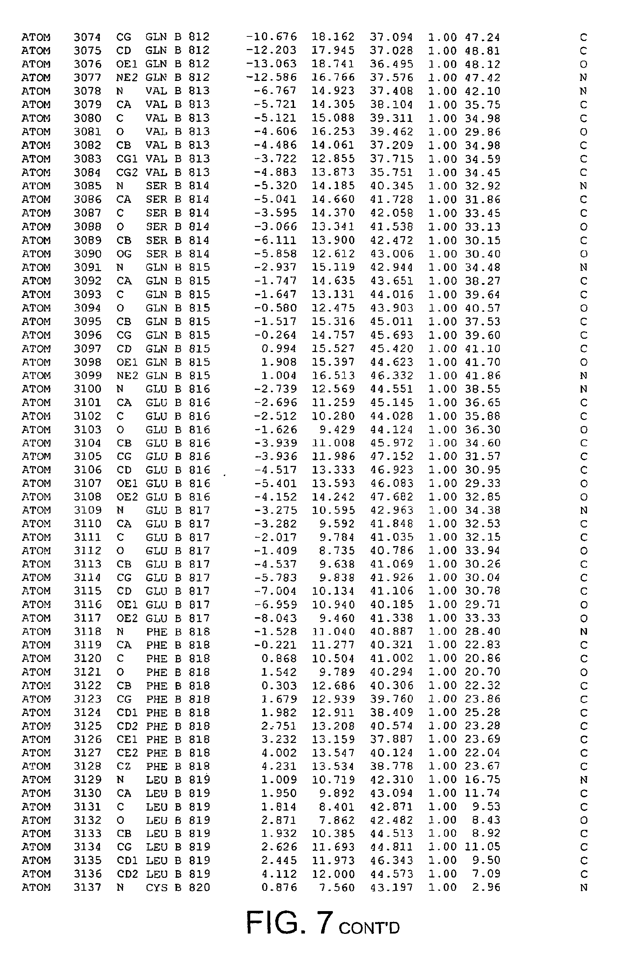

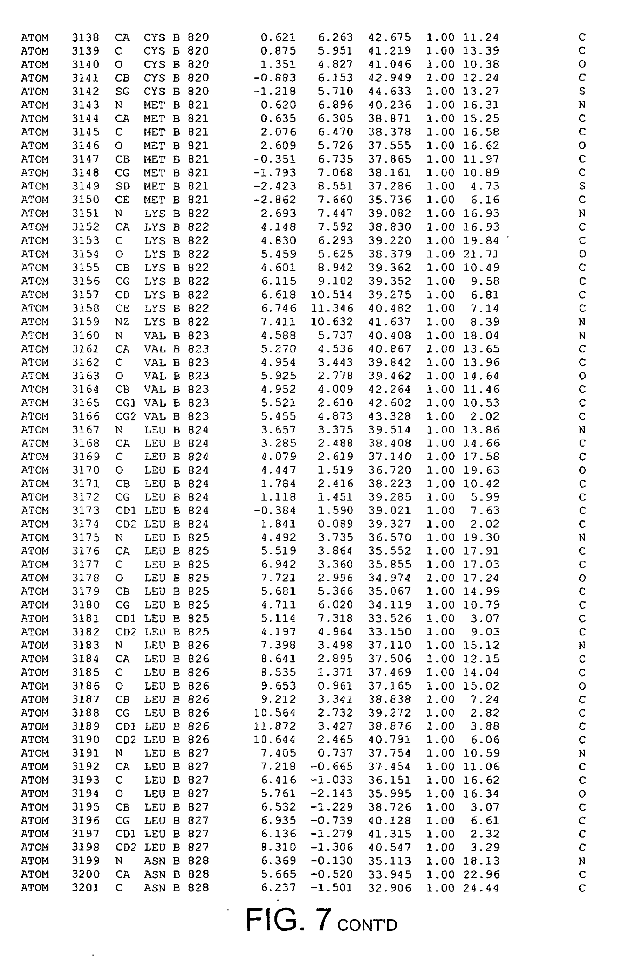

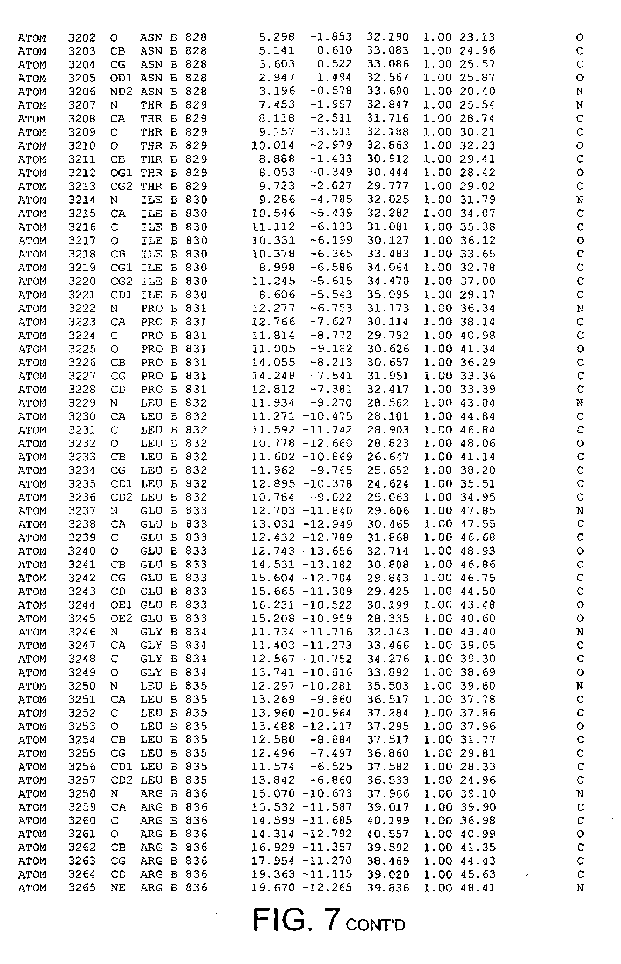

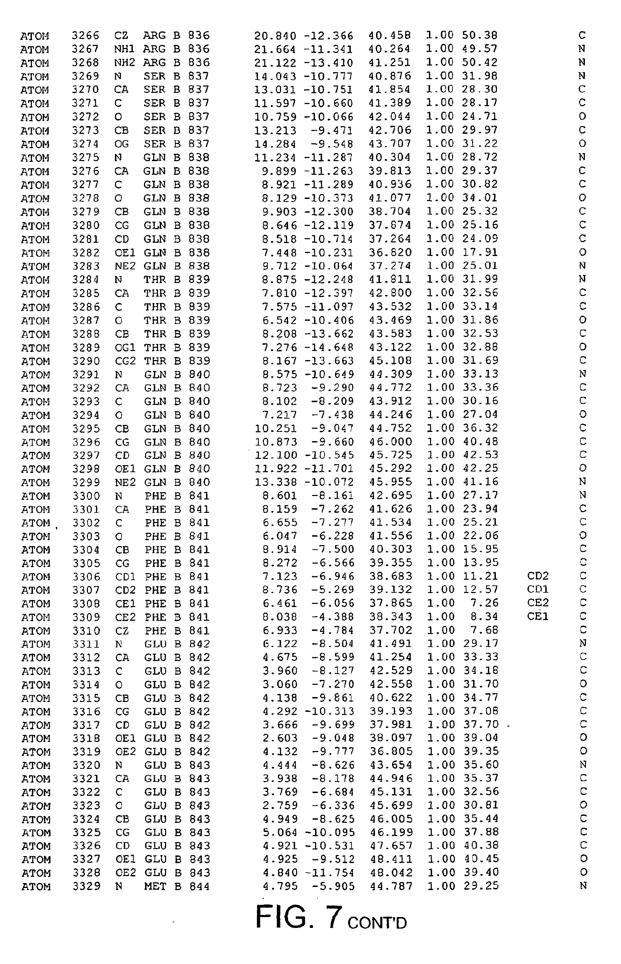

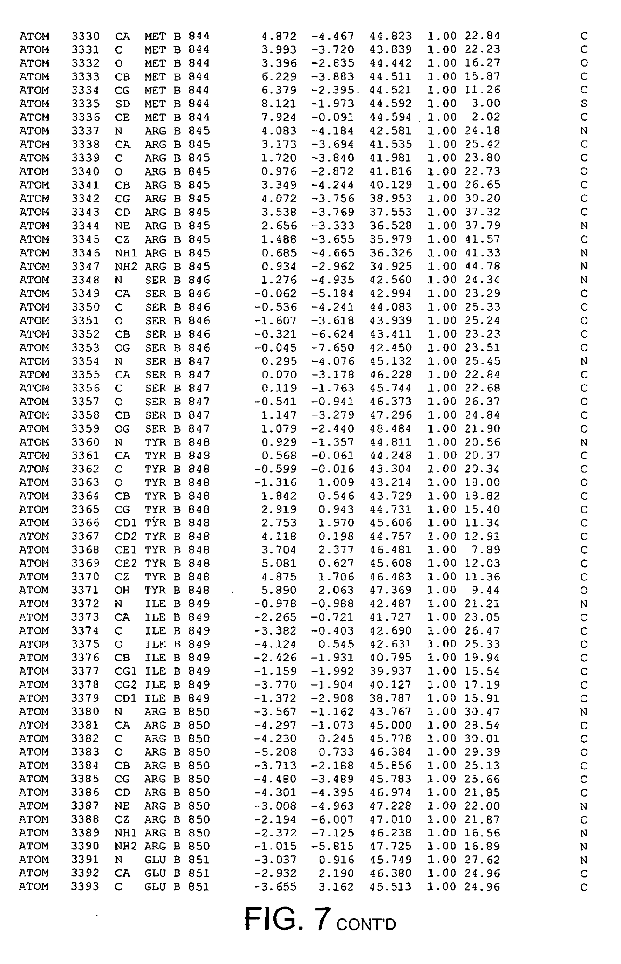

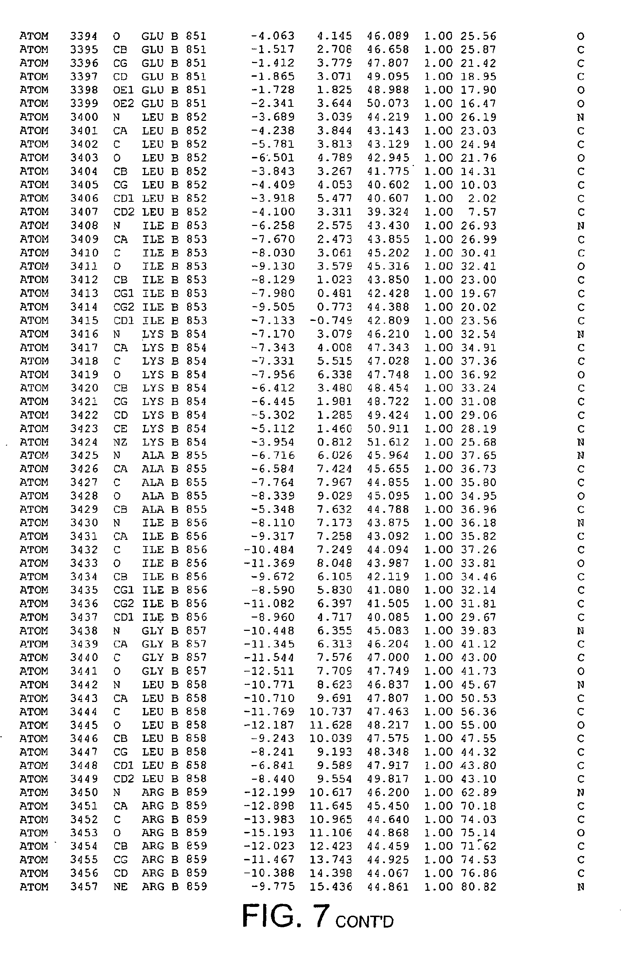

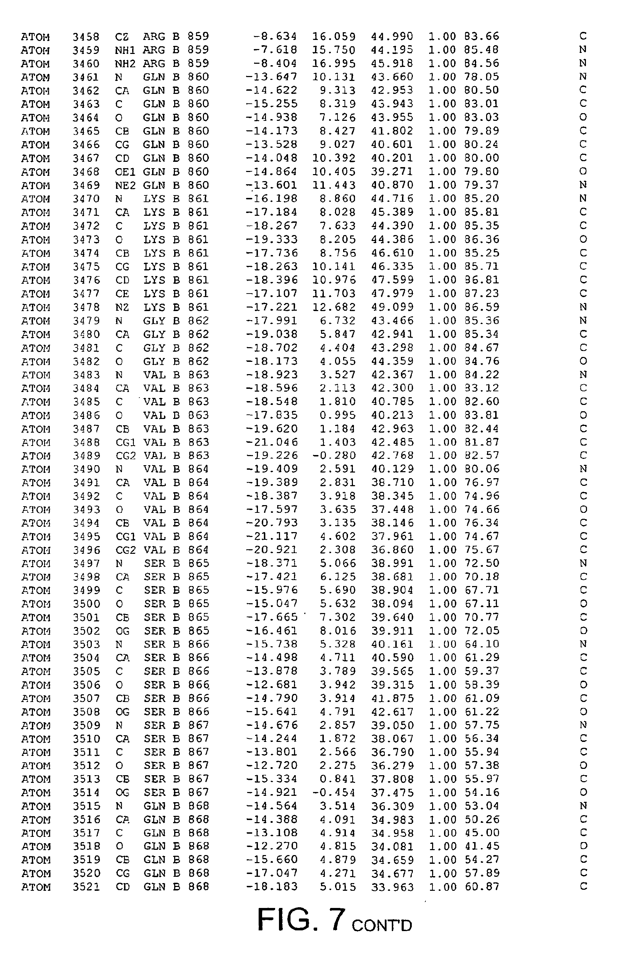

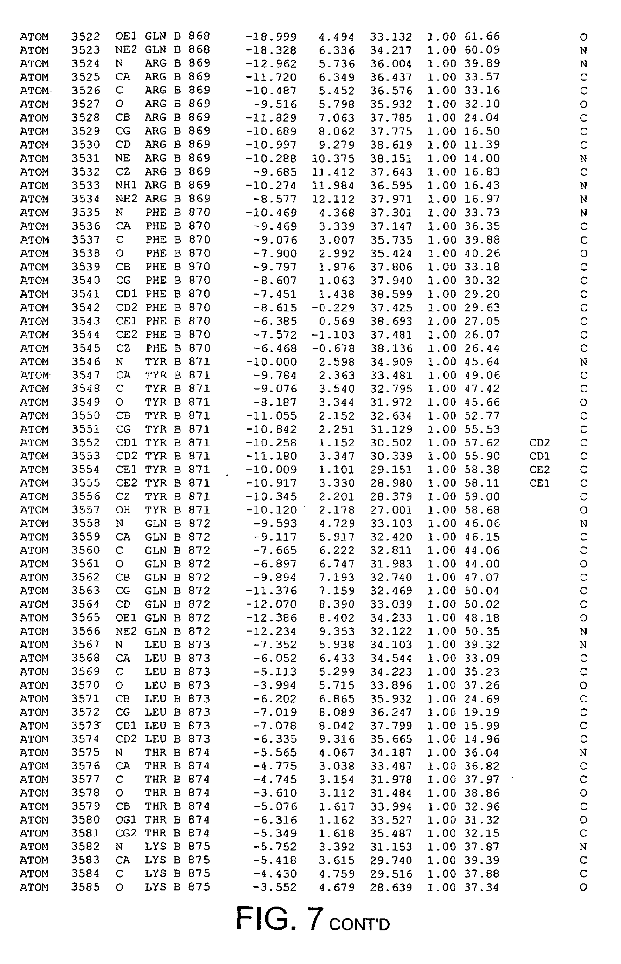

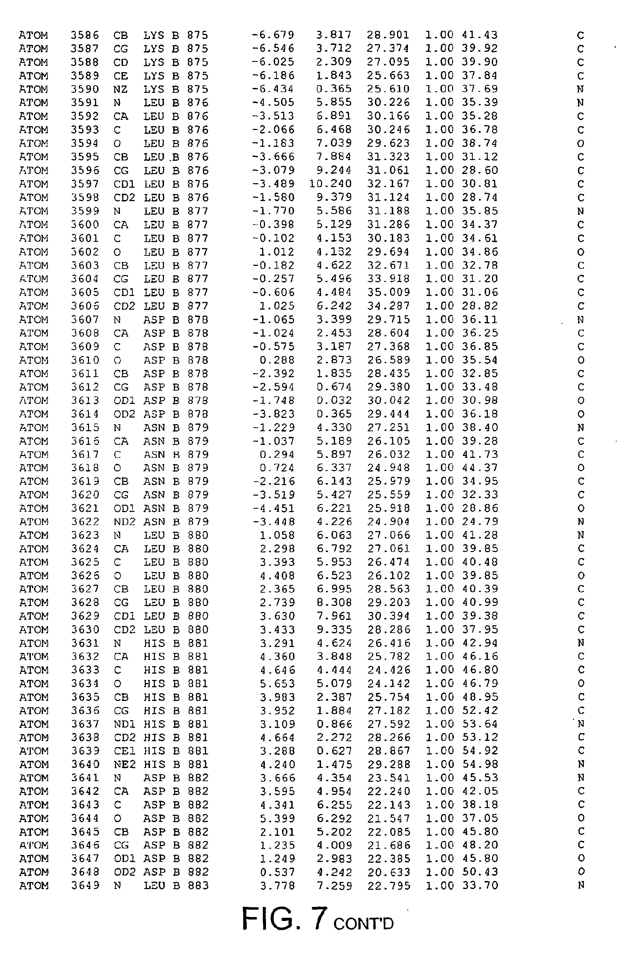

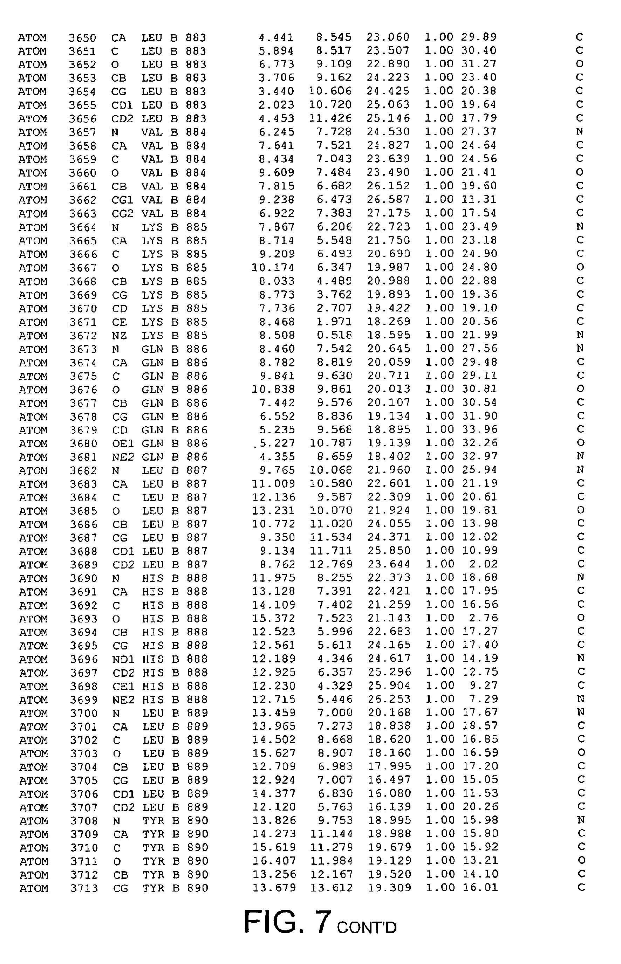

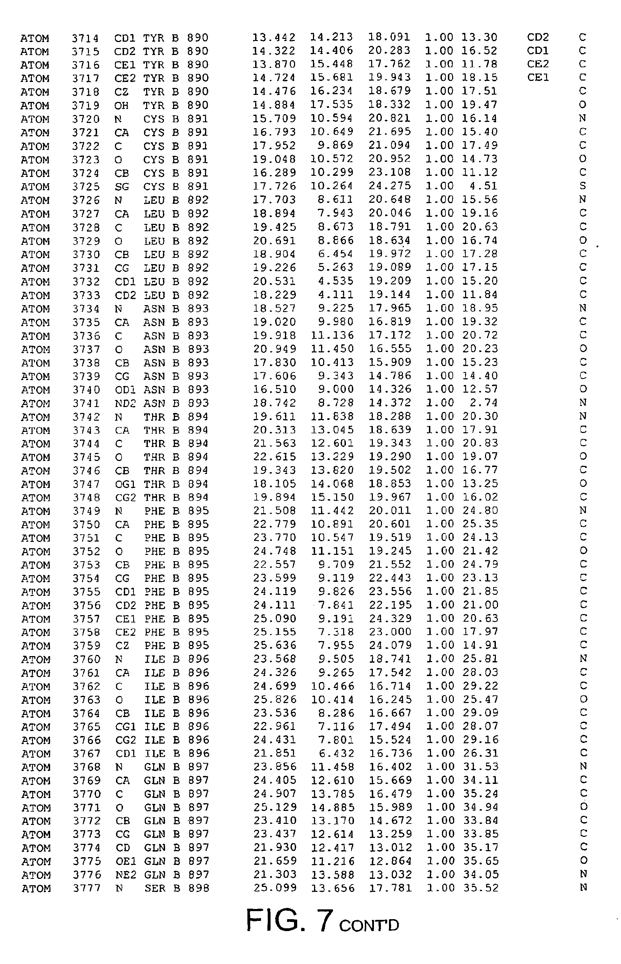

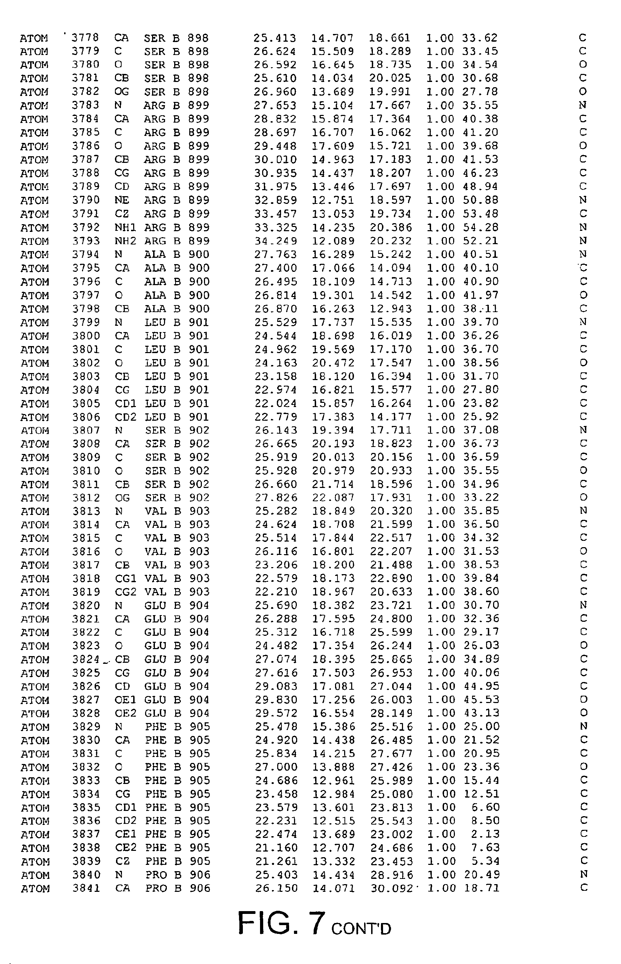

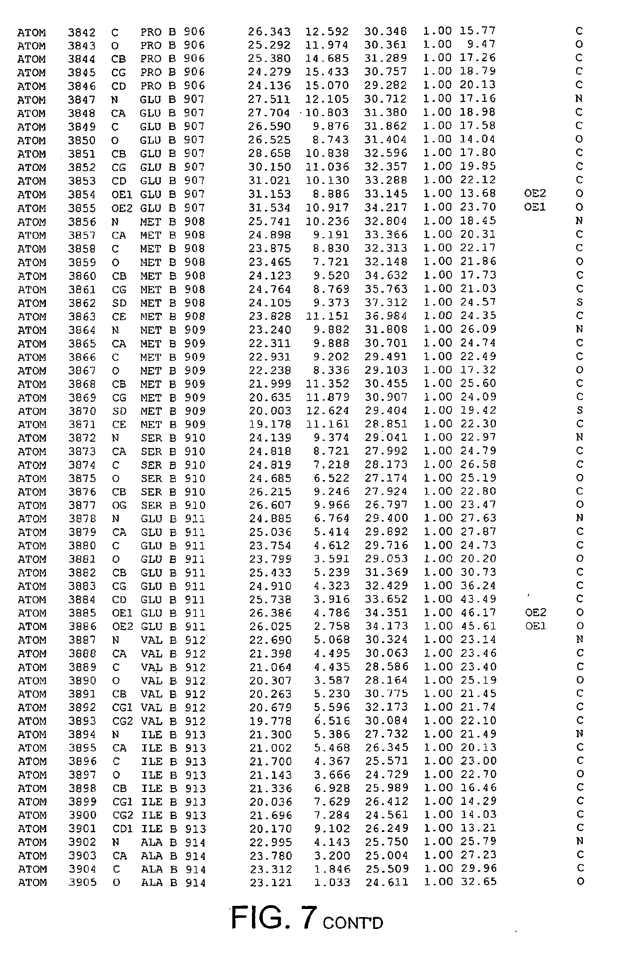

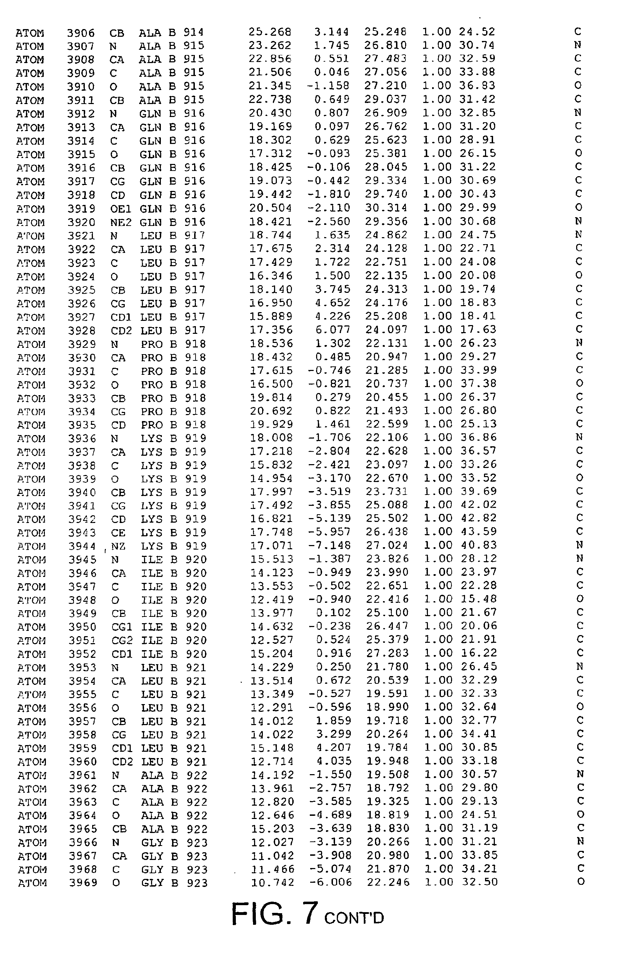

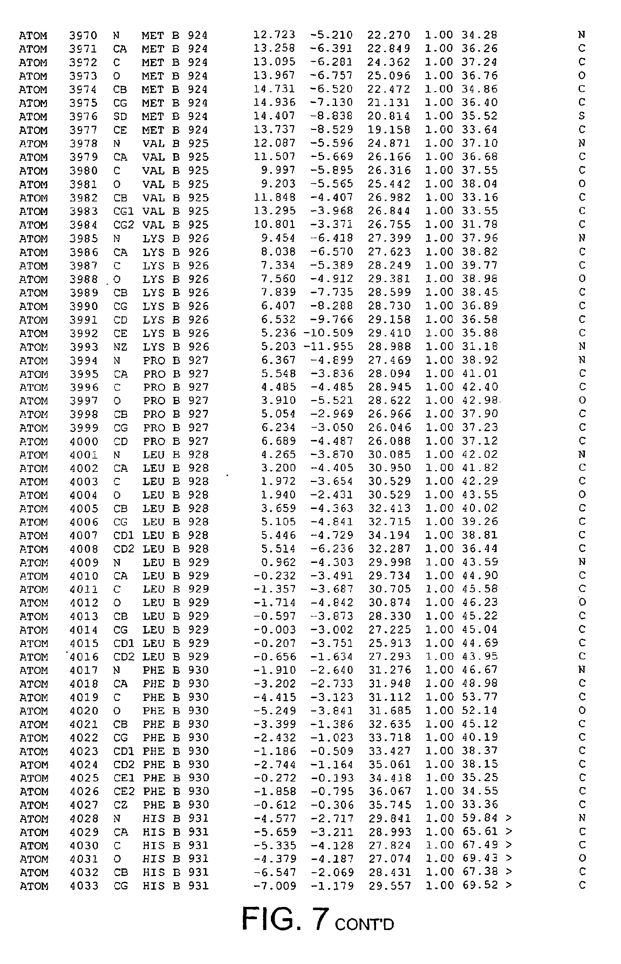

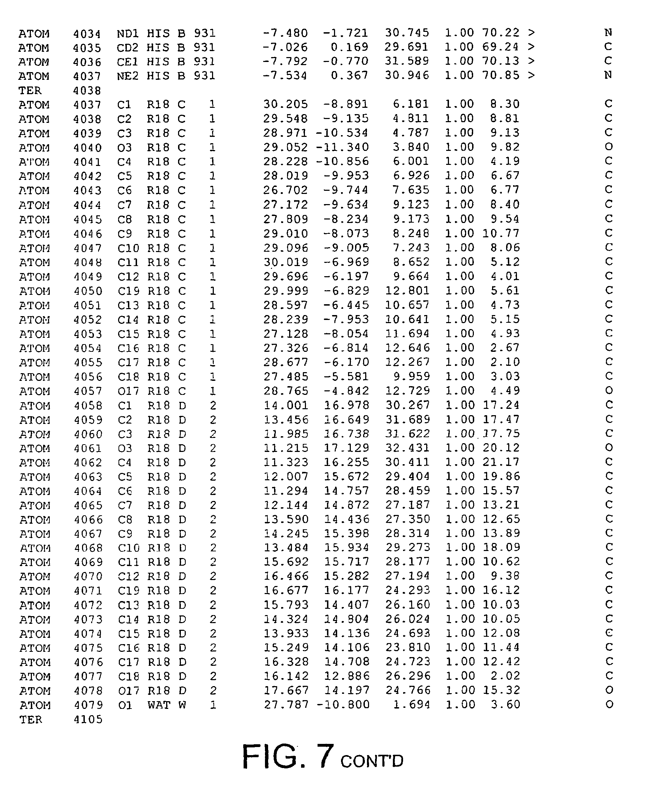

The present invention also provides a crystal model comprising the hPR-LBD which is built from all or part of the crystal co-ordinate data as shown in Table 5 (see Figure 7).

The present invention also covers these novel aspects and their uses. In this respect, the teachings of the AR-LBD (i.e. hAR-LBD) are equally applicable to the novel aspects of the PR-LBD (i.e. hPR-LBD).

Thus, for example, aspects of the present invention concerning PR-LBD relate to;
A crystal structure comprising a PR-LBD.
A crystal structure for PR-LBD.
A crystal PR-LBD having the structural co-ordinates as set forth in Table 5.
Acrystal structure comprising a PR-LBD-ligand complex.
A crystal structure comprising a PR-LBP.
A model of at least part of an PR-LBD made using or comprising or depicting a crystal structure according to any one of the foregoing aspects of the invention. The crystal structure and the model may be provided in the form of a computer readable medium.
A method of screening for a ligand capable of binding an androgen receptor binding domain, comprising the use of a crystal structure or a model of PR-LBD. For example, the method may comprise the step of contacting the PR-LBD with a test compound, and determining if said test compound binds to said ligand binding domain. The method may be an *in vitro* method and/or an *in silico* method and/or an *in vivo* method.

A ligand identified by a screening method of a foregoing aspect of the invention. Preferably the ligand is capable of modulating the activity of a PR-LBD. As mentioned above, ligands which are capable of modulating the activity of PR-LBDs have considerable therapeutic and prophylactic potential.

The use of a ligand according to the foregoing aspect of the invention, in the manufacture of a medicament to treat and/or prevent a disease in a mammalian patient. There is also provided a pharmaceutical composition comprising such a ligand and a method of treating and/or preventing a disease comprising administering the step of administering such a ligand according or pharmaceutical composition to a mammalian patient.

The crystal structures and models described above also provide information about the secondary and tertiary structure of PR-LBDs. This can be used to gleen structural information about other, previously uncharacterised polypeptides. Thus, according to one aspect of the invention there is provided a method of determining the secondary and/or tertiary structures of polypeptides with unknown (or only partially known) structure comprising the step of using such a crystal or model. The polypeptide under investigation is preferably structurally or functionally related to the progesterone receptor ligand binding domain. For example, the polypeptide may show a degree of homology over some or all parts of the primary amino acid sequence. Alternatively, the polypeptide may perform an analogous function or be suspected to show a similar binding mechanism to the PR-LBD.

### EXAMPLES

The invention will now be further described only by way of example in which reference is made to the following Figures:
Figure 1 which shows a sequence listing for hAR-LBD (SEQ ID No 1) and HPR LBD (SEQ ID No 3) amino acid sequences and a secondary structure for hAR-LBD (SEQ ID No 2). SEQ ID No 1 is presented in the second line of Figure 1. SEQ ID No 3 is presented as the first line in Figure 1. SEQ ID No 2 is presented as the third line in Figure 1.
Figure 2 which shows chemical formulae;
Figure 3 which shows three dimensional structures of hAR LBD and hPR LBD complexed with metribolone (R1881);
Figure 4 which shows a stereo diagrams showing interactions between a bound ligand and protein chain in hAR-LBD and hPR-LBD ligand complexes;
Figure 5 which shows a stereo diagram showing the location of hAR-LBP pathogenic mutations;
Figure 6 which presents Table 4, which has the structural co-ordinates for the hAR-LBD; and
Figure 7 which presents Table 5, which has the structural co-ordinates for the hPR-LBD.

In more detail:
Figure 1 shows a comparison between hAR LBD and hPR LBD amino acid sequences. The numbering scheme of AR is according to [Lubahn, 1988]. The sequence alignment was performed with CLUSTALW [Thompson, 1994]. The residue number applies to the residue directly above or below the last digit. Identical residues are outlined in solid black boxes; gray shading denotes the residues not located in the electron density and thus not included in the model. Selected secondary structure elements are from PROCHECK [Laskowski, 1993] according to Kabsch & Sander [Kabsch, 1983]: E, strand in β-sheet; H, α-helix; Amino acids interacting with bound ligands (R1881 or progesterone) are coloured red (van der Waals cutoff distance 4.0 Å). The mutations presently known for AIS in the hAR LBD are marked below the appropriate position of the respective amino acid in the hAR LBD. Abbreviations: x = prostate cancer, p = PAIS/MAIS, c = CAIS, a = PAIS/MAIS and CAIS, b = PAIS/MAIS and prostate cancer, v = CAIS and prostate cancer, w = PAIS/MAIS and CAIS and prostate cancer.

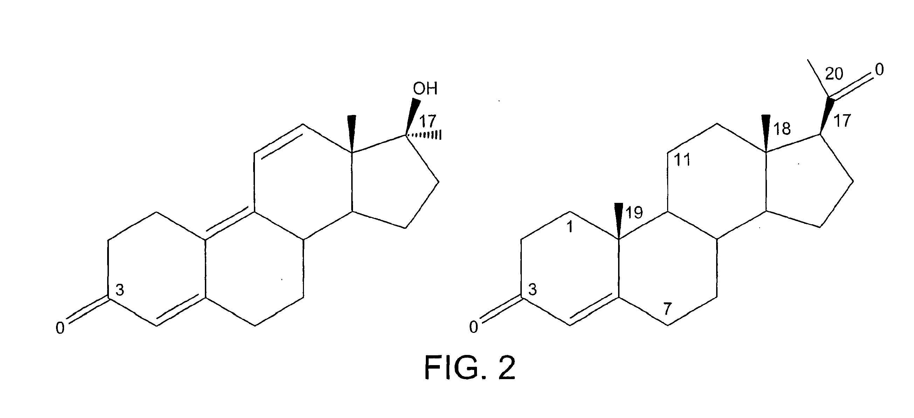

Figure 2 shows the numbering scheme of R1881 (left) and progesterone (right).

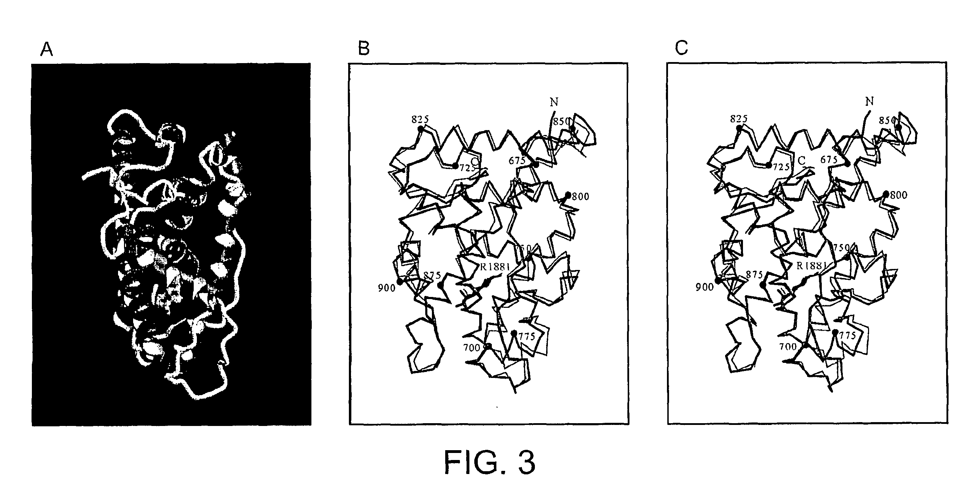

Figure 3 shows the diagrams of the three-dimensional structures of hAR-LBD and hPR-LBD complexed with R1881. (A) MOLSCRIPT/Raster3D [Kraulis, 1991; Merritt, 1994] ribbon diagram of hAR LBD. (B) MOLSCRIPT Stereoview of the C^{α}-trace of the superimposed hAR LBD R1881 (black) and hPR LBD R1881 (red) structures showing the hAR-LBD residue numbering. The (B) view is related to (A) by a clockwise 90° rotation about the vertical axis.
Figure 4 shows the stereo diagrams showing the interactions between the bound ligand and the protein chain in hAR LBD - R1881 (A), hPR LBD - R1881 (molecule B) (B) and hPR LBD - progesterone (C). Residues included are either hydrogen-bonded or have Van der Waals contacts (cutoff distance 4.0 Å) with any of the ligands. Residues V685, Y763 in hAR LBD and corresponding residues I699, Y777 in hPR LBD are hydrogen-bonded to other residues or water molecules near the ligand binding site and are also included. Bound ligand is coloured black, conserved residues are coloured gray, different residues in hAR LBD and hPR LBD are coloured red. Residue labels with an asterisk (*) denote residues that do not have Van der Waals contacts within the specified cutoff distance with the ligand. Hydrogen bond distances for the hPR LBD - progesterone complex were calculated from the PDB deposited coordinates of molecule A. Figures produced with MOLSCRIPT [Kraulis, 1991].

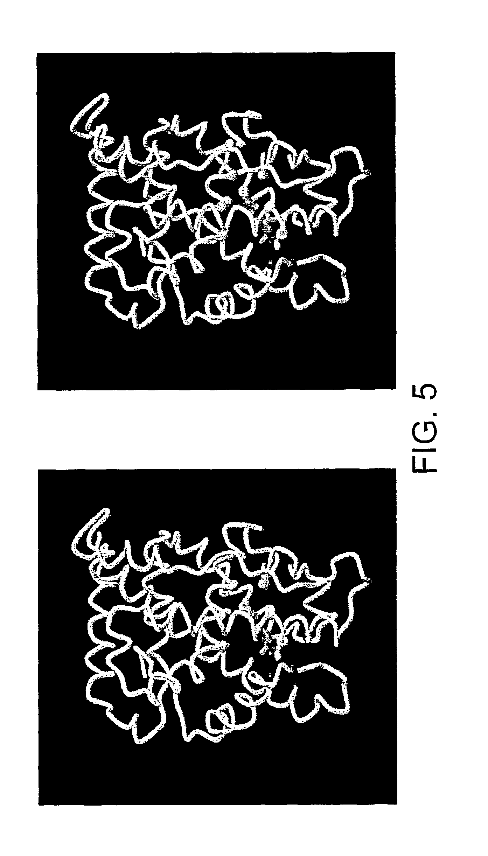

Figure 5 shows the stereo diagram showing the location of the hAR LBP pathogenic mutations: the coloured spheres are represented at the residue's C^{α} position: mutations observed in prostate cancer (PC) are represented in red, those observed for CAIS are shown in yellow and those observed for PAIS/MAIS are drawn in cyan. Mutation of one residue (Met 749) is implicated in both prostate cancer and CAIS and is represented in orange. Figure produced with MOLSCRIPT [Kraulis, 1991] and Raster3D [Merritt, 1994]. The view is rotated by about 80° clockwise about a vertical axis with respect to the orientation shown in Figure 3A.

### Example 1

### Plasmid constructs

The cDNAs coding for the human androgen and progesterone receptors were obtained from the groups of A. Cato (Forschungszentrum Karlsruhe, Germany) and P. Chambon (IGBMC, Strasbourg, France) respectively. The ligand binding domains (LBD) of the androgen receptor (amino acid residues (aa) 663 - 919) and the progesterone receptor (aa 678 - 933) were amplified by the PCR technology using appropriate primers and cloned into a pGEX-KG vector [Hakes, 1991]. The resulting fusion proteins consisted of a glutathion-S-transferase, containing a C-terminal thrombin cleavage site, optimised by a glycine-rich "linker" region followed by the corresponding LBD. The constructs were then transformed into the *E. coli* strain BL21 (DE3).

### Protein expression and purification

Fermentation using the corresponding recombinant *E.Coli* strains expressing hAR LBD was carried out in 2XYT medium in the presence of ampicillin (200 ug/ml) supplemented with 10uM R1881. Expression was induced with 30 µM IPTG (isopropyl-β-D-thiogalactoside) and the fermentation (10 L) was continued at 15°C for 14 - 16 hours. Cells were harvested by centrifugation and disrupted twice in a continuous high pressure homogeniser (9000PSI) in a buffer containing 50 mM Tris/HCl, pH 8, 150 mM NaCl, 5 mM EDTA, 10 % Glycerol, 100 uM R1881, 100 uM PMSF and 10 mM DTT. All buffers were purged with nitrogen before adding DTT. The supernatants from ultracentrifugation were loaded onto a glutathione sepharose column, washed with 50 mM Tris_HCl, pH 8, 150 mM NaCl, 5 mM EDTA, 10 % Glycerol, 10 uM R1881, 0.1% n-octyl-β-glucoside and 1 mM DTT and the fusion protein was eluted using the same buffer supplemented with 15 mM reduced glutathione. The eluate was diluted with 100 mM HEPES pH 7.2, 150 mM NaCl, 0.5 mM EDTA, 10% glycerol, 10 uM R1881, 1mM DTT and 0.1% n-octyl-β-glucoside up to a fused protein concentration of 1 mg/ml. A thrombin cleavage (2 N.I.H. units/mg fusion protein) was performed overnight at 4°C. The protein mixture was further diluted three fold with 10 mM HEPES pH 7.2, 10% glycerol, 10 nM R1881, 10 mM DTT and 0.1% n-octyl-β-glucoside and loaded onto a Fractogel SO₃⁻ column and eluted with a gradient of 50-500 mM NaCl in a 10 mM HEPES buffer pH 7.2, 10% glycerol supplemented with 10 nM R1881, 10 mM DTT and 0.1% n-octyl-β-glucoside. Approximately 2.4 mg of purified hAR LBD can be recovered from 1L *E.Coli* cell cultures. Protein concentration was determined with Bio-rad Protein Assay. Fermentation and purification of the hPR LBD was performed identically but a HEPES buffer pH 7.3 was used from the beginning.

### Results 1

### Protein expression and purification

Glutathion-S-transferase fusion proteins can be expressed to very high levels in the *E. coli* strain BL 21 (DE3) [Hakes, 1991]. We and others have used this system successfully for the production of the ligand binding domains of the human progesterone [Williams, 1998] and androgen receptors. An optimal and stable expression of soluble fusion proteins strongly depends on the presence of ligand in the cells during fermentation (data not shown). During cell disruption, purification and concentration any protein oxidation was avoided. Therefore all buffers were purged carefully with nitrogen and DTT was used as an antioxidant. Fusion proteins were purified by the use of Glutathion sepharose and subsequently cleaved with thrombin. Ligand binding domains were separated from the cleavage products and thrombin by cation exchange chromatography. Concentration was performed with the aid of a nitrogen pressure diafiltration system and the concentrate was immediately used for crystallisation experiments.

### Example 2

### Crystallisation and data collection

Both proteins were dialysed after purification with buffer containing 50mM HEPES pH 7.2 for hAR LBD, or 10mM HEPES pH 7.2 for hPR LBD, respectively, 10% glycerol, 10mM DTT, 0.1% n-octyl-β-glucoside, 10mM R1881 and 150mM Li₂SO₄ and were concentrated up to 3 mg/ml for the hPR LBD - R1881 and up to 4.4 mg/ml for the hAR LBD - R1881 respectively. Both proteins were crystallised using the vapour diffusion method at 20°C for the hAR LBD complex and at 4°C for hPR LBD complex respectively. Due to the instability and continuous precipitation of both proteins, crystallisation experiments had to be set up immediately after concentration. For the hAR LBD - R1881 complex, the reservoir solution contained 0.4M Na₂HPO₄·2(H₂O), 0.4M K₂HPO₄, 0.1M TRIS-HCl pH 8.5, 0.1M (NH₄)₂HPO₄ and 5% PEG200. Drops were composed of equal volumes of protein and reservoir solution and were set up using the sitting drop method. Within two days crystals appeared and grew to typical dimensions of 50x50x80 µm³ surrounded of precipitate. Crystals were flash frozen using a cryoprotecting solution of 60% PEG 400 in 0.1M TRIS-HCl pH 8.5. Data was collected from one crystal at the ESRF (Grenoble, France) at beamline ID14-EH4 to a resolution of 2.4 Å. For the hPR LBD - R1881 complex, the reservoir solution contained 10% iso-propanol and 100mM sodium citrate in 50mM HEPES pH 7.5. The drops were set up using the hanging drop method and were composed of a 2:1 ratio of protein and reservoir solution. First crystals appeared after five weeks and grew to a size of approximately 160x120x40 µm³. One crystal was flash frozen using a cryo-protecting solution containing 30% glycerol. Data were collected at beamline BM14 at the ESRF (Grenoble, France) to a resolution of 2.8 Å. Before data collection was complete the crystal decomposed in the X-ray beam.

Both data sets were integrated and reduced using DENZO and SCALEPACK [Otwinowski, 1997]. Statistics of X-ray data collection and processing are summarised in **Table 1**

**Table 1. Summary of data collection, processing and scaling**

| | hAR LBD - R18811 | hPR LBD - R1881 |
|---|---|---|
| Space group | P2₁2₁2₁ | P2₁ |
| Unit cell | a=54.28 b=66.14 c=71.72 Å | a=58.40 b=65.01 c=71.18 Å β=95.7° |
| Wavelength (Å) | 0.9324 | 0.9537 |
| Resolution range (Å) | 24.4-2.40 | 12.47-2.80 |
| N _{observations} | 37,443 | 67,655 |
| N _{reflections} | 10,638 | 8,875 |
| % Completeness * | 99.8 (99.9) | 67.0 (68.8) |
| Redundancy | 3.5 | 7.6 |
| R_{merge}* | 0.078 (0.351) | 0.048 (0.151) |
| I/σ(I) | 12.0 | 15.2 |
| Estimated Bₒᵥₑᵣₐₗₗ | 49.4 | 48.2 |

| | | |
|---|---|---|
| ● Values in parentheses refer to the last resolution shell, 2.46 ≥ d ≥ 2.40 Å for hAR LBD - R1881 complex and 2.87 ≥ d ≥ 2.80 Å for hPR LBD - R1881 complex. | | |

### Structure determination

Contrary to the hPR LBD - progesterone complex which crystallises with one homodimer in the monoclinic space group P2₁ the hAR LBD crystallises with one monomer in the orthorhombic spacegroup P2₁2₁2₁. Therefore the structure determination for the hAR LBD - R1881 complex was carried out using the molecular replacement method in AMoRe [Navaza, 1994] with the coordinates of only the monomer A of the hPR LBD dimer (PDB entry: 1A28, [Williams, 1998 ]) without the progesterone ligand. The hPR LBD - R1881 complex crystallises in the same monoclinic space group P2₁ and with similar cell constants as the hPR LBD - progesterone complex and thus the whole dimer without the ligand was used as a search model in AMoRe. Clear solutions were obtained for both structures using data between 15.0 and 3.5 Å for the hAR LBD and 12.0 and 3.5 Å for the hPR LBD, respectively.

### Refinement of hAR LBD - R1881 complex

The molecular replacement solution obtained was refined using X-PLOR [Brünger, 1992]. In all refinements and map calculations with X-PLOR a bulk solvent correction was used and all low resolution data was included. Prior to the refinement calculations, a random 5% sample of the reflection data was flagged for R-free calculations [Brünger, 1992]. All model interactive visualisation and editing was carried out using TURBO [Roussel, 1990]. Refinement started using data up to 3.5 Å and resolution was gradually extended to 2.4 Å. The model was edited according to the known hAR LBD sequence [Lubahn, 1988] using 2|Fₒ|-|F_{c}| and |Fₒ|-|F_{c}| maps calculated at 3.2 Å resolution and simulated annealed omit maps. The fast wARP [Lamzin, 1997 ; Perrakis, 1997] molecular replacement protocol was also applied after each XPLOR refinement to further improve the 2|Fₒ|-|F_{c}| electron density map. Prior to its inclusion in the model, the electron density for the R1881 ligand was clearly visible in all maps. A model for the ligand was obtained from the Cambridge Structural Database entry HMESTR [Precigoux, 1981; Allen, 1979 ]. The XPLOR topology and parameter dictionaries were built using program XPLO2D [Kleywegt, 1995]. In the final refinement at 2.4 Å, 26 water molecules were included in the model, and individual restrained B-factors were refined for all non-hydrogen atoms. The final values of R and R-free were 21.0 % and 29.7 %, respectively. The R-free/R ratio is only slightly smaller than expected [Tickle, 1998] for the number of atoms and reflections used in the refinement. The refinement results and statistics are shown in Table 2.

**Table 2. Final refinement statistics for hAR LBD and hPR LBD complexed with R1881**

| R1881 in complex with | hAR LBD | hPR LBD |
|---|---|---|
| Final R-factor (%) | 21.0 | 21.7 |
| Final R-free (%) | 29.7 | 34.3 |
| Number of non-hydrogen protein atoms | 2044 | 4027 |
| non-hydrogen protein atoms missing | 22 | 32 |
| non-hydrogen ligand atoms | 21 | 42 |
| solvent molecules | 26 | 1 |
| Estimated overall r.m.s. coordinate error (A) * | 0.47 | 0.53 |
| Model r.m.s. deviations from ideality: | | |
| Bond distances (Å) / Bond angles (°) | 0.01 / 1.7 | 0.02 / 4.4 |
| Average B values (Å²): | | |
| Main-chain / Side-chain | 48.3 / 52.1 | 33.2 / 28.7 |
| Ligand / Solvent | 45.2 / 49.2 | 10.2 / 3.6 |

| | | |
|---|---|---|
| * calculated with SIGMAA [Centre, 1999 ; Read, 1986]. | | |

### Refinement of hPR LBD - R1881 complex

The molecular replacement solution obtained was refined using REFMAC [Murshudov, 1997] using the maximum-likelihood approach. Bulk solvent scaling of Fo and Fc was applied based on Tronrud's solvent correction and all available data with no sigma cut-offs were used. All map calculations were done including calculated F-values for missing reflections. To avoid model bias, calculated maps using only Fo were checked. After the first refinement step the sigmaA-weighted calculated 2|Fₒ|-|F_{c}| and |Fₒ|-|F_{c}| maps were inspected using the program O [Jones, 1991 ] and electron density of the ligand was clearly observed. The ligand was build up in SYBYL6.5 (Tripos Inc., 1998) and was included in further refinement steps. A dictionary file for distance restraints for the R1881 molecule was prepared using MAKEDICT [Collaborative Computational Project Number 4, 1994]. The model was furthermore refined with alternating cycles of interactive model building and iterative refinement steps. Towards the end of the refinement, only one water molecule in the LBP was added. Although some more possible water sites were located in the electron density we decided not to include them in the model due to the low resolution and missing data. The final model comprises 4027 protein atoms, 42 ligand atoms and 1 water molecule with final R values of R = 21.7 % and R-free = 34.3 %, respectively. A summary of the refinement and model statistics is included in Table 2.

### Results 2

### Structure analysis and comparison of the hAR LBD - R1881 and the hPR LBD - R1881 complexes

Both crystal structures were analysed with PROCHECK [Laskowski, 1993] and their stereochemical quality parameters were within their respective confidence intervals. In the Ramachandran ϕ,φ plot for the non-proline and non-glycine residues (not shown) 87.7% for the hAR LBD - R1881 and 85% for the hPR LBD - R1881 structures respectively lie within the most favoured regions. For the hAR LBD - R1881 complex no residue is outside the normally allowed regions whereas in the hPR LBD - R1881 complex two residues are located in disallowed regions (Asn 705 and Ser 793 in molecule A) and three residues (Thr 796 in molecule A, Asn 705 and Ser 793 in molecule B) are located in generously allowed regions. These residues are not involved in ligand binding and are located in loop regions which are most probably not involved in ligand recognition. In the hAR LBD - R1881 structure there is only one close contact (2.6 Å) between Met895 and Ala896 carbonyl oxygens. In the hPR LBD - R1881 structure some close contacts were observed but due to the resolution and completeness of the data this is not surprising. The overall fold of the hAR and hPR LBD - R1881 structures is very similar, and also with that of hPR LBD complexed with progesterone [Williams, 1998]. On the basis of the secondary structure calculated with PROCHECK [Laskowski, 1993] according to Kabsch & Sander [Kabsch, 1983], the hAR LBD - R1881 structure contains 9 α-helices, two 3₁₀ helices and four short β-strands associated in two anti-parallel β-sheets. The helices are arranged in the typical 'helical sandwich' pattern as in hPR LBD - progesterone complex [Williams, 1998] and helices H4, H5 and H10, H11 are contiguous. There are a few minor variations in secondary structure between hAR LBD - R1881 and hPR LBD - progesterone but probably the most interesting is that in hAR LBD - R1881 helix H12 seems to be split into two shorter helical segments, with nine and five residues each respectively. This observation was not seen in the hPR LBD - R1881 structure, although a bending of helix H12 is also seen here. Figure 1 shows a comparison between the amino acid sequences of hAR LBD and hPR LBD. A ribbon diagram of the hAR LBD - R1881 structure is shown in Figure 3 along with a superimposed C^{α}-trace of the hAR LBD - R1881 and hPR LBD - R1881 molecules. The crystal structure coordinates of hAR LBD - R1881 were superimposed with those of hPR LBD - R1881 (molecule B) and hPR LBD - progesterone (molecule A) using LSQKAB [Kabsch, 1976]. For the superposition the main chain atoms except three N-terminal (Cys 669-Pro 671) and one C-terminal (Thr 918) residues were used. The r.m.s. coordinate deviations were 1.16 and 1.22 Å respectively, again an indication of the similarity of the overall fold of these three molecules. In hAR LBD - R1881, Cys 669 and Cys 844 are very close and a disulphide bridge between them was modelled, based on the electron density. However there is no supporting biochemical evidence so far and it should be noted that the temperature factors of both cysteine residues and the adjacent residues are very high. A *cis* peptide bond is found at position Pro 849 in hAR LBD - R1881.

### Example 3

### Comparative modeling

A model of the hAR LBD was built based on the coordinates of the hPR LBD - progesterone complex (molecule A) [Williams, 1998]. Amino acid substitutions were made based on the sequence alignment in Figure 1 using the Insight 98.0 software (MSI Inc., San Diego, CA USA 1998). Water molecules as observed in the hPR LBD crystal structure (molecule A) were included in the calculations.

Soaking of the initial model and the energy minimisation protocols applied are described in detail elsewhere [Letz, 1999].

### Results 3

### Comparison of model and crystal hAR LBD structure

The model and the crystal structure of the hAR LBD are very similar with respect to their overall structure, the ligand binding pocket (LBP) and the ligand orientation. The root-mean-square (r.m.s.) deviation between 149 equivalent C^{α} atoms in helices between the model and crystal structure of the hAR LBD is 1.09 Å. It is comparable to the r.m.s. deviation of 0.84 Å and 0.85 Å between the crystal structures of the hAR LBD and the hPR LBD - progesterone complex, on which the model of the hAR LBD was based on and the hAR LBD model and the hPR LBD - progesterone crytsal structure, respectively. The most striking difference between the model and the crystal structure was found in the region of helix H6. In the hAR LBD crystal structure, this region was identified as an α-helix (calculated with the Kabsch & Sander algorithm [Kabsch, 1983] as implemented in Insight98.0 (MSI Inc., San Diego USA, 1998), whereas in the hPR LBD - progesterone complex (molecule A) no α-helix is observed. There is also no α-helix in the hAR LBD model in this area. The ligand orientation in both the hAR LBD - R1881 model and crystal structure is very similar. The same hydrogen bonds are found between the 03 of R1881 and Arg 752 with a distance of 3.0 Å in the crystal and 3.4 Å in the model structure, respectively. In the ligand D-ring, 017 is within hydrogen bond distance to Asn 705 and Thr 877, 3.1 and 3.0 Å in the crystal structure, 2.6 and 3.3 Å in the model structure, respectively.

### Discussion

### Comparative modelling

The model of the hAR LBD which is based on the hPR LBD - progesterone complex is very similar to the hAR LBD crystal structure with respect to the overall fold and ligand orientation. The most striking differences were a stronger bending of helices H10 and H11 in the model compared to the crystal structure of the hAR LBD. We modelled helix H9 with the same length as in the crystal structure of the hPR LBD - progesterone complex. In the hAR LBD crystal structure it is one helical turn shorter. This region is far away from the LBP and therefore has no influence on the size of the LBP. Our model structure differs from other published models [Yong, 1998] with respect to the secondary structure alignment, as the authors based their model on the crystal structure of the RARα LBD [Bourguet, 1995]. The secondary structure assignment by Yong *et al*. as compared to the hAR LBD crystal structure is similar between helices H3 and H10, the assignment differs most for helices H11, H12 and the additional helix at the C-terminal end.

### Ligand binding pocket(LBP) interactions

There are a total of 18 amino acid residues in hAR LBD and hPR LBD that interact with the bound ligand (either R1881 or progesterone). These residues are highlighted in Figure 1 and included in Figure 4. Most of these residues are hydrophobic and interact mainly with the steroid scaffold, while a few are polar and may form hydrogen bonds to the polar atoms in the ligand. The hydrogen-bonding scheme to 03 of R1881 and progesterone is similar but not identical, as shown in Figure 4. In the hAR LBD - R1881 crystal structure, this oxygen atom forms a hydrogen bond to Arg 752 (Arg 766 in hPR LBD), but in contrast with the hPR LBD - progesterone complex the distance of 3.9 Å to Gln 711 (Gln 725 in hPR LBD) does not allow a hydrogen bond. There is a water molecule near 03 that is hydrogen-bonded to three other residues with a nearly triangular geometry (R752 N^{η1}, M745 O and Q711 O^{ε1} in hAR LBD; R766 N^{η1}, M759 O and Q725 O^{ε1} in hPR LBD - progesterone). Two of these residues are acceptors, therefore a third acceptor atom (03 in either progesterone or R1881) in a direction perpendicular to the plane of the triangle is unlikely, also due to unfavourable geometry. The water molecule hydrogen-bonded to Q711 N^{ε2} in hAR LBD (Q725 in hPR LBD) has hydrogen bonds to two other residues (V685 O and F764 O in hAR LBD, I699 O and F777 O in hPR LBD) and in hAR LBD it is hydrogen bonded to a further water molecule, the overall hydrogen bond geometry being nearly tetrahedral. In the hPR LBD - R1881 structure, the ligands in molecules A and B possess slightly different hydrogen bond patterns. In molecule A, 03 of R1881 forms two hydrogen bonds (3.2 Å to Gln 725 N^{ε2} and 2.9 Å to Arg 766 N^{η2}). One water molecule was located in the Fₒ-F_{c} electron density with the same tetrahedral geometry as observed in the hAR LBD - R1881 structure. In molecule B, the ligand is in a slightly different position and the hydrogen bond pattern differs from that observed in molecule A. The 03 of R1881 forms again one hydrogen bond to Arg 766 N^{η2} with a distance of 2.9 Å whereas the distance to Gln725 N^{ε2} is now 3.7 Å, outside the acceptable range for a hydrogen bond.

The 17 β hydroxyl group of R1881 forms different hydrogen bonds, when bound to hAR LBD or hPR LBD (Figure 4). In hAR LBD, the 17 β hydroxyl group is hydrogen-bonded to Asn 705 (2.8 Å) and Thr 877 (2.9 A). The same pattern is observed in molecule B of hPR LBD - R1881 complex where the 17β hydroxyl group of R1881 also forms strong interaction to Asn 719 (2.8 Å), whereas in molecule A the corresponding distance of 3.5 Å is only in the range of a weak interaction. In contrast to the hAR LBD, in both hPR LBD monomers Cys 891 (Thr877 in hAR LBD) shows only a weak interaction with the 17β hydroxyl group of R1881 (3.7 Å in molecule A and of 4.0 Å in molecule B, respectively). However, the relative orientation of the Cys 891 side chain with regard to the hydroxyl group does suggest that this interaction is relevant to the binding of the ligand.

### Structural basis for ligand specificity in hAR LBD

The ligand R1881 binds with a relative binding affinity (RBA) of 290 to the wild-type hAR as compared to a value of 180 for DHT and 100 for testosterone, respectively [Teutsch, 1994]. As for the wild-type hPR, the relative binding affinity of R1881 is 190 with respect to progesterone (RBA = 100). Overall, R1881 shows comparable good binding affinities to both receptors, which is also reflected in the orientation of the ligand in the LBPs of the hAR LBD and the hPR LBD (Figure 4). Thr 894 in hPR LBD is replaced by Leu 880 in hAR LBD and the C^{δ2} atom of this leucine makes a van der Waals contact (3.9 Å) with the oxygen atom of the 17β hydroxy group of R1881. This bulkier side chain, along with the substitution of Cys 891 in hPR LBD by Thr 877 in hAR LBD is very likely responsible for the specific recognition of the 17β hydroxyl group of R1881 contrary to the 17β acetyl group of progesterone. Not only there is an extra polar residue (Thr 877 besides Asn 705 which is conserved in AR) which can form an additional hydrogen bond to the 17β hydroxyl oxygen, but the directed decrease in pocket volume caused by the change of Thr894 to Leu880 will very likely inhibit the binding of other bulkier ligands such as progesterone. As previously noted [Williams, 1998] there are no strong hydrogen-bonded interactions between the O20 carbonyl oxygen atom of progesterone and the protein in hPR LBD indicating that the recognition of this group is probably made only through hydrophobic and steric interactions. The hPR LBD can bind R1881 as well as progesterone and, as seen from the above discussion of the hydrogen bonding and van der Waals interaction pattern between protein chain and ligand in the crystal structure, the hPR LBD molecule appears to exhibit two different binding modes for R1881, one resembling that of progesterone (03 with two hydrogen bonds to the protein chain and the 17β function weakly interacting with the protein chain) and one similar to that of hAR LBD (03 with only one hydrogen bond to the protein chain and the 17β function also hydrogen bonded to the protein chain). However, these binding modes do not seem to imply significant changes in ligand position and orientation within the LBP.

### Mutations

We analysed whether the mutated amino acid residues are predominantly found in the interior of the protein or at the surface. Comparison of the solvent accessibility of these residues revealed that a nearly even distribution is found between buried, medium or fully accessible residues. Table 3 lists all those mutations in or near the AR ligand binding pocket (LBP) which are known to be involved in AIS and prostate cancer (PC), their location with respect to secondary structural elements as well as the potential effect of the mutations.

**Table 3: hAR LBD mutations observed in prostate cancer, CAIS and PAIS/MAIS. For convenience, the equivalent positions of the amino acid residues (aar) in the hPR LBD are given. Bold numbers indicate available mutant data in the PR. All mutations are taken from the androgen receptor gene mutations data base (Gottlieb et al. 1998 and references therein)**

| | Mutation in AR | aar in PR | Location in LBD | Vicinity of ligand | Comment |
|---|---|---|---|---|---|
| prostate cancer | Leu701-His | 715 | H3 | D | His: too close contacts to Phe876, hydrophobic environment for His: Met780, Phe876; Leu880 |
| | Met749-Ile | 763 | H5 | A | Ile either too close to Arg752 or Phe764 |
| | Thr877-Ala | 891 | H11 | D | No H-bond partner for ligand 17 β OH |
| | Thr877-Ser | 891 | H11 | D | 2 energetically favourable conformations for Ser similar to the O^{γ} or C^{γ} position of Thr |
| | Leu880-Gln | 894 | H11 | D | Hydrophobic environment for Gln: Leu 701, Met 780, Phe 876 |
| | Phe891-Leu | 905 | Loop H11/H12 | D | Leu side chain too close to Leu881 in the 2 most often observed side chain conformations for Leu |
| CAIS | Asn705-Ser | 719 | H3 | D | Ser: too small for H-bond partner to ligand 17 β OH |
| | Leu707-Arg | 721 | H3 | A | Arg: too elongated for this area |
| | Met749-Val | 763 | H5 | A | Val: branched aar, C^{γ} too close to ligand |
| PAIS/MAI S | Gly708-Ala | 722 | H3 | C | No hindrance for Ala |
| | Gly708-Val | 722 | H3 | C | Val: too close to Trp 741, Met 895, ligand |
| | Met742-Val | 756 | H5 | B/C | Val fits into LBP but environment is less tightly packed, the LBP is enlarged |
| | Met742-Ile | 756 | H5 | B/C | Ile fits into LBP but environment is less tightly packed, LBP is enlarged |
| | Met745-Thr | 759 | H5 | A | Val too close to ligand |
| | Val746-Met | 760 | H5 | B | Met too close to Met 741, Leu 873, ligand |
| | Arg752-Gln | 766 | H5 | A | Gln too small for H-bond partner to ligand 03 |
| | Phe764- Ser | 778 | S1 | A | Ser: no stacking with A-Ring of ligand possible |
| | Met787-Val | 801 | H7 | B | No hindrance for Val, but fewer contacts to Val 746, Leu 873 and ligand |

Mutations are reported for 12 of the 18 residues considered to interact with the ligand R1881 within 4.0 Å as discussed above, as well as two additional residues within 5.0 Å of the ligand (G708 and V746 in hAR LBD, G722 and Val760 in hPR LBD). In some cases the same amino acid can be mutated into different residues, e.g. T877A and T877S. For most of these mutations, a structural effect can be associated with the substitution. For example, when Met 749 in hAR LBD is substituted by the branched amino acid valine, the C^{γ} side chain atoms would become too close to the ligand. The location of these mutations in the three-dimensional structure of hAR LBD - R1881 is shown in Figure 5, and it can be seen that the mutations involved in the prostate cancer (PC) cluster mainly near the R1881 17β hydroxyl group while those involved in AIS are arranged mainly around the other parts of the ligand. One notable exception is Met 749 which has mutations implicated in both PC and CAIS and is located in the vicinity of R1881 03, opposite from the other PC-implicated mutations.

### Mutations in the LBP observed in the prostate cancer cell line LNCaP

The prostate tumor cell line LNCaP contains an AR receptor showing a significant increased binding affinity for gestagenic and estrogenic steroids but shows identical R1881 binding (Veldscholte *et al.* 1990). A single point mutation (T877A) is associated with this abnormal behaviour. With an alanine at this position an important hydrogen bond partner for the 17β hydroxyl group in R1881, testosterone or dihydrotestosterone (DHT) would be missing, but the other hydrogen bond partner, Asn 705, involved in ligand binding could still orient the ligand in the LBP. Mutagenesis experiments of hPR emphasised the critical role of this asparagine residue in ligand interaction (Letz *et al.* 1999). In the crystal structure of the hPR LBD - progesterone complex, Cys 891 is found at the position of Thr 877, but no hydrogen bond of the 17β acetyl group of progesterone was observed although Cys 891 is relatively close (4.3 Å in molecule A, 4.4 Å in molecule B) to 020 of progesterone. However, bacterial extracts of a mutated hPR LBD (C891S or C891V) showed a large decrease in relative binding affinity for progesterone and the purified mutated hPR LBD was completely inactive in binding assays [Letz, 1999].

### Mutations in the LBP observed in CAIS

The three mutations in the hAR LBP described for CAIS are substitutions that considerably change the size of the respective amino acid side chains, N705S [Bellis, 1992; Pinsky, 1992], L707R [Lumbroso, 1996] and M749V [Bellis, 1992; Jakubicza, 1992]. This change in size alters the LBP such that the local structure and interactions to the ligand are disturbed.

In the AR LBD and PR LBD crystal structures, Asn 705 or Asn 719 respectively is one of the hydrogen bond partners to the ligand R1881, but not to progesterone. If this residue is substituted to Val in hPR LBD, only a moderate effect was observed on the binding activity of progesterone, considering the K_{D} and half-life values [Letz, 1999]. In the crystal structure of the hPR LBD - progesterone complex, Asn 719 is involved in the stabilisation of the loop between H11 and H12, via hydrogen bond between Asn 719 N^{δ2} and Glu 904 O. In the hAR LBD, an identical stabilisation is found, by means of a hydrogen bond between Asn 705 N^{δ2} and Asp 890 O. A N705S mutation, observed in a patient suffering from CAIS would have a two-fold effect, destablilization of the structure and loss of a hydrogen bond partner for the ligand.

In the described hAR mutant L707R, the structure integrity disturbance is also reflected in the binding constants. Considering a van der Waals cutoff distance of 4.0 Å, the side chain of Leu 707 makes close contacts with the A-ring of R1881 as well as five residues in the protein chain: V685, A687, Q711, F764 and L768. The first two residues are located in a loop region between H2 and H3, the third is located within H3 and is involved in the hydrogen bonding pattern of a water molecule near the 03 atom of R1881, and the final two belong to each of the two strands S1 and S2 of the first short β-sheet. Clearly, such a variation in the size of the side-chain would have a large impact, not only in the LBP but in disrupting the overall protein fold itself. The mutated receptor shows undetectable binding affinity to the ligand R1881 as obtained by Scatchard plot analysis and no transcriptional activity is found [Lumbroso, 1996].

### Mutations in the LBP observed with PAIS/MAIS

Seven described mutations in the hAR LBP are associated with PAIS/ MAIS, and multiple substitutions were observed for amino acids at position 708 [Albers, 1997] and 742 [Bevan, 1996 ]. In the hAR LBD crystal structure, a substitution of Gly 708 to alanine should be tolerated whereas a valine at this position would interfere with ligand binding. The closest distance of the C atom of an alanine residue to the ligand would be 3.0 Å, however, the Cg atoms of a valine would be too close to the ligand atoms (1.5 Å). The substitution of the equivalent Gly 722 in the hPR receptor to serine does not influence the binding of agonists, but rather that of the antagonist RU486 [Benhamou, 1992].

In all steroid receptors, the steroid is stabilised by a hydrogen bond between the A-ring of the ligand and an arginine (Arg 752 in hAR). A smaller amino acid residue at this position (mutation to glutamine in hAR) should have a dramatic impact on ligand binding as the stabilisation of the A-ring would be severely hampered due to the lack of a electrostatic interaction (Cabral *et al.* 1998, Komori, 1998). A similar effect has been reported for the hPR receptor where a mutation (R766H) resulted in a low or even non-detectable binding affinity. The side-chain of histidine is too small to serve as a hydrogen bond partner to the 03 atom in progesterone [Letz, 1999].

In the hAR mutation F764S, R1881 shows a similar binding affinity as the wild type receptor, but a rapid ligand dissociation is observed [Marcelli, 1994]. In the crystal structure, Phe 764 is involved in the stabilisation of the A-ring position. A smaller amino acid like serine would allow binding of the ligand, but very likely not contribute to the tight binding of R1881.

Mutations M742V or M7421 both dramatically reduce the binding affinity of R1881 [Bevan, 1996]. Although Ile and Val fit into the LBP, the changed environment is less tightly packed and the LBP is enlarged, thus affecting the binding of the ligand.

However, not all mutations can be related to a disturbance of the structure. In case of the M787V mutation in the hAR LBD, it was found by Scatchard analysis that R1881 and DHT binding was undetectable or strongly reduced [Nakao, 1992]. The lack of androgen binding was thought to be the cause for AIS. In the crystal structure, a methionine to valine substitution could be tolerated. The lack of binding affinity found for R1881 may account for a destabilisation in the LBP as the Met 787 side chain is in van der Waals contact with other amino acids like Val 760 and Leu 887 as well as ligand atoms.

### Example 4

### Modified method for isolating hPR-LBD

### Purification of hPR LBD with R1881:

The pGEX-KG-hPR LBD construct rather than the pGEX-KG-hAR LBD construct was used for fermentation. As a result, compared to "normal" hPR LBD purification, there were a few differences at the beginning of the purification procedure. These differences were related to the size of the construct and to different pH values, salt and additive concentrations:
- Construct: "normal" hPR LBD purification: pGEX-2T-hPR LBD construct (Gly-hPR LBD 677-933), this time: pGEX-KG-hPR LBD: (GSPGISGGGGGI-hPR LBD 678-933) (N-terminal end expended by 10 residues).
- pH: Reduction from pH 8.0 to pH 7.3 (instead of pH 7.5)
- NaCl: Increase from 200 to 300 mM
- EDTA: Increase from 0.5 to 5 mM
- DTT: Increase from 5 to 10 mM
- R1881: 100 µM on lysis and binding to glutathione sepharose column
- Urea: Reduction from 2 M to 0 M (purification without urea!)

### Results 4

Purification was successful and the protein was concentrated to 3 mg/ml (total protein 1.0 mg after SDS PAGE

### Example 5

### HAR-LBD-Ligand complexes

Energy minimisation calculations were performed with the ligands R1881, testosterone and 19Nor-testosterone. In a first step the protocols used in the calculations were optimized such that the energy minimisation calculation of the hAR LBD - R1881 complex reproduced the interactions between the protein and the ligand as observed in the crystal structure of the same complex especially the hydrogen bond partners of the 03 and O17 atoms of the ligand with the protein, i.e. Arg752, Gln711 and Asn705. Then the same protocols were used for the calculations of the hAR LBD - testosterone and the hAR LBD-19Nor-testosterone complexes.

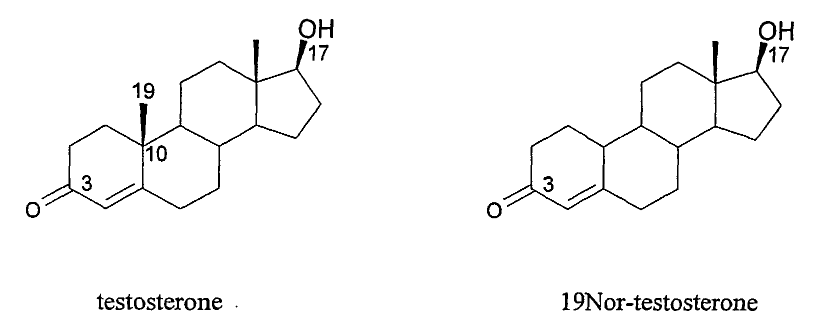

### Results 5

The results of the energy minimisation calculations confirm the hydrogen bond interactions at atom 03 of both testosterone and 19Nor-testosterone as observed in the crystal structure between R1881 and the hAR LBD (with Arg752 and Gln711). However, the interaction partners of the O17 atom at the D-ring are different due to the methyl substituent attached to position 10 of the steroid skeleton (position 19).

In case of the ligand 19Nor-testosterone, the 017 atom interacts with the side chain of Asn705. The calculations of the hAR LBD in complex with the ligand testosterone showed a shift in the orientation of the ligand in the ligand binding pocket (LBP) most likely due to the presence of the methyl group attached to position 10 of the steroid scaffold. Here, an interaction of the 017 atom with the side chain of Thr877 is observed in the calculations. The methyl group at that position in the ligand would be too close to amino acid residues Trp741 and Met745. In order to accommodate this ligand in the LBP, the ligand is shifted as well as the side chains of the amino acid residues Trp741 and Met745.

The amino acid residues of the hAR LBD within a radius of 4 Å around the respective ligands are the same for R1881 and 19Nor-testosterone. Due to the slight shift of testosterone of about 1.5 Å in the D-ring area, amino acid residues Trp741 and Ile899 are now farer away from testosterone.

### SUMMARY

A crystal comprising an androgen receptor ligand binding domain (AR-LBD) is provided. The crystal structures of the human Androgen Receptor (hAR) in comparison with the human Progesterone Receptor (hPR) Ligand Binding Domains (LBDs) in complex with the same ligand metribolone (R1881) is also provided. The three-dimensional structures of the hAR LBD as well as the hPR LBD show the typical nuclear receptor fold. The change of two residues in the ligand binding pocket (LBP) between hPR and hAR was identified as the most likely source for the specificity of the R1881 ligand binding to hAR LBD. The AR-LBD amino acid residues are Leu 880 and Thr 877. The corresponding PR amino acid residues Thr894 and Cys891. In addition, there are three other amino acid changes which maybe involved in binding of ligands other than R1881. The AR amino acid residues are Gln 783, Met 749 and Phe 876. The PR amino acid residues are Leu 797, Leu 763 and Tyr 890. The structural implications of the 14 known mutations in the LBP of the hAR LBD associated with either prostate cancer or the partial or complete androgen receptor insensitivity syndrome were analysed. The effects of most of these mutants could be explained on the basis of the crystal structure.

In one aspect, the present invention provides a method of identifying a compound that modulates (ie increases or decreases) AR activity, comprising: modeling test compounds that fit spatially into an AR LBD of interest using a model of the AR-LBD or portion thereof, screening the test compounds in an assay, for eg, a biological assay, characterised by binding of a test compound to the LBD and identifying a test compound that modulates AR activity wherein the structural model comprises structural co-ordinates of the LBD amino acid residues: L701; L704; N705; L707; Q711; M742; L744; M745; M749; R752; F764; Q783; M787; F876; T877; L880; F891; M895 or a homologue thereof.

In another aspect, the present invention relates to a computer readable medium having stored thereon a model of a crystal comprising an LBD structure of the AR-LBD.

In a further aspect, the present invention relates to a computer readable medium having stored thereon a model of a crystal comprising an AR-LBD wherein said model is built from all or part of the X-ray diffraction data shown in Table 1 and/or Table 2.

In an even further aspect, there is provided the use of the structural co-ordinates provided in Table 4 for the identification of a ligand or for building a crystal structure for an AR-LBD.

In another aspect, the present invention relates to a computer controlled method for designing a ligand capable of binding to the AR receptor comprising:
(i) providing a model of the crystal structure of the AR-LBD;
(ii) analysing said model to design a ligand which binds to the LBD; and
(iii) determining the effect of said ligand on said AR-LBD.

In a further aspect, there is provided a machine-readable data storage medium, comprising a data storage material encoded with machine readable data which, when using a machine programmed with instructions for using said data, is capable of displaying a graphical three dimensional representation of a crystal or a homologue of said crystal.

The present invention also provides a computer comprising such a storage medium.

The present invention also provides the use of such a computer in an industrial context, such as identifying putative ligands.

In another aspect, there is provided a method for homology modelling a crystal comprising an AR-LBD or a homologue thereof comprising:
(i) aligning the sequence of the AR-LBD (SEQ ID No 1 or SEQ ID No 2) or an AR-LBD homologue with the AR-LBD sequence and incorporating this sequence into the AR-LBD model;
(ii) subjecting a preliminary AR-LBD model to energy minimisation resulting in an energy minimised model;
(iii) remodeling the regions of said energy minimised model where stereochemistry restraints are violated; and
(iv) obtaining a final homology model.

### REFERENCES

Albers, N., Ulrichs, C., Glüer, S., Hiort, O., Sinnecker, G.H.G., Mildenberger, H. and Brodehl, J. (1997) Etiologic classification of severe hypospadias: Implications for prognosis and management. J. Pediatr., 131, 386-393.
Allen, F.H., Bellard, S., Brice, M.D., Cartwright, B., Doubleday, A., Higgs, H., Hummelink, T., Hummelink-Peters, B.G., Kennard, O., Motherwell, W.D.S., Rodgers, J.R. and Watson, D.G. (1979) Cambridge Structural Database. Acta Crystallogr., Sect. B, 35, 2331-2339.
Bellis, A.D., Quigley, C.A., Cariello, N.F., El-Awady, M.K., Sar, M., Lane, M.V., Wilson, E.M. and French, F.S. (1992) Single base mutations in the androgen receptor gene causing complete androgen insensitivity: rapid detection by a modified denaturing gradient gel electrophoresis technique.. Mol Endocrinol., 6, 1909-1920.
Benhamou, B., Garcia, T., Lerouge, T., Vergezac, A., Gofflo, D., Begogne, C., Chambon, P. and Gronemeyer, H. (1992) A single amino acid that determines the sensitivity of progesterone receptors to RU486.. Science, 255, 206-209.
Bevan, C.L., Brown, B.B., Davies, H.R., B. A. J. Evans, Hughes, I.A. and Patterson, M.N. (1996) Functional analysis of six androgen receptor mutations identified in patients with partial androgen insensitivity syndrome. Hum. Mol. Genet., 5, 265-273.
Bourguet, W., Ruff, M., Chambon, P., Gronemeyer, H. and Moras, D. (1995) Crystal structure of the ligand-binding domain of the human nuclear receptor RXR-alpha. Nature, 375, 377-382.
Brünger, A.T. (1992a) Free R value: A novel statistical quantity for assessing the accuracy of crystal structures.. Nature, 355, 472-474.
Brünger, A.T. (1992b) X-PLOR: a system for Crystallography and NMR.. The Howard Hughes Medical Institute and Department of Molecular Biophysics and Biochemistry, , Yale University, U.S.A.
Brzozowski, A.M., Pike, A.C.W., Dauter, Z., Hubbard, R.E., Bonn, T., Engström, O., Öhman, L., Greene, G.L., Gustafsson, J.A. and Carlquist, M. (1997) Molecular basis of agonism and antagonism in the oestrogen receptor.. Nature, 389, 753-758.
Cabral, D.F., Maciel-Guerra, A.T. and Hackel, C. (1998) Mutations of the androgene receptor gene in Brazilian patients with male pseudohermaphroditism. Brazilian J. Med. Biol. Research, 31, 775-778.
Collaborative Computational Project Number 4, C. (1994) The CCP4 suite: programs for protein crystallography. Acta Crystallogr., Sect D., 50, 760-763.
Gottlieb, B., Lehvaslaiho, H., Beitel, L.K., Lumbroso, R., Pinsky, L. and Trifiro, M. (1998) The androgen receptor gene mutation database.. Nucleic Acid Res., 26, 234-238.
Hakes, D.J. and Dixon, J.E. (1991) New vectors for high level expression of recombinant proteins in bacteria. Anal. Biochem., 202, 293-298.
Jakubicza, S., Werder, E.A. and Weiacker, P. (1992) Point mutation in the steroid binding domain of the androgen receptor gene in a family with complete androgen insensitivity syndrome (CAIS). Hum. Genet., 90, 311-312.
Jones, T.A., Zou, J.Y., Cowan, S.W. and Kjeldgaard, M. (1991) Improved methods for building protein models in electron density maps and the location of errors in these models. Acta Crystallogr. A, 47, 110-119.
Kabsch, W. (1976) A solution for the best rotation to relate two sets of vectors. Acta. Crystallogr. A, 32, 922-923.
Kabsch, W. and Sander, C. (1983) Dictionary of protein secondary structure: pattern recognition of hydrogen- bonded and geometrical features.. Biopolymers, 22, 2577-2637.
Klaholz, B.P., Renaud, J.P., Mitschler, A., Zusi, C., Chambon, P., Gronemeyer, H. and Moras, D. (1998) Conformational adaption of agonists to the human nuclear receptor RAR gamma. %J Nature Struct. Biol., 5, 199-202.
   Kleywegt, G.J. (1995) Dictionaries for Heteros. Joint CCP4 and ESF-EACMB Newsletter on Protein Oystallography, Vol. 31, pp. 45-50.
Komori, S., Kasumi, H., Sakata, K., Tanaka, H., Hamada, K. and Koyama, K. (1998) Molecular analysis of the androgen receptor gene in four patients with complete androgen insensitivity.. Arch. Gynecol. Obstet.95-100, 261**.**
Kraulis, P.J. (1991) MOLSCRIPT: A program to produce both detailed and schematic plots of protein structures.. J. Appl. Cryst., 24, 946-950.
Lamzin, V.S. and Wilson, K.S. (1997) Automated refinement for protein crystallography. In Jr., C.W.C. and Sweet, R.M. (eds.), Macromolecular Crystallography part B. Academic Press, New York, U.S.A., Vol. 277, pp. 269-305.
Laskowski, R.A., MacArthur, M.W., Moss, D.S. and Thornton, J.M. (1993) PROCHECK - a program to check the stereochemical quality of proteins. J Appl Crystallogr, 26, 283-291.
Letz, M., Bringmann, P., Mann, M., A. Mueller-Fahrnow, D, R., P. Scholz, P., Wurtz, J.M. and Egner, U. (1999) Investigation of the binding interactions of progesterone using muteins of the human progesterone receptor ligand binding domain designed on the basis of a three-dimensional model. Biochim. Biophys. Acta, 1429, 391-400.
Lubahn, D.B., Joseph, D.R., Sar, M., Tan, J., Higgs, H.N., Larson, R.E., French, F.S. and Wilson, E.M. (1988) The human androgen receptor: complementary deoxyribonucleic acid cloning, sequence analysis and gene expression in prostate. Mol. Endocrinol., 2, 1265-1275.
Lumbroso, R., Lobaccaro, J.M., Georget, V., Leger, J., Poujol, N., Rerouanne, B., Evian-Brion, D., Czernichow, P. and Sultan, C. (1996) A novel substitution Leu707Arg in exon 4 of the androgen receptor causes complete androgen resistance. J. Clin. Endocrinol. Metab., 81, 1984-1996.
Marcelli, M., Zoppi, S., Wilson, C.M., Griffin, J.E. and McPhaul, M.J. (1994) Amino acid substitutions of the hormone-binding domain of the human androgen receptor alter the stability of the hormone receptor complex. J. Clin. Invest., 94, 1642-1650.
   Merritt, E.A. and Murphy, M.E.P. (1994) Raster3D version 2.0. A program for photorealistic molecular graphics.. Acta Crystallogr. D, 50, 869-873.
Moras, D. and Gronemeyer, H. (1998) The nuclear receptor ligand-binding domain: structure and function. Curr. Opinion Cell Biol., 10, 384-391.
Murshudov, G.N., Vagin, A.A. and Dodson, E.J. (1997) Refinement of macromolecular structures by the maximum-likelihood method.. Acta Crystallogr., Sect. D , 53, 240-255.
Nakao, R., Haji, M., Yanase, T., Ogo, A., Takayanagi, R., Katsube, T., Fukumaki, Y. and Nawata, H. (1992) A single amino acid substitution (Met786Val) in the steroid binding domain of human androgen receptor leads to complete androgen insensitivity syndrome. J. Clin. Endocrinol. Metab., 74, 1152-1157.
Navaza, J. (1994) Acta Crystallogr., Sect A,, 50, 157-163.
Nolte, R.T., Wisely, G.B., Westin, S., Cobb, J.E., Lambert, M.H., Kurokawa, R., Rosenfeld, M.G., Willson, T.M., Glass, C.K. and Milburn, M.V. (1998) Ligand binding and co-activator assembly of the peroxisome proliferator-activated receptor-gamma. Nature, 395, 137-143.
Otwinowski, Z. and Minor, W. (1997) Processing of x-ray diffraction data collected in oscillation mode. In Jr, C.W.C. and Sweet, R.M. (eds.), Macromolecular Crystallography part A. Academic Press, New York, USA., Vol. 276 , pp. 307-326.
Perrakis, A., Sixma, T.K., Wilson, K.S. and Lamzin, V.S. (1997) wARP: improvement and extension of crystallographic phases by weighted averaging of multiple refined dummy atomic models. Acta Cryst. D, 53, 448-455.
Pinsky, L., Trifiro, M., Kaufman, M., Beitel, L.K., Mhatre, A., Kazemi-Esfarjani, P., Sabbaghian, N., Lumbroso, R., Alvarado, C., Vasiliou, M. and B. Gottlieb, B. (1992) Androgen resistance due to mutation of the androgen receptor.. Clin. Invest. Med., 15, 456-472.
Precigoux, G., Busetta, B. and S.Geoffre. (1981) 17beta-Hydroxy-17alphamethyl-4,9,11-estratrien-3-one. Acta Crystallogr., Sect. B, 37, 291-293.
Read, R.J. (1986) Improved Fourier coefficients for maps using phases from partial structures with errors. Acta Cryst A, 42, 140-149.
Renaud, J.P., Rochel, N., Ruff, M., Vivat, V., Chambon, P., Gronemeyer, H. and Moras, D. (1995) Crystal structure of the RAR-gamma ligand-binding domain bound to all-trans retinoic acid. Nature, 378, 681-689.
Ribeiro, R.C.J., Apriletti, J.W., Wagner, R.L., Feng, W., Kushner, P.J., Nilsson, S., Scanlan, T.S., West, B.L., Fletterick, R.J. and J. D. Baxter, J.D. (1998) X-ray crystallographic and functional studies of thyroid hormone receptor. J. Steroid Biochem. Molec. Biol., 65, 133-141.
Rochel, N., Wurtz, J.M., Mitchler, A., Klaholz, B. and Moras, D. (2000) Crystal structure of the nuclear receptor for vitamin D bound to its natural ligand. Molec. Cell, 5, 173-9.
Roussel, A., Fontecilla-Camps, J.C. and Cambillau, C. (1990) TURBO-FRODO: a new program for protein crystallography and modelling.. XV IUCr Congress, Bordeaux, France., pp. 66-67.
Shiau, A.K., Barstad, D., Loria, P.M., Cheng, L., Kushner, P.J., Agard, D.A. and Greene, G.L. (1998) The structural basis of estrogen receptor/ coactivator recognition and the antagonism of this interaction by tamoxifen. Cell, 95, 927-937.
Tannenbaum, D.M., Y. Wang, Williams, S.P. and Sigler, P.B. (1998) Crystallographic comparison of the estrogen and progesterone receptor's ligand binding domains.. Proc. Natl. Acad. Sci. USA, 95, 5998-6003.
Teutsch, G., Goubet, F., Battmann, T., Bonfils, A., Bouchoux, F., Cerede, E., Gofflo, D., Kelly, M.G. and Philibert, D. (1994) Non-steroidal antiandrogens: Synthesis and biological profile of high-affinity ligands for the androgen receptor.. J. Steroid Biochem. Molec. Biol., 48, 111-119.
Thompson, J.D., Higgins, D.G. and Gibson, T.J. (1994) CLUSTAL W: improving the sensitivity of progressive multiple sequence alignment through sequence weighting, positions-specific gap penalties and weight matrix choice. Nucl. Acids Res., 22, 4673-4680.
Tickle, I.J., Laskowski, R.A. and Moss, D.S. (1998) R-free and the R-free Ratio. I. Derivation of the expected values of cross-validation residuals used in macromolecular least-squares refinement. Acta Crystallogr., Sect. D, 54, 547-557.
Uppenberg, J., Svensson, C., Jaki, M., Bertilsson, G., Jendeberg, L. and Berkenstam, A. (1998) Crystal structure of the ligand binding domain of the human nuclear receptor PPARgamma.. J. Biol. Chem., 273, 31108-31112.
Veldscholte, J., Ris-Stalpers, C., Kuiper, G.G.J.M., Jenster, G., Berrevoets, C., Claassen, E., Rooij, H.C.J.v., Trapman, J., Brinkmann, A.O. and Mulder, E. (1990) A mutation in the ligand binding domain of the androgen receptor of human LNCaP cells affects steroid binding characteristics and response to antiandrogens. Biochem. Biophys. Res. Commun., 173, 534-540.
Wagner, R.L., Apriletti, J.W., McGrath, M.E., West, B.L., Baxter, J.D. and Fletterick, R.J. (1995) A structural role for hormone in the thyroid hormone receptor.. Nature, 378, 690-697.
   Williams, S.P. and Sigler, P.B. (1998) Atomic structure of progesterone complexed with its receptor. Nature, 393, 392-396.
Yong, E.L., Tut, T.G., Ghadessy, F.J., Prins, G. and Ratnam, S.S. (1998) Partial androgen insensitivity and correlations with the predicted three dimensional structure of the androgen receptor ligand-binding domain. Mol. Cell Endocrinol., 137,41-50.

## Claims

1. A crystal consisting of an androgen receptor ligand binding domain (AR-LBD) wherein said AR-LBD comprises a ligand binding pocket (LBP) and a ligand bound to the LBP wherein the ligand is metribolone (R1881); and wherein said crystal has the structural co-ordinates provided in Table 4 (Figure 6).

2. A method of screening for a ligand capable of binding to a LBD wherein the method comprises the use of a crystal according to claim 1.

3. A method for screening for a ligand capable of binding to a LBP wherein the LBP is defined in claim 1 and the method comprises contacting the LBP with a test compound, and determining if said test compound binds to said LBP.

4. A method according to claim 3 wherein the method is to screen for a ligand useful in the prevention and/or treatment of an androgen related disorder wherein the androgen related disorder is selected from the group consisting of androgen insensitivity syndrome (AIS), partial androgen insensitivity syndrome (PAIS), mild androgen insensitivity syndrome (MAIS), complete androgen insensitivity syndrome (CAIS) and prostrate cancer (PC).

5. Use of a crystal, according to claim 1, wherein the AR-LBD is used to screen for ligands that can modulate the activity of the AR-LBD.

6. A method for predicting, simulating or modeling the molecular characteristics and/or molecular interactions of a ligand binding domain (LBD) comprising the use of a computer model, said computer model comprising, using or depicting the structural coordinates of a ligand binding domain as defined in claim 1 to provide an image of said ligand binding domain and to optionally display said image.

## Patentansprüche

1. Kristall, bestehend aus einer Androgenrezeptor-Ligandenbindungsdomäne (AR-LBD), wobei die AR-LBD eine Ligandenbindungstasche (LBP) und einen an die LBP gebundenen Liganden umfaßt, wobei der Ligand Metribolon (R1881) ist, und wobei der Kristall die in Tabelle 4 (Figur 6) angegebenen Strukturkoordinaten hat.

2. Verfahren zum Screenen nach einem Liganden, der an eine LBD binden kann, wobei das Verfahren die Verwendung eines Kristalls nach Anspruch 1 umfaßt.

3. Verfahren zum Screenen nach einem Liganden, der an eine LBP binden kann, wobei die LBP in Anspruch 1 definiert ist und das Verfahren das inkontaktbringen der LBP mit einer Testverbindung und das Feststellen, ob die Testverbindung an die LBP bindet, umfaßt.

4. Verfahren nach Anspruch 3, wobei das Verfahren zum Screenen nach einem Liganden dienen soll, der bei der Prävention und/oder Behandlung einer mit Androgen im Zusammenhang stehenden Störung von Nutzen ist, wobei die mit Androgen im Zusammenhang stehende Störung aus der Gruppe ausgewählt ist, bestehend aus Androgenresistenzsyndrom (AIS), partiellem Androgenresistenzsyndrom (PAIS), mildem Androgenresistenzsyndrom (MAIS), komplettem Androgenresistenzsyndrom (CAIS) und Prostatakrebs (PC).

5. Verwendung eines Kristalls nach Anspruch 1, wobei die AR-LBD verwendet wird, um nach Liganden zu screenen, die die Aktivität der AR-LBD modulieren können.

6. Verfahren zum Vorhersagen, Simulieren oder Modellieren der molekularen Charakteristika und/oder der molekularen Wechselwirkungen einer Ligandenbindungsdomäne (LBD), welches die Verwendung eines Computermodells umfaßt, wobei das Computermodell die Strukturkoordinaten einer Ligandenbindungsdomäne, wie in Anspruch 1 definiert, umfaßt, verwendet oder darstellt, um eine Abbildung der Ligandenbindungsdomäne bereitzustellen und die Abbildung optional anzuzeigen.

## Revendications

1. Cristal consistant en un domaine de liaison de ligand de récepteur d'androgène (AR-LBD), dans lequel ledit AR-LBD comprend une poche de liaison de ligand (LBP) et un ligand lié à la LBP, ledit ligand étant la métribolone (R1881) ; ledit cristal ayant les coordonnées structurales indiquées sur le Tableau 4 (figure 6).

2. Méthode pour sélectionner un ligand capable de se lier à un LBD, ladite méthode comprenant l'utilisation d'un cristal suivant la revendication 1.

3. Méthode pour sélectionner un ligand capable de se lier à une LBP, dans laquelle la LBP est définie dans la revendication 1, ladite méthode comprenant les étapes consistant à mettre en contact la LBP avec un composé d'essai et à déterminer si ledit composé d'essai se lie à ladite LBP.

4. Méthode suivant la revendication 3, ladite méthode consistant à sélectionner un ligand utile dans la prévention et/ou le traitement d'un trouble en rapport avec un androgène, ledit trouble en rapport avec un androgène étant choisi dans le groupe consistant en le syndrome d'insensibilité aux androgènes (AIS), le syndrome d'insensibilité partielle aux androgènes (PAIS), le syndrome d'insensibilité bénigne aux androgènes (MAIS), le syndrome d'insensibilité totale aux androgènes (CAIS) et le cancer de la prostate (PC).

5. Utilisation d'un cristal suivant la revendication 1, dans laquelle le AR-LBD est utilisé pour sélectionner des ligands qui peuvent moduler l'activité du AR-LBD.

6. Méthode pour prévoir, simuler ou modéliser les caractéristiques moléculaires et/ou les interactions moléculaires d'un domaine de liaison de ligand (LBD), comprenant l'utilisation d'un modèle informatique, ledit modèle informatique comprenant, utilisant ou représentant les coordonnées structurales d'un domaine de liaison de ligand tel que défini dans la revendication 1 pour fournir une image dudit domaine de liaison de ligand et pour représenter facultativement ladite image.
